# EUROPEAN PATENT SPECIFICATION

(11) **EP 4 211 116 B1**
(45) Date of publication and mention of the grant of the patent: **07.08.2024**
(21) Application number: 21773522.4
(22) Date of filing: 06.09.2021
(51) Int. Cl.: C07D 231/14, C07D 401/12, C07D 401/14, C07D 403/12, C07D 403/14, A61K 31/4166, A61K 31/4178, A61P 31/04

(54) **NOVEL HETEROCYCLIC ANTIBIOTICS**
NEUE HETEROCYCLISCHE ANTIBIOTIKA
NOUVEAUX ANTIBIOTIQUES HÉTÉROCYCLIQUES

(30) Priority: 07.09.2020 WO PCT/CN2020/113771
(43) Date of publication of application: 19.07.2023
(73) Proprietor: F. Hoffmann-La Roche AG, 4070 Basel (CH)
(72) Inventor: CHENG, Zhanling, Shanghai, 201203 (CN); LERNER, Christian, 4070 Basel (CH); LIU, Yongqiang, Shanghai, 201203 (CN); NETTEKOVEN, Matthias, 4070 Basel (CH); PFLIEGER, Philippe, 4070 Basel (CH); PUELLMANN, Bernd, 4070 Basel (CH); WANG, Jianhua, Shanghai, 201203 (CN); WANG, Min, Shanghai, 201203 (CN); WANG, Yongguang, Shanghai, 201203 (CN); YANG, Song, Shanghai, 201203 (CN); ZHOU, Chengang, Shanghai, 201203 (CN)
(74) Representative: Neuhaus, Christian Michel
(86) International application number: PCT/EP2021/074432
(87) International publication number: WO 2022/049272

(56) References cited:
- WO-A1-2019/016782
- WO-A1-2020/126956
- WO-A1-2020/182648
- WO-A1-2021/148420

## Description

### Field of the Invention

The present invention relates to novel heterocyclic compounds which exhibit antibacterial properties. Also disclosed are methods of using the compounds for the treatment or prevention of bacterial infections and resulting diseases, in particular for the treatment or prevention of infections with *Acinetobacter baumannii* and resulting diseases.

### Background of the Invention

*Acinetobacter baumannii* is a Gram-negative, aerobic, nonfermenting bacterium recognized over the last decades as an emergining pathogen with very limited treatment options.

*A. baumannii* is considered to be a serious threat by the US Centers for Disease Control and Prevention and belongs to the so called 'ESKAPE' pathogens (***E**nterococcus faecium, **S**taphylococcus aureus, **K**lebsiella pneumoniae, **A**cinetobacter baumannii, **P**seudomonas aeruginosa* and ***E**nterobacter species & E. coli*) that currently cause the majority of nosocomial infections and effectively "escape" the activity of antimicrobial agents.

*A. baumannii* is most often encountered in intensive care units and surgical wards, where extensive antibiotic use has enabled selection for resistance against all known antimicrobials and where it causes infections that include bacteremia, pneumonia, meningitis, urinary tract infection, and wound infection.

*A. baumannii* has an exceptional ability to upregulate and acquire resistance determinants and shows an environmental persistance that allows its survival and spread in the nosocomial setting, making this organism a frequent cause of outbreaks of infection and an endemic, health care-associated pathogen.

Due to increasing antibiotic resistance to most if not all available therapeutic options, Muti-Drug Resistant (MDR) *A. baumanniii* infections, especially those caused by Carbapenem resistant A. *baumannii,* are extremely difficult or even impossible to treat with high mortality rate as well as increased morbidity and length of stay in intensive care unit.

*Acinetobacter baumannii* has been defined and still remains "a prime example of a mismatch between unmet medical needs and the current antimicrobial research and development pipeline" according to the Antimicrobial Availability Task Force (AATF) of the Infectious Diseases Society of America (IDSA). Thus, there is a high demand and need to identify compounds suitable for the treatment of diseases and infections caused by *Acinetobacter baumannii.*

The present invention provides novel compounds which exhibit activity against drug-susceptible as well as drug-resistant strains of *Acinetobacter baumannii.*

### Summary of the Invention

In a first aspect, the present invention provides a compound of formula (I) 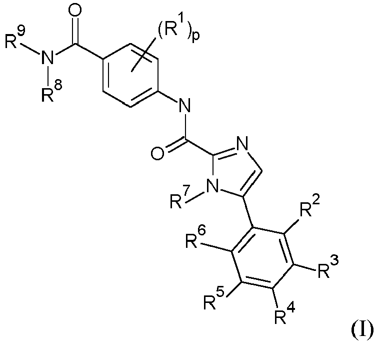 or a pharmaceutically acceptable salt thereof, wherein R¹ to R⁹ and p are as defined herein.

In one aspect, the present invention provides a process of manufacturing the compounds of formula (I) described herein, wherein said process is as described in any one of Schemes 1 to 4 herein.

In a further aspect, the present invention provides a compound of formula (I) as described herein, when manufactured according to the processes described herein.

In a further aspect, the present invention provides a compound of formula (I) as described herein, or a pharmaceutically acceptable salt thereof, for use as therapeutically active substance.

In a further aspect, the present invention provides a pharmaceutical composition comprising a compound of formula (I) as described herein, or a pharmaceutically acceptable salt thereof, and a therapeutically inert carrier.

In a further aspect, the present invention provides a compound of formula (I) as described herein, or a pharmaceutically acceptable salt thereof, for use as antibiotic.

In a further aspect, the present invention provides a compound of formula (I) as described herein, or a pharmaceutically acceptable salt thereof, for use in the treatment or prevention of nosocomial infections and resulting diseases.

In a further aspect, the present invention provides a compound of formula (I) as described herein, or a pharmaceutically acceptable salt thereof, for use in the treatment or prevention of infections and resulting diseases caused by Gram-negative bacteria.

In a further aspect, the present invention provides a compound of formula (I) as described herein, or a pharmaceutically acceptable salt thereof, for use in the treatment or prevention of infections and resulting diseases caused by ***E**nterococcus faecium, **S**taphylococcus aureus, **K**lebsiella pneumoniae, **A**cinetobacter baumannii, **P**seudomonas aeruginosa, **E**nterobacter species* or *E*. *coli*, or a combination therof.

Also disclosed is a method for the treatment or prevention of infections and resulting diseases caused by ***E**nterococcus faecium, **S**taphylococcus aureus, **K**lebsiella pneumoniae, **A**cinetobacter baumannii, **P**seudomonas aeruginosa, **E**nterobacter species* or *E. coli*, or a combination therof, which method comprises administering a compound of formula (I) as described herein, or a pharmaceutically acceptable salt thereof, to a mammal.

Also disclosed is the use of a compound of formula (I) as described herein, or a pharmaceutically acceptable salt thereof, as an antibiotic.

Also disclosed is the use of a compound of formula (I) as described herein, or a pharmaceutically acceptable salt thereof, for the treatment or prevention of infections and resulting diseases caused by ***E**nterococcus faecium, **S**taphylococcus aureus, **K**lebsiella pneumoniae, **A**cinetobacter baumannii, **P**seudomonas aeruginosa, **E**nterobacter species* or *E. coli*, or a combination therof.

In a further aspect, the present invention provides the use of a compound of formula (I) as described herein, or a pharmaceutically acceptable salt thereof, for the preparation of medicaments useful for the treatment or prevention of infections and resulting diseases caused by ***E**nterococcus faecium, **S**taphylococcus aureus, **K**lebsiella pneumoniae, **A**cinetobacter baumannii, **P**seudomonas aeruginosa, **E**nterobacter species* or *E. coli*, or a combination therof.

### Detailed Description of the Invention

### Definitions

Features, integers, characteristics, compounds, chemical moieties or groups described in conjunction with a particular aspect, embodiment or example of the invention are to be understood to be applicable to any other aspect, embodiment or example described herein, unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The term "alkyl" refers to a mono- or multivalent, e.g., a mono- or bivalent, linear or branched saturated hydrocarbon group of 1 to 6 carbon atoms ("C₁-C₆-alkyl"), e.g., 1, 2, 3, 4, 5, or 6 carbon atoms. In some embodiments, the alkyl group contains 1 to 3 carbon atoms, e.g., 1, 2 or 3 carbon atoms. Some non-limiting examples of alkyl include methyl, ethyl, propyl, 2-propyl (isopropyl), n-butyl, iso-butyl, sec-butyl, tert-butyl, and 2,2-dimethylpropyl. Particularly preferred, yet non-limiting examples of alkyl include methyl and ethyl.

The term "alkoxy" refers to an alkyl group, as previously defined, attached to the parent molecular moiety via an oxygen atom. Unless otherwise specified, the alkoxy group contains 1 to 6 carbon atoms ("C₁-C₆-alkoxy"). In some preferred embodiments, the alkoxy group contains contains 1 to 4 carbon atoms. In still other embodiments, the alkoxy group contains 1 to 3 carbon atoms. Some non-limiting examples of alkoxy groups include methoxy, ethoxy, n-propoxy, isopropoxy, n-butoxy, isobutoxy and tert-butoxy. A particularly preferred, yet non-limiting example of alkoxy is methoxy.

The term "halogen" or "halo" refers to fluoro (F), chloro (Cl), bromo (Br), or iodo (I). Preferably, the term "halogen" or "halo" refers to fluoro (F), chloro (Cl) or bromo (Br).

Particularly preferred, yet non-limiting examples of "halogen" or "halo" are fluoro (F) and chloro (Cl).

The term "cycloalkyl" as used herein refers to a saturated or partly unsaturated monocyclic or bicyclic hydrocarbon group of 3 to 10 ring carbon atoms ("C₃-C₁₀-cycloalkyl"). In some preferred embodiments, the cycloalkyl group is a saturated monocyclic hydrocarbon group of 3 to 8 ring carbon atoms. "Bicyclic cycloalkyl" refers to cycloalkyl moieties consisting of two saturated carbocycles having two carbon atoms in common, i.e., the bridge separating the two rings is either a single bond or a chain of one or two ring atoms, and to spirocyclic moieties, i.e., the two rings are connected via one common ring atom. Preferably, the cycloalkyl group is a saturated monocyclic hydrocarbon group of 3 to 6 ring carbon atoms, e.g., of 3, 4, 5 or 6 carbon atoms. Some non-limiting examples of cycloalkyl include cyclopropyl, cyclobutyl, cyclopentyl, cyclohexyl, cycloheptyl, and spiro[2.3]hexan-5-yl.

The term "aminoalkoxy" refers to an alkoxy group, wherein at least one of the hydrogen atoms of the alkoxy group has been replaced by an amino group. Preferably, "aminoalkoxy" refers to an alkoxy group wherein 1, 2 or 3 hydrogen atoms of the alkoxy group have been replaced by an amino group. Preferred, yet non-limiting examples of aminoalkoxy are aminomethoxy and 1-aminoethoxy.

The term "heterocyclyl" refers to a saturated or partly unsaturated mono- or bicyclic, preferably monocyclic ring system of 3 to 14 ring atoms, preferably 3 to 10 ring atoms, more preferably 3 to 8 ring atoms wherein 1, 2, or 3 of said ring atoms are heteroatoms selected from N, O and S, the remaining ring atoms being carbon. Preferably, 1 to 2 of said ring atoms are selected from N and O, the remaining ring atoms being carbon. "Bicyclic heterocyclyl" refers to heterocyclic moieties consisting of two cycles having two ring atoms in common, i.e., the bridge separating the two rings is either a single bond or a chain of one or two ring atoms, and to spirocyclic moieties, i.e., the two rings are connected via one common ring atom. Some non-limiting examples of heterocyclyl groups include azetidin-3-yl; azetidin-2-yl; oxetan-3-yl; oxetan-2-yl; piperidyl; piperazinyl; pyrrolidinyl; 2-oxopyrrolidin-1-yl; 2-oxopyrrolidin-3-yl; 5-oxopyrrolidin-2-yl; 5-oxopyrrolidin-3-yl; 2-oxo-1-piperidyl; 2-oxo-3-piperidyl; 2-oxo-4-piperidyl; 6-oxo-2-piperidyl; 6-oxo-3-piperidyl; 1-piperidinyl; 2-piperidinyl; 3-piperidinyl; 4-piperidinyl; morpholino; morpholin-2-yl; morpholin-3-yl; pyrrolidinyl (e.g., pyrrolidin-3-yl); 3-azabicyclo[3.1.0]hexan-6-yl; 2,5-diazabicyclo[2.2.1]heptan-2-yl; 2-azaspiro[3.3]heptan-2-yl; 2,6-diazaspiro[3.3]heptan-2-yl; and 2,3,3a,4,6,6a-hexahydro-1H-pyrrolo[3,4-c]pyrrol-5-yl.

The term "aryl" refers to a monocyclic, bicyclic, or tricyclic carbocyclic ring system having a total of 6 to 10 ring members ("C₆-C₁₀-aryl") and wherein at least one ring in the system is aromatic. A particularly preferred, yet non-limiting example of aryl is phenyl.

The term "heteroaryl" refers to a mono- or multivalent, monocyclic or bicyclic, preferably bicyclic ring system having a total of 5 to 14 ring members, preferably, 5 to 12 ring members, and more preferably 5 to 10 ring members, wherein at least one ring in the system is aromatic, and at least one ring in the system contains one or more heteroatoms. Preferably, "heteroaryl" refers to a 5-10 membered heteroaryl comprising 1, 2, 3 or 4 heteroatoms independently selected from O, S and N. Most preferably, "heteroaryl" refers to a 5-10 membered heteroaryl comprising 1 to 2 heteroatoms independently selected from O and N. Some non-limiting examples of heteroaryl include 2-pyridyl, 3-pyridyl, 4-pyridyl, pyrimidin-2-yl, pyrimidin-4-yl, pyrimidin-5-yl, pyrimidin-6-yl, indol-1-yl, 1H-indol-2-yl, 1H-indol-3-yl, 1H-indol-4-yl, 1H-indol-5-yl, 1H-indol-6-yl, 1H-indol-7-yl, 1,2-benzoxazol-3-yl, 1,2-benzoxazol-4-yl, 1,2-benzoxazol-5-yl, 1,2-benzoxazol-6-yl, 1,2-benzoxazol-7-yl, 1H-indazol-3-yl, 1H-indazol-4-yl, 1H-indazol-5-yl, 1H-indazol-6-yl, 1H-indazol-7-yl, pyrazol-1-yl, 1H-pyrazol-3-yl, 1H-pyrazol-4-yl, 1H-pyrazol-5-yl, imidazol-1-yl, 1H-imidazol-2-yl, 1H-imidazol-4-yl, 1H-imidazol-5-yl, oxazol-2-yl, oxazol-4-yl, oxazol-5-yl, thiazol-4-yl, and 1,2,4-oxadiazol-3-yl. Most preferably, "heteroaryl" refers to pyridyl and pyrimidinyl.

The term "hydroxy" refers to an -OH group.

The term "amino" refers to an -NH₂ group.

The term "cyano" refers to a -CN (nitrile) group.

The term "carbamoyl" refers to a -C(O)NH₂ group.

The term "carbonyl" refers to a carbon radical having two of the four covalent bonds shared with an oxygen atom (C=O).

The term "haloalkyl" refers to an alkyl group, wherein at least one of the hydrogen atoms of the alkyl group has been replaced by a halogen atom, preferably fluoro. Preferably, "haloalkyl" refers to an alkyl group wherein 1, 2 or 3 hydrogen atoms of the alkyl group have been replaced by a halogen atom, most preferably fluoro. Non-limiting examples of haloalkyl are fluoromethyl, difluoromethyl, trifluoromethyl, trifluoroethyl, 2-fluoroethyl, and 2,2-difluoroethyl. A particularly preferred, yet non-limiting example of haloalkyl is trifluoromethyl.

The term "cyanoalkyl" refers to an alkyl group, wherein at least one of the hydrogen atoms of the alkyl group has been replaced by cyano group. Preferably, "cyanoalkyl" refers to an alkyl group wherein 1, 2 or 3 hydrogen atoms of the alkyl group have been replaced by a cyano group. Most preferably, "cyanoalkyl" refers to an alkyl group wherein 1 hydrogen atom of the alkyl group has been replaced by a cyano group. A preferred, yet non-limiting example of cyanoalkyl is cyanomethyl.

The term "haloalkoxy" refers to an alkoxy group, wherein at least one of the hydrogen atoms of the alkoxy group has been replaced by a halogen atom, preferably fluoro. Preferably, "haloalkoxy" refers to an alkoxy group wherein 1, 2 or 3 hydrogen atoms of the alkoxy group have been replaced by a halogen atom, most preferably fluoro. Particularly preferred, yet non-limiting examples of haloalkoxy are fluoromethoxy (FCH₂O-), difluoromethoxy (F₂CHO-), and trifluoromethoxy (F₃CO-).

The term "cyanoalkoxy" refers to an alkoxy group, wherein at least one of the hydrogen atoms of the alkoxy group has been replaced by cyano group. Preferably, "cyanoalkoxy" refers to an alkoxy group wherein 1, 2 or 3 hydrogen atoms of the alkoxy group have been replaced by a cyano group. Most preferably, "cyanoalkoxy" refers to an alkoxy group wherein 1 hydrogen atom of the alkoxy group has been replaced by a cyano group. A preferred, yet non-limiting example of cyanoalkoxy is cyanomethoxy.

The term "carbamoylalkoxy" refers to an alkoxy group, wherein at least one of the hydrogen atoms of the alkoxy group has been replaced by a carbamoyl group. Preferably, "carbamoylalkoxy" refers to an alkoxy group wherein 1, 2 or 3 hydrogen atoms of the alkoxy group have been replaced by a carbamoyl group. Most preferably, "carbamoylalkoxy" refers to an alkoxy group wherein 1 hydrogen atom of the alkoxy group has been replaced by a carbamoyl group.

The term "hydroxyalkyl" refers to an alkyl group, wherein at least one of the hydrogen atoms of the alkyl group has been replaced by a hydroxy group. Preferably, "hydroxyalkyl" refers to an alkyl group wherein 1, 2 or 3 hydrogen atoms, most preferably 1 hydrogen atom of the alkyl group have been replaced by a hydroxy group. Preferred, yet non-limiting examples of hydroxyalkyl are hydroxymethyl, hydroxyethyl (e.g. 2-hydroxyethyl), and 3-hydroxy-3-methylbutyl.

The term "aminoalkyl" refers to an alkyl group, wherein at least one of the hydrogen atoms of the alkyl group has been replaced by an amino group. Preferably, "aminoalkyl" refers to an alkyl group wherein 1, 2 or 3 hydrogen atoms, most preferably 1 hydrogen atom of the alkyl group have been replaced by an amino group. Preferred, yet non-limiting examples of aminoalkyl are aminomethyl, aminoethyl (e.g. 2-aminoethyl), 3-amino-3-methyl-butyl, aminopentyl (e.g., 5-aminopentyl), and aminohexyl (e.g., 6-aminohexyl).

The term "pharmaceutically acceptable salt" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as acetic acid, trifluoroacetic acid, propionic acid, glycolic acid, pyruvic acid, oxalic acid, maleic acid, malonic acid, succinic acid, fumaric acid, tartaric acid, lactic acid, citric acid, benzoic acid, cinnamic acid, mandelic acid, methanesulfonic acid, ethanesulfonic acid, p-toluenesulfonic acid, salicylic acid, N-acetylcystein and the like. In addition these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of compounds of formula (I) are hydrochlorides, fumarates, lactates (in particular derived from L-(+)-lactic acid), tartrates (in particular derived from L-(+)-tartaric acid) and trifluoroacetates.

The compounds of formula (I) can contain several asymmetric centers and can be present in the form of optically pure enantiomers, mixtures of enantiomers such as, for example, racemates, optically pure diastereioisomers, mixtures of diastereoisomers, diastereoisomeric racemates or mixtures of diastereoisomeric racemates.

According to the Cahn-Ingold-Prelog Convention, the asymmetric carbon atom can be of the "R" or "S" configuration.

The term "treatment" as used herein includes: (1) inhibiting the state, disorder or condition (e.g. arresting, reducing or delaying the development of the disease, or a relapse thereof in case of maintenance treatment, of at least one clinical or subclinical symptom thereof); and/or (2) relieving the condition (i.e., causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

The term "prophylaxis" as used herein includes: preventing or delaying the appearance of clinical symptoms of the state, disorder or condition developing in a mammal and especially a human that may be afflicted with or predisposed to the state, disorder or condition but does not yet experience or display clinical or subclinical symptoms of the state, disorder or condition.

The term "mammal" as used herein includes both humans and non-humans and includes but is not limited to humans, non-human primates, canines, felines, murines, bovines, equines, and porcines. In a particularly preferred embodiment, the term "mammal" refers to humans.

The term "nosocomial infection" refers to a hospital-acquired infection (HAI), which is an infection that is acquired in a hospital or other health care facility. To emphasize both hospital and nonhospital settings, it is sometimes instead called a health care-associated infection (HAI or HCAI). Such an infection can be acquired in hospitals, nursing homes, rehabilitation facilities, outpatient clinics, or other clinical settings.

### Compounds of the Invention

In a first aspect, the present invention provides a compound of formula (I) 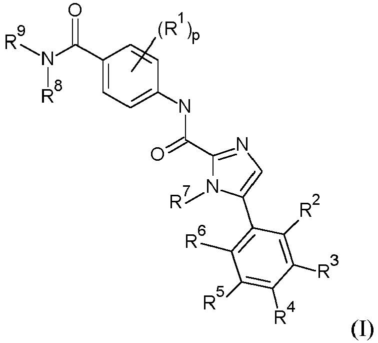 or a pharmaceutically acceptable salt thereof, wherein:
- R¹: is, at each occurrence, independently selected from halogen, cyano, C₁-C₆-alkyl, C₁-C₆-alkoxy, halo-C₁-C₆-alkyl, and halo-C₁-C₆-alkoxy;
- R², R³, R⁴, R⁵ and R⁶: are each independently selected from hydrogen, halogen, C₁-C₆-alkyl, C₁-C₆-alkoxy, cyano, halo-C₁-C₆-alkyl, cyano-C₁-C₆-alkyl, (C₁-C₆-alkyl)₂N-, halo-C₁-C₆-alkoxy, cyano-C₁-C₆-alkoxy, C₁-C₆-alkoxy-C₁-C₆-alkoxy-, (C₁-C₆-alkyl)₂N-C(O)-, and carbamoyl-C₁-C₆-alkoxy;
- R⁷: is selected from hydrogen, C₁-C₆-alkyl, and halo-C₁-C₆-alkyl;
- R⁸: is selected from C₁-C₆-alkyl, amino-C₁-C₆-alkyl, amino-C₁-C₆-alkyl-C(O)-, (C₁-C₆-alkyl)₂N-C₁-C₆-alkyl-, C₁-C₆-alkyl-NH-C₁-C₆-alkyl-, amino-C₁-C₆-alkoxy-C₁-C₆-alkyl-, C₁-C₆-alkyl-NH-C₁-C₆-alkoxy-C₁-C₆-alkyl-, (C₁-C₆-alkyl)₂N-C₁-C₆-alkoxy-C₁-C₆-alkyl-, amino-C₁-C₆-alkoxy-C₁-C₆-alkyl-C(O)-, C₁-C₆-alkyl-NH-C₁-C₆-alkoxy-C₁-C₆-alkyl-C(O)-, (C₁-C₆-alkyl)₂N-C₁-C₆-alkoxy-C₁-C₆-alkyl-C(O)-, amino-C₁-C₆-alkyl-C(O)-NH-C₁-C₆-alkyl-, C₁-C₆-alkyl-NH-C₁-C₆-alkyl-C(O)-NH-C₁-C₆-alkyl-, (C₁-C₆-alkyl)₂N-C₁-C₆-alkyl-C(O)-NH-C₁-C₆-alkyl-, and a group 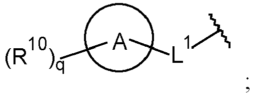
- R⁹: is selected from hydrogen and C₁-C₆-alkyl;
- R¹⁰: is selected from halogen, cyano, amino, hydroxy, C₁-C₆-alkyl, C₁-C₆-alkoxy, halo-C₁-C₆-alkyl, halo-C₁-C₆-alkoxy, amino-C₁-C₆-alkyl-, C₁-C₆-alkyl-NH-C₁-C₆-alkyl-, (C₁-C₆-alkyl)₂N-C₁-C₆-alkyl-, amino-C₁-C₆-alkyl-C(O)-, C₁-C₆-alkyl-NH-C₁-C₆-alkyl-C(O)-, (C₁-C₆-alkyl)₂N-C₁-C₆-alkyl-C(O)-, amino-C₁-C₆-alkyl-NH-C(O)-, amino-C₁-C₆-alkoxy-C₁-C₆-alkyl-C(O)-, hydroxy-C₁-C₆-alkyl, and a group 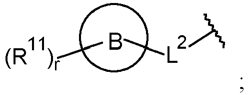
- R¹¹: is selected from halogen, cyano, amino, hydroxy, C₁-C₆-alkyl, C₁-C₆-alkoxy, halo-C₁-C₆-alkyl, halo-C₁-C₆-alkoxy, amino-C₁-C₆-alkyl-, C₁-C₆-alkyl-NH-C₁-C₆-alkyl-, (C₁-C₆-alkyl)₂N-C₁-C₆-alkyl-, amino-C₁-C₆-alkyl-C(O)-, C₁-C₆-alkyl-NH-C₁-C₆-alkyl-C(O)-, (C₁-C₆-alkyl)₂N-C₁-C₆-alkyl-C(O)-, amino-C₁-C₆-alkyl-NH-C(O)-, amino-C₁-C₆-alkoxy-C₁-C₆-alkyl-C(O)-, and hydroxy-C₁-C₆-alkyl;
- A and B: are each independently selected from 3- to 14-membered heterocyclyl, C₃-C₁₀-cycloalkyl, 5- to 14-membered heteroaryl, and C₆-C₁₀-aryl;
- L¹: is selected from a covalent bond, carbonyl, -NH-C(O)-, C₁-C₆-alkyl, -C(O)-NH-C₁-C₆-alkyl-, -C₁-C₆-alkyl-NH-C(O)-, and -NH-C(O)-NH-C₁-C₆-alkyl-;
- L²: is selected from a covalent bond, carbonyl, -NH-C(O)-, -C(O)-NH-, C₁-C₆-alkyl,-C(O)-NH-C₁-C₆-alkyl-, -C₁-C₆-alkyl-NH-C(O)-, and -NH-C(O)-NH-C₁-C₆-alkyl-;
- p: is selected from 0, 1, 2, 3, or 4; and
- q and r: are independently selected from 0, 1, 2, and 3.

In one embodiment, the present invention provides a compound of formula (I) as described herein, or a pharmaceutically acceptable salt thereof, wherein said compound of formula (I) is a compound of formula (I-I) 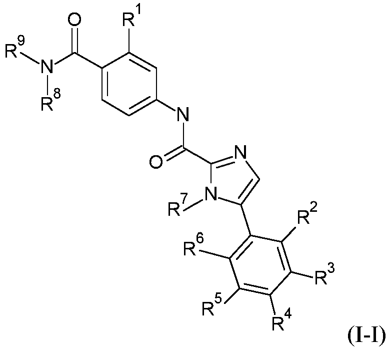 wherein R¹ to R⁹ are as defined herein.

In one embodiment, the present invention provides a compound of formula (I) as described herein, or a pharmaceutically acceptable salt thereof, wherein said compound of formula (I) is a compound of formula (I-II) 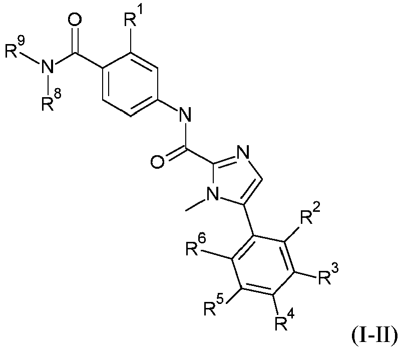 wherein R¹ to R⁶, R⁸ and R⁹ are as defined herein.

In one embodiment, the present invention provides a compound of formula (I) as described herein, or a pharmaceutically acceptable salt thereof, wherein said compound of formula (I) is a compound of formula (I-III) 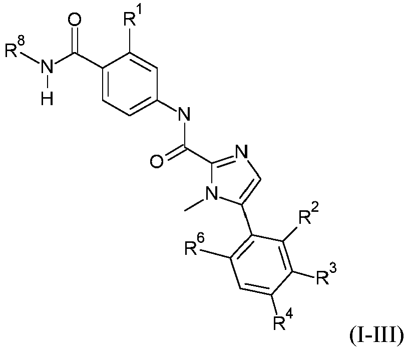 wherein R¹ to R⁴, R⁶ and R⁸ are as defined herein.

In one embodiment, the present invention provides a compound of formula (I) as described herein, or a pharmaceutically acceptable salt thereof, wherein:
- R¹: is C₁-C₆-alkyl or halogen; and
- p: is 1.

In a preferred embodiment, the present invention provides a compound of formula (I) as described herein, or a pharmaceutically acceptable salt thereof, wherein:
- R¹: is halogen; and
- p: is 1.

In a particularly preferred embodiment, the present invention provides a compound of formula (I) as described herein, or a pharmaceutically acceptable salt thereof, wherein:
- R¹: is chloro; and
- p: is 1.

In one embodiment, the present invention provides a compound of formula (I) as described herein, or a pharmaceutically acceptable salt thereof, wherein:
- R²: is hydrogen or halogen;
- R³: is hydrogen or halogen;
- R⁴: is selected from C₁-C₆-alkoxy, halo-C₁-C₆-alkoxy, cyano-C₁-C₆-alkoxy, and carbamoyl-C₁-C₆-alkoxy;
- R⁵: is hydrogen; and
- R⁶: is hydrogen or halogen.

In a preferred embodiment, the present invention provides a compound of formula (I) as described herein, or a pharmaceutically acceptable salt thereof, wherein:
- R² and R³: are both halogen;
- R⁴: is selected from C₁-C₆-alkoxy and halo-C₁-C₆-alkoxy; and
- R⁵ and R⁶: are both hydrogen.

In a particularly preferred embodiment, the present invention provides a compound of formula (I) as described herein, or a pharmaceutically acceptable salt thereof, wherein:
- R² and R³: are both fluoro;
- R⁴: is selected from methoxy and difluoromethoxy; and
- R⁵ and R⁶: are both hydrogen.

In one embodiment, the present invention provides a compound of formula (I) as described herein, or a pharmaceutically acceptable salt thereof, wherein R⁷ is C₁-C₆-alkyl.

In a preferred embodiment, the present invention provides a compound of formula (I) as described herein, or a pharmaceutically acceptable salt thereof, wherein R⁷ is methyl.

In one embodiment, the present invention provides a compound of formula (I) as described herein, or a pharmaceutically acceptable salt thereof, wherein:
- R⁸: is selected from C₁-C₆-alkyl, amino-C₁-C₆-alkyl, (C₁-C₆-alkyl)₂N-C₁-C₆-alkyl-, amino-C₁-C₆-alkoxy-C₁-C₆-alkyl-, (C₁-C₆-alkyl)₂N-C₁-C₆-alkoxy-C₁-C₆-alkyl-, amino-C₁-C₆-alkyl-C(O)-NH-C₁-C₆-alkyl-, (C₁-C₆-alkyl)₂N-C₁-C₆-alkyl-C(O)-NH-C₁-C₆-alkyl-, and a group 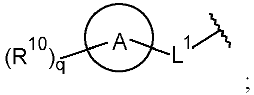
- R⁹: is hydrogen;
- R¹⁰: is selected from amino, hydroxy, C₁-C₆-alkyl, amino-C₁-C₆-alkyl-C(O)-, (C₁-C₆-alkyl)₂N-C₁-C₆-alkyl-C(O)-, amino-C₁-C₆-alkyl-NH-C(O)-, amino-C₁-C₆-alkoxy-C₁-C₆-alkyl-C(O)-, hydroxy-C₁-C₆-alkyl, and a group 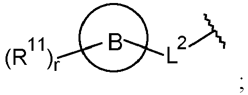
- R¹¹: is selected from hydroxy, amino, and amino-C₁-C₆-alkyl;
- A and B: are each independently selected from 3- to 14-membered heterocyclyl and C₃-C₁₀-cycloalkyl;
- L¹: is selected from a covalent bond, C₁-C₆-alkyl, -C(O)-NH-C₁-C₆-alkyl-, and -NH-C(O)-NH-C₁-C₆-alkyl-;
- L²: is selected from carbonyl, C₁-C₆-alkyl, -C(O)-NH-, and -NH-C(O)-; and q and r are each independently selected from 0 and 1.

In a preferred embodiment, the present invention provides a compound of formula (I) as described herein, or a pharmaceutically acceptable salt thereof, wherein:
- R⁸: is a group 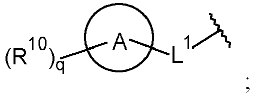
- R⁹: is hydrogen;
- R¹⁰: is selected from amino-C₁-C₆-alkyl-C(O)-, (C₁-C₆-alkyl)₂N-C₁-C₆-alkyl-C(O)-, amino-C₁-C₆-alkoxy-C₁-C₆-alkyl-C(O)-, and a group 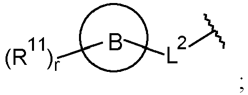
- R¹¹ is: selected from hydroxy and amino;
- A and B: are each independently selected from 3- to 14-membered heterocyclyl and C₃-C₁₀-cycloalkyl;
- L¹ is: selected from a covalent bond and C₁-C₆-alkyl;
- L² is: selected from carbonyl, -C(O)-NH-, and -NH-C(O)-;
- q is: 1; and
- r is: selected from 0 and 1.

In a particularly preferred embodiment, the present invention provides a compound of formula (I) as described herein, or a pharmaceutically acceptable salt thereof, wherein:
- R⁸: is a group 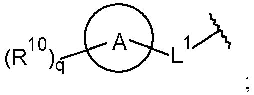
- R⁹: is hydrogen;
- R¹⁰: is selected from 2-aminoacetyl, 2-aminopropanoyl, 2-(dimethylamino)acetyl, 3-(2-aminoethoxy)propanoyl, and a group 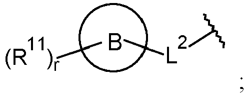
- R¹¹: is selected from hydroxy and amino;
- A: is selected from azetidinyl, cyclobutyl, pyrrolidinyl, and piperidyl;
- B: is selected from cyclobutyl, pyrrolidinyl, and piperidyl;
- L¹: is selected from a covalent bond and -(CH₂)₂-;
- L²: is selected from carbonyl, -C(O)-NH-, and -NH-C(O)-;
- q: is 1; and
- r: is selected from 0 and 1.

In one embodiment, the present invention provides a compound of formula (I) as described herein, or a pharmaceutically acceptable salt thereof, wherein:
- R¹: is C₁-C₆-alkyl or halogen;
- R²: is hydrogen or halogen;
- R³: is hydrogen or halogen;
- R⁴: is selected from C₁-C₆-alkoxy, halo-C₁-C₆-alkoxy, cyano-C₁-C₆-alkoxy, and carbamoyl-C₁-C₆-alkoxy;
- R⁵ and R⁹: are both hydrogen;
- R⁶: is hydrogen or halogen;
- R⁷: is C₁-C₆-alkyl;
- R⁸: is selected from C₁-C₆-alkyl, amino-C₁-C₆-alkyl, (C₁-C₆-alkyl)₂N-C₁-C₆-alkyl-, amino-C₁-C₆-alkoxy-C₁-C₆-alkyl-, (C₁-C₆-alkyl)₂N-C₁-C₆-alkoxy-C₁-C₆-alkyl-, amino-C₁-C₆-alkyl-C(O)-NH-C₁-C₆-alkyl-, (C₁-C₆-alkyl)₂N-C₁-C₆-alkyl-C(O)-NH-C₁-C₆-alkyl-, and a group 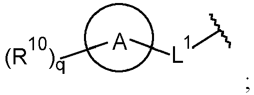
- R¹⁰: is selected from amino, hydroxy, C₁-C₆-alkyl, amino-C₁-C₆-alkyl-C(O)-, (C₁-C₆-alkyl)₂N-C₁-C₆-alkyl-C(O)-, amino-C₁-C₆-alkyl-NH-C(O)-, amino-C₁-C₆-alkoxy-C₁-C₆-alkyl-C(O)-, hydroxy-C₁-C₆-alkyl, and a group 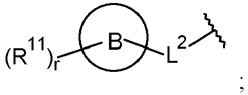
- R¹¹: is selected from hydroxy, amino, and amino-C₁-C₆-alkyl;
- A and B: are each independently selected from 3- to 14-membered heterocyclyl and C₃-C₁₀-cycloalkyl;
- L¹: is selected from a covalent bond, C₁-C₆-alkyl, -C(O)-NH-C₁-C₆-alkyl-, and -NH-C(O)-NH-C₁-C₆-alkyl-;
- L²: is selected from carbonyl, C₁-C₆-alkyl, -C(O)-NH-, and -NH-C(O)-;
- p: is 1; and
- q and r: are each independently selected from 0 and 1.

In a preferred embodiment, the present invention provides a compound of formula (I) as described herein, or a pharmaceutically acceptable salt thereof, wherein:
- R¹, R² and R³: are each independently halogen;
- R⁴: is selected from C₁-C₆-alkoxy and halo-C₁-C₆-alkoxy;
- R⁵, R⁶ and R⁹: are all hydrogen;
- R⁷: is C₁-C₆-alkyl;
- R⁸: is a group 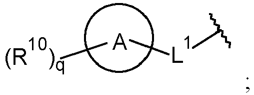
- R¹⁰: is selected from amino-C₁-C₆-alkyl-C(O)-, (C₁-C₆-alkyl)₂N-C₁-C₆-alkyl-C(O)-, amino-C₁-C₆-alkoxy-C₁-C₆-alkyl-C(O)-, and a group 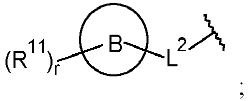
- R¹¹: is selected from hydroxy and amino;
- A and B: are each independently selected from 3- to 14-membered heterocyclyl and C₃-C₁₀-cycloalkyl;
- L¹: is selected from a covalent bond and C₁-C₆-alkyl;
- L²: is selected from carbonyl, -C(O)-NH-, and -NH-C(O)-;
- q: is 1;
- r: is selected from 0 and 1; and
- p: is 1.

In a particularly preferred embodiment, the present invention provides a compound of formula (I) as described herein, or a pharmaceutically acceptable salt thereof, wherein:
- R¹: is chloro;
- R² and R³: are both fluoro;
- R⁴: is selected from methoxy and difluoromethoxy;
- R⁵, R⁶ and R⁹: are all hydrogen;
- R⁷: is methyl;
- R⁸: is a group 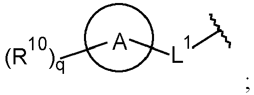
- R¹⁰: is selected from 2-aminoacetyl, 2-aminopropanoyl, 2-(dimethylamino)acetyl, 3-(2-aminoethoxy)propanoyl, and a group 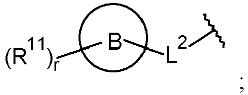
- R¹¹: is selected from hydroxy and amino;
- A: is selected from azetidinyl, cyclobutyl, pyrrolidinyl, and piperidyl;
- B: is selected from cyclobutyl, pyrrolidinyl, and piperidyl;
- L¹: is selected from a covalent bond and -(CH₂)₂-;
- L²: is selected from carbonyl, -C(O)-NH-, and -NH-C(O)-;
- q: is 1;
- r: is selected from 0 and 1; and
- p: is 1.

In one embodiment, the present invention provides a compound of formula (I) as described herein, or a pharmaceutically acceptable salt thereof, wherein R¹ is C₁-C₆-alkyl or halogen.

In one embodiment, the present invention provides a compound of formula (I) as described herein, or a pharmaceutically acceptable salt thereof, wherein R² is hydrogen or halogen.

In one embodiment, the present invention provides a compound of formula (I) as described herein, or a pharmaceutically acceptable salt thereof, wherein R³ is hydrogen or halogen.

In one embodiment, the present invention provides a compound of formula (I) as described herein, or a pharmaceutically acceptable salt thereof, wherein R⁴ is selected from C₁-C₆-alkoxy, halo-C₁-C₆-alkoxy, cyano-C₁-C₆-alkoxy, and carbamoyl-C₁-C₆-alkoxy.

In one embodiment, the present invention provides a compound of formula (I) as described herein, or a pharmaceutically acceptable salt thereof, wherein R⁵ is hydrogen.

In one embodiment, the present invention provides a compound of formula (I) as described herein, or a pharmaceutically acceptable salt thereof, wherein R⁶ is hydrogen or halogen.

In one embodiment, the present invention provides a compound of formula (I) as described herein, or a pharmaceutically acceptable salt thereof, wherein R⁷ is C₁-C₆-alkyl.

In one embodiment, the present invention provides a compound of formula (I) as described herein, or a pharmaceutically acceptable salt thereof, wherein R⁸ is selected from C₁-C₆-alkyl, amino-C₁-C₆-alkyl, (C₁-C₆-alkyl)₂N-C₁-C₆-alkyl-, amino-C₁-C₆-alkoxy-C₁-C₆-alkyl-, (C₁-C₆-alkyl)₂N-C₁-C₆-alkoxy-C₁-C₆-alkyl-, amino-C₁-C₆-alkyl-C(O)-NH-C₁-C₆-alkyl-, (C₁-C₆-alkyl)₂N-C₁-C₆-alkyl-C(O)-NH-C₁-C₆-alkyl-, and a group

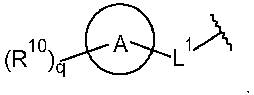

In one embodiment, the present invention provides a compound of formula (I) as described herein, or a pharmaceutically acceptable salt thereof, wherein R⁹ is hydrogen.

In one embodiment, the present invention provides a compound of formula (I) as described herein, or a pharmaceutically acceptable salt thereof, wherein R¹⁰ is selected from amino, hydroxy, C₁-C₆-alkyl, amino-C₁-C₆-alkyl-C(O)-, (C₁-C₆-alkyl)₂N-C₁-C₆-alkyl-C(O)-, amino-C₁-C₆-alkyl-NH-C(O)-, amino-C₁-C₆-alkoxy-C₁-C₆-alkyl-C(O)-, hydroxy-C₁-C₆-alkyl, and a group

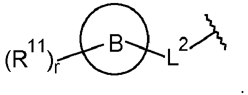

In one embodiment, the present invention provides a compound of formula (I) as described herein, or a pharmaceutically acceptable salt thereof, wherein R¹¹ is selected from hydroxy, amino, and amino-C₁-C₆-alkyl.

In one embodiment, the present invention provides a compound of formula (I) as described herein, or a pharmaceutically acceptable salt thereof, wherein A is selected from 3- to 14-membered heterocyclyl and C₃-C₁₀-cycloalkyl.

In one embodiment, the present invention provides a compound of formula (I) as described herein, or a pharmaceutically acceptable salt thereof, wherein B is selected from 3- to 14-membered heterocyclyl and C₃-C₁₀-cycloalkyl.

In one embodiment, the present invention provides a compound of formula (I) as described herein, or a pharmaceutically acceptable salt thereof, wherein L¹ is selected from a covalent bond, C₁-C₆-alkyl, -C(O)-NH-C₁-C₆-alkyl-, and -NH-C(O)-NH-C₁-C₆-alkyl-.

In one embodiment, the present invention provides a compound of formula (I) as described herein, or a pharmaceutically acceptable salt thereof, wherein L² is selected from carbonyl, C₁-C₆-alkyl, -C(O)-NH-, and -NH-C(O)-.

In one embodiment, the present invention provides a compound of formula (I) as described herein, or a pharmaceutically acceptable salt thereof, wherein p is 1.

In one embodiment, the present invention provides a compound of formula (I) as described herein, or a pharmaceutically acceptable salt thereof, wherein q is selected from 0 and 1.

In one embodiment, the present invention provides a compound of formula (I) as described herein, or a pharmaceutically acceptable salt thereof, wherein r is selected from 0 and 1.

In a preferred embodiment, the present invention provides a compound of formula (I) as described herein, or a pharmaceutically acceptable salt thereof, wherein R¹ is halogen.

In a preferred embodiment, the present invention provides a compound of formula (I) as described herein, or a pharmaceutically acceptable salt thereof, wherein R² is halogen.

In a preferred embodiment, the present invention provides a compound of formula (I) as described herein, or a pharmaceutically acceptable salt thereof, wherein R³ is halogen.

In a preferred embodiment, the present invention provides a compound of formula (I) as described herein, or a pharmaceutically acceptable salt thereof, wherein R⁴ is selected from C₁-C₆-alkoxy and halo-C₁-C₆-alkoxy.

In a preferred embodiment, the present invention provides a compound of formula (I) as described herein, or a pharmaceutically acceptable salt thereof, wherein R⁶ is hydrogen.

In a preferred embodiment, the present invention provides a compound of formula (I) as described herein, or a pharmaceutically acceptable salt thereof, wherein R⁸ is a group

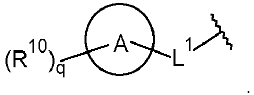

In a preferred embodiment, the present invention provides a compound of formula (I) as described herein, or a pharmaceutically acceptable salt thereof, wherein R¹⁰ is selected from amino-C₁-C₆-alkyl-C(O)-, (C₁-C₆-alkyl)₂N-C₁-C₆-alkyl-C(O)-, amino-C₁-C₆-alkoxy-C₁-C₆-alkyl-C(O)-, and a group

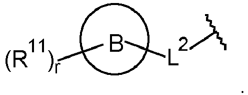

In a preferred embodiment, the present invention provides a compound of formula (I) as described herein, or a pharmaceutically acceptable salt thereof, wherein R¹¹ is selected from hydroxy and amino.

In a preferred embodiment, the present invention provides a compound of formula (I) as described herein, or a pharmaceutically acceptable salt thereof, wherein L¹ is selected from a covalent bond and C₁-C₆-alkyl.

In a preferred embodiment, the present invention provides a compound of formula (I) as described herein, or a pharmaceutically acceptable salt thereof, wherein L² is selected from carbonyl, -C(O)-NH-, and -NH-C(O)-.

In a preferred embodiment, the present invention provides a compound of formula (I) as described herein, or a pharmaceutically acceptable salt thereof, wherein q is 1.

In a particularly preferred embodiment, the present invention provides a compound of formula (I) as described herein, or a pharmaceutically acceptable salt thereof, wherein R¹ is chloro.

In a particularly preferred embodiment, the present invention provides a compound of formula (I) as described herein, or a pharmaceutically acceptable salt thereof, wherein R² is fluoro.

In a particularly preferred embodiment, the present invention provides a compound of formula (I) as described herein, or a pharmaceutically acceptable salt thereof, wherein R³ is fluoro.

In a particularly preferred embodiment, the present invention provides a compound of formula (I) as described herein, or a pharmaceutically acceptable salt thereof, wherein R⁴ is selected from methoxy and difluoromethoxy.

In a particularly preferred embodiment, the present invention provides a compound of formula (I) as described herein, or a pharmaceutically acceptable salt thereof, wherein R⁷ is methyl.

In a particularly preferred embodiment, the present invention provides a compound of formula (I) as described herein, or a pharmaceutically acceptable salt thereof, wherein R¹⁰ is selected from 2-aminoacetyl, 2-aminopropanoyl, 2-(dimethylamino)acetyl, 3-(2-aminoethoxy)propanoyl, and a group

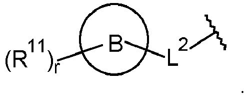

In a particularly preferred embodiment, the present invention provides a compound of formula (I) as described herein, or a pharmaceutically acceptable salt thereof, wherein A is selected from azetidinyl, cyclobutyl, pyrrolidinyl, and piperidyl.

In a particularly preferred embodiment, the present invention provides a compound of formula (I) as described herein, or a pharmaceutically acceptable salt thereof, wherein B is selected from cyclobutyl, pyrrolidinyl, and piperidyl.

In a particularly preferred embodiment, the present invention provides a compound of formula (I) as described herein, or a pharmaceutically acceptable salt thereof, wherein L¹ is selected from a covalent bond and -(CH₂)₂-.

In one embodiment, the present invention provides a compound of formula (I) as described herein, or a pharmaceutically acceptable salt thereof, wherein said compound of formula (I) is selected from:
N-[4-[2-(2-Aminoethoxy)ethylcarbamoyl]-3-chloro-phenyl]-5-(2,3-difluoro-4-methoxy-phenyl)-1 -methyl-imidazole-2-carboxamide;
N-[3-chloro-4-(2-piperazin-1-ylethylcarbamoyl)phenyl]-5-(2,3-difluoro-4-methoxy-phenyl)-1-methyl-imidazole-2-carboxamide;
N-[3-chloro-4-(pyrrolidin-3-ylcarbamoyl)phenyl]-5-(2,3-difluoro-4-methoxy-phenyl)-1-methylimidazole-2-carboxamide;
N-[3-chloro-4-(4-piperidylcarbamoyl)phenyl]-5-(2,3-difluoro-4-methoxy-phenyl)-1-methylimidazole-2-carboxamide;
N-[3-chloro-4-(4-piperidylmethylcarbamoyl)phenyl]-5-(2,3-difluoro-4-methoxy-phenyl)-1-methyl-imidazole-2-carboxamide;
N-[4-(6-aminohexylcarbamoyl)-3-chloro-phenyl]-5-(2,3-difluoro-4-methoxy-phenyl)-1-methylimidazole-2-carboxamide;
N-[4-[[3-(aminomethyl)cyclobutyl]methylcarbamoyl]-3-chloro-phenyl]-5-(2,3-difluoro-4-methoxy-phenyl)-1-methyl-imidazole-2-carboxamide;
N-[4-[(3-aminocyclobutyl)methylcarbamoyl]-3-chloro-phenyl]-5-(2,3-difluoro-4-methoxyphenyl)-1-methyl-imidazole-2-carboxamide;
N-[4-[2-(2-aminoethoxy)ethylcarbamoyl]-3-chloro-phenyl]-5-[4-(cyanomethoxy)-2,3-difluorophenyl]-1-methyl-imidazole-2-carboxamide;
N-[3-chloro-4-(2-piperazin-1-ylethylcarbamoyl)phenyl]-5-[4-(cyanomethoxy)-2,3-difluorophenyl]-1-methyl-imidazole-2-carboxamide;
N-[3-chloro-4-(3-piperazin-1-ylpropylcarbamoyl)phenyl]-5-[4-(cyanomethoxy)-2,3-difluorophenyl]-1-methyl-imidazole-2-carboxamide;
5-[4-(cyanomethoxy)-2,3-difluoro-phenyl]-1-methyl-N-[3-methyl-4-(3-piperazin-1-ylpropylcarbamoyl)phenyl]imidazole-2-carboxamide;
N-[4-[2-(2-aminoethoxy)ethylcarbamoyl]-3-ethyl-phenyl]-5-(2,3-difluoro-4-methoxy-phenyl)-1-methyl-imidazole-2-carboxamide;
N-[4-[2-(2-aminoethoxy)ethylcarbamoyl]-3-ethyl-phenyl]-5-[4-(difluoromethoxy)phenyl]-1-methyl-imidazole-2-carboxamide;
N-[4-[2-(2-aminoethoxy)ethylcarbamoyl]-3-ethyl-phenyl]-5-(3-fluoro-4-isopropoxy-phenyl)-1-methyl-imidazole-2-carboxamide;
N-[4-[2-(2-aminoethoxy)ethylcarbamoyl]-3-ethyl-phenyl]-5-(2-chloro-4-methoxy-phenyl)-1-methyl-imidazole-2-carboxamide;
N-[4-[2-(2-aminoethoxy)ethylcarbamoyl]-3-ethyl-phenyl]-5-[4-(difluoromethoxy)-2,3-difluorophenyl]-1-methyl-imidazole-2-carboxamide;
N-[4-[2-(2-aminoethoxy)ethylcarbamoyl]-3-ethyl-phenyl]-5-(2,6-difluoro-4-methoxy-phenyl)-1-methyl-imidazole-2-carboxamide;
N-[4-(3-Aminopropylcarbamoyl)-3-chloro-phenyl]-5-[4-(difluoromethoxy)-2,3-difluorophenyl]-1-methyl-imidazole-2-carboxamide;
N-[4-[2-(2-aminoethoxy)ethylcarbamoyl]-3-ethyl-phenyl]-5-[4-(cyanomethoxy)-2,3-difluorophenyl]-1-methyl-imidazole-2-carboxamide;
N-[4-[2-(2-aminoethoxy)ethylcarbamoyl]-3-ethyl-phenyl]-5-[4-(2-amino-2-oxo-ethoxy)-2,3-difluoro-phenyl]-1-methyl-imidazole-2-carboxamide;
N-[4-[2-(2-aminoethoxy)ethylcarbamoyl]-3-ethyl-phenyl]-5-[2-chloro-4-(cyanomethoxy)-3-fluoro-phenyl]-1-methyl-imidazole-2-carboxamide;
N-[3-Chloro-4-[[1-(piperidine-4-carbonyl)pyrrolidin-3-yl]carbamoyl]phenyl]-5-(2,3-difluoro-4-methoxy-phenyl)-1-methyl-imidazole-2-carboxamide;
N-[3-Chloro-4-[[1-[2-(dimethylamino)acetyl]pyrrolidin-3-yl]carbamoyl]phenyl]-5-(2,3-difluoro-4-methoxy-phenyl)-1 -methyl-imidazole-2-carboxamide;
N-[3-chloro-4-[2-[[2-(dimethylamino)acetyl]amino]ethylcarbamoyl]phenyl]-5-(2,3-difluoro-4-methoxy-phenyl)-1-methyl-imidazole-2-carboxamide;
N-[3-chloro-4-[[1-[2-(dimethylamino)acetyl]-4-piperidyl]carbamoyl]phenyl]-5-(2,3-difluoro-4-methoxy-phenyl)-1-methyl-imidazole-2-carboxamide;
N-[3-chloro-4-[5-(dimethylamino)pentylcarbamoyl]phenyl]-5-(2,3-difluoro-4-methoxy-phenyl)-1-methyl-imidazole-2-carboxamide;
N-[3-chloro-4-(2-isopentyloxyethylcarbamoyl)phenyl]-5-[4-(cyanomethoxy)-2,3-difluorophenyl]-1-methyl-imidazole-2-carboxamide;
N-[3-chloro-4-[2-(3-hydroxypyrrolidin-1-yl)ethylcarbamoyl]phenyl]-5-(2,3-difluoro-4-methoxyphenyl)-1-methyl-imidazole-2-carboxamide;
N-[3-chloro-4-[2-[3-(hydroxymethyl)pyrrolidin-1-yl]ethylcarbamoyl]phenyl]-5-(2,3-difluoro-4-methoxy-phenyl)-1-methyl-imidazole-2-carboxamide;
5-(2,3-difluoro-4-methoxy-phenyl)-N-[4-[3-[2-(dimethylamino)ethoxy]propylcarbamoyl]-3-ethyl-phenyl]-1-methyl-imidazole-2-carboxamide;
N-[3-chloro-4-[[1-[(3R)-pyrrolidine-3-carbonyl]pyrrolidin-3-yl]carbamoyl]phenyl]-5-(2,3-difluoro-4-methoxy-phenyl)-1-methyl-imidazole-2-carboxamide;
N-[3-chloro-4-[[1-[(2S,4R)-4-hydroxypyrrolidine-2-carbonyl]pyrrolidin-3-yl]carbamoyl]phenyl]-5-(2,3-difluoro-4-methoxy-phenyl)-1-methyl-imidazole-2-carboxamide;
N-[4-[[1-[3-(2-aminoethoxy)propanoyl]pyrrolidin-3-yl]carbamoyl]-3-chloro-phenyl]-5-(2,3-difluoro-4-methoxy-phenyl)-1-methyl-imidazole-2-carboxamide;
N-[3-chloro-4-[[1-(piperidine-4-carbonyl)-4-piperidyl]carbamoyl]phenyl]-5-(2,3-difluoro-4-methoxy-phenyl)-1-methyl-imidazole-2-carboxamide;
N-[3-chloro-4-[[1-[(3R)-pyrrolidine-3-carbonyl]-4-piperidyl]carbamoyl]phenyl]-5-(2,3-difluoro-4-methoxy-phenyl)-1 -methyl-imidazole-2-carboxamide;
N-[3-chloro-4-[[1-[(2S,4R)-4-hydroxypyrrolidine-2-carbonyl]-4-piperidyl]carbamoyl]phenyl]-5-(2,3-difluoro-4-methoxy-phenyl)-1-methyl-imidazole-2-carboxamide; N-[4-[[1-[3-(2-aminoethoxy)propanoyl]-4-piperidyl]carbamoyl]-3-chloro-phenyl]-5-(2,3-difluoro-4-methoxy-phenyl)-1-methyl-imidazole-2-carboxamide;
N-[4-[[3-[[3-(aminomethyl)cyclobutanecarbonyl]amino]cyclobutyl]carbamoyl]-3-chlorophenyl]-5-(2,3-difluoro-4-methoxy-phenyl)-1-methyl-imidazole-2-carboxamide;
N-[4-[2-[1-(3-aminopropanoyl)azetidin-3-yl]ethylcarbamoyl]-3-chloro-phenyl]-5-[4-(difluoromethoxy)-2,3-difluoro-phenyl]-1-methyl-imidazole-2-carboxamide;
N-[4-[3-(3-aminopropanoylamino)propylcarbamoyl]-3-chloro-phenyl]-5-[4-(difluoromethoxy)-2,3-difluoro-phenyl]-1-methyl-imidazole-2-carboxamide;
N-[3-[[4-[[5-[4-(difluoromethoxy)-2,3-difluoro-phenyl]-1-methyl-imidazole-2-carbonyl]amino]-2-ethyl-benzoyl]amino]propyl]piperidine-4-carboxamide;
N-[2-[[4-[[5-[4-(cyanomethoxy)-2,3-difluoro-phenyl]-1-methyl-imidazole-2-carbonyl]amino]-2-methyl-benzoyl] amino] ethyl] piperidine-4-carboxamide;
N-[2-[[4-[[5-[4-(cyanomethoxy)-2,3-difluoro-phenyl]-1-methyl-imidazole-2-carbonyl]amino]-2-methyl-benzoyl]amino]ethyl]-4-hydroxy-piperidine-4-carboxamide;
N-[2-[[2-chloro-4-[[5-[4-(cyanomethoxy)-2,3-difluoro-phenyl]-1-methyl-imidazole-2-carbonyl] amino]benzoyl] amino] ethyl]piperidine-4-carboxamide;
N-[2-[[2-chloro-4-[[5-[4-(cyanomethoxy)-2,3-difluoro-phenyl]-1-methyl-imidazole-2-carbonyl]amino]benzoyl]amino]ethyl]-4-hydroxy-piperidine-4-carboxamide;
N-[4-[[1-(2-aminoacetyl)azetidin-3-yl]carbamoyl]-3-chloro-phenyl]-5-(2,3-difluoro-4-methoxyphenyl)-1-methyl-imidazole-2-carboxamide;
N-[4-[[1-(2-aminoacetyl)azetidin-3-yl]methylcarbamoyl]-3-chloro-phenyl]-5-(2,3-difluoro-4-methoxy-phenyl)-1-methyl-imidazole-2-carboxamide;
N-[4-[[3-(2-aminoethylcarbamoyl)cyclobutyl]carbamoyl]-3-chloro-phenyl]-5-(2,3-difluoro-4-methoxy-phenyl)-1-methyl-imidazole-2-carboxamide;
N-[4-[[3-(3-aminoazetidine-1-carbonyl)cyclobutyl]carbamoyl]-3-chloro-phenyl]-5-(2,3-difluoro-4-methoxy-phenyl)-1-methyl-imidazole-2-carboxamide;
N-[4-[[3-[(3-aminocyclobutyl)carbamoyl]cyclobutyl]carbamoyl]-3-chloro-phenyl]-5-(2,3-difluoro-4-methoxy-phenyl)-1-methyl-imidazole-2-carboxamide;
N-[4-[[3-(2-aminoethylcarbamoyl)cyclobutyl]carbamoyl]-3-ethyl-phenyl]-5-(2,3-difluoro-4-methoxy-phenyl)-1-methyl-imidazole-2-carboxamide;
N-[4-[[1-(2-aminoacetyl)azetidin-3-yl]carbamoyl]-3-ethyl-phenyl]-5-(2,3-difluoro-4-methoxyphenyl)-1-methyl-imidazole-2-carboxamide;
5-[4-(cyanomethoxy)-2,3-difluoro-phenyl]-N-[4-[2-[[(2R,4R)-4-hydroxy-4-methyl-pyrrolidine-2-carbonyl]amino]ethylcarbamoyl]-3-methyl-phenyl]-1-methyl-imidazole-2-carboxamide;
5-[4-(cyanomethoxy)-2,3-difluoro-phenyl]-N-[4-[2-[[(2R,4R)-4-ethyl-4-hydroxy-pyrrolidine-2-carbonyl] amino] ethylcarbamoyl]-3-methyl-phenyl]-1-methyl-imidazole-2-carboxamide;
5-(2,3-difluoro-4-methoxy-phenyl)-N-[4-[2-[[(2R,4R)-4-hydroxy-4-methyl-pyrrolidine-2-carbonyl] amino] ethylcarbamoyl]-3-methyl-phenyl]-1-methyl-imidazole-2-carboxamide;
5-(2,3-difluoro-4-methoxy-phenyl)-N-[4-[2-[[(2R,4R)-4-ethyl-4-hydroxy-pyrrolidine-2-carbonyl] amino] ethylcarbamoyl]-3-methyl-phenyl]-1-methyl-imidazole-2-carboxamide;
5-(2,3-difluoro-4-methoxy-phenyl)-N-[4-[2-[[(3R,4R)-4-hydroxypyrrolidin-3-yl] carbamoylamino] ethylcarbamoyl]-3-methyl-phenyl]-1-methyl-imidazole-2-carboxamide;
5-(2,3-difluoro-4-methoxy-phenyl)-N-[4-[2-[[(3S,4S)-4-hydroxypyrrolidin-3-yl]carbamoylamino]ethylcarbamoyl]-3-methyl-phenyl]-1-methyl-imidazole-2-carboxamide;
5-[4-(cyanomethoxy)-2,3-difluoro-phenyl]-N-[4-[2-[[(3R,4R)-4-hydroxypyrrolidin-3-yl]carbamoylamino]ethylcarbamoyl]-3-methyl-phenyl]-1-methyl-imidazole-2-carboxamide;
5-[4-(cyanomethoxy)-2,3-difluoro-phenyl]-N-[4-[2-[[(3S,4S)-4-hydroxypyrrolidin-3-yl]carbamoylamino]ethylcarbamoyl]-3-methyl-phenyl]-1-methyl-imidazole-2-carboxamide;
(3R,4R)-N-[2-[[2-chloro-4-[[5-[4-(cyanomethoxy)-2,3-difluoro-phenyl]-1-methyl-imidazole-2-carbonyl]amino]benzoyl]amino]ethyl]-3-hydroxy-piperidine-4-carboxamide;
(3S,4S)-N-[2-[[2-chloro-4-[[5-[4-(cyanomethoxy)-2,3-difluoro-phenyl]-1-methyl-imidazole-2-carbonyl]amino]benzoyl]amino]ethyl]-3-hydroxy-piperidine-4-carboxamide;
(3S,4R)-N-[2-[[2-chloro-4-[[5-[4-(cyanomethoxy)-2,3-difluoro-phenyl]-1-methyl-imidazole-2-carbonyl]amino]benzoyl]amino]ethyl]-3-hydroxy-piperidine-4-carboxamide;
(3R,4S)-N-[2-[[2-chloro-4-[[5-[4-(cyanomethoxy)-2,3-difluoro-phenyl]-1-methyl-imidazole-2-carbonyl]amino]benzoyl]amino]ethyl]-3-hydroxy-piperidine-4-carboxamide;
N-[3-chloro-4-[2-[[(2R,4R)-4-hydroxy-4-methyl-pyrrolidine-2-carbonyl]amino]ethylcarbamoyl]phenyl]-5-[4-(cyanomethoxy)-2,3-difluoro-phenyl]-1-methyl-imidazole-2-carboxamide;
N-[3-chloro-4-[2-[[(2R,4R)-4-ethyl-4-hydroxy-pyrrolidine-2-carbonyl]amino]ethylcarbamoyl]phenyl]-5-[4-(cyanomethoxy)-2,3-difluoro-phenyl]-1-methyl-imidazole-2-carboxamide;
N-[3-chloro-4-[[3-[[(4R)-4-hydroxypyrrolidine-2-carbonyl]amino]cyclobutyl]carbamoyl]phenyl]-5-(2,3-difluoro-4-methoxy-phenyl)-1-methylimidazole-2-carboxamide;
N-[3-[[2-chloro-4-[[5-(2,3-difluoro-4-methoxy-phenyl)-1-methyl-imidazole-2-carbonyl] amino]benzoyl] amino] cyclobutyl]piperidine-4-carboxamide;
N-[3-[[2-chloro-4-[[5-(2,3-difluoro-4-methoxy-phenyl)-1-methyl-imidazole-2-carbonyl] amino]benzoyl] amino] cyclobutyl]-4-hydroxy-piperidine-4-carboxamide;
(3R,4R)-N-[3-[[2-chloro-4-[[5-(2,3-difluoro-4-methoxy-phenyl)-1-methyl-imidazole-2-carbonyl]amino]benzoyl]amino]cyclobutyl]-3-hydroxy-piperidine-4-carboxamide;
(3S,4S)-N-[3-[[2-chloro-4-[[5-(2,3-difluoro-4-methoxy-phenyl)-1-methyl-imidazole-2-carbonyl]amino]benzoyl]amino]cyclobutyl]-3-hydroxy-piperidine-4-carboxamide;
N-[4-[2-[1-(Azetidin-3-ylmethyl)azetidin-3-yl]ethylcarbamoyl]-3-chloro-phenyl]-5-[4-(difluoromethoxy)-2,3-difluoro-phenyl]-1-methyl-imidazole-2-carboxamide;
N-[3-[[4-[[5-[4-(difluoromethoxy)-2,3-difluoro-phenyl]-1-methyl-imidazole-2-carbonyl]amino]-2-ethyl-benzoyl]amino]propyl]-1-(pyrrolidin-3-ylmethyl)piperidine-4-carboxamide;
1-(azetidin-3-ylmethyl)-N-[3-[[4-[[5-[4-(difluoromethoxy)-2,3-difluoro-phenyl]-1-methylimidazole-2-carbonyl]amino]-2-ethyl-benzoyl]amino]propyl]piperidine-4-carboxamide;
N-[4-[[1-(azetidin-3-ylmethyl)-4-piperidyl]methylcarbamoyl]-3-chloro-phenyl]-5-(2,3-difluoro-4-methoxy-phenyl)-1 -methyl-imidazole-2-carboxamide;
5-(4-Methoxyphenyl)-1-methyl-N-[3-methyl-4-(methylcarbamoyl)phenyl] imidazole-2-carboxamide;
N-[3-chloro-4-[[(exo)-3-[(2S,4R)-4-hydroxyprolyl]-3-azabicyclo[3.1.0]hexan-6-yl] carbamoyl]phenyl]-5-(2,3-difluoro-4-methoxy-phenyl)-1-methyl-imidazole-2-carboxamide; and
(exo)-6-[[2-chloro-4-[[5-(2,3-difluoro-4-methoxy-phenyl)-1-methyl-imidazole-2-carbonyl]amino]benzoyl]amino]-N-[(trans)-4-hydroxypyrrolidin-3-yl]-3-azabicyclo[3.1.0]hexane-3-carboxamide.

In a preferred embodiment, the present invention provides a compound of formula (I) as described herein, or a pharmaceutically acceptable salt thereof, wherein said compound of formula (I) is selected from:
N-[3-chloro-4-[[1-[2-(dimethylamino)acetyl]-4-piperidyl]carbamoyl]phenyl]-5-(2,3-difluoro-4-methoxy-phenyl)-1-methyl-imidazole-2-carboxamide;
N-[3-chloro-4-[[1-[(3R)-pyrrolidine-3-carbonyl]pyrrolidin-3-yl]carbamoyl]phenyl]-5-(2,3-difluoro-4-methoxy-phenyl)-1-methyl-imidazole-2-carboxamide;;
N-[4-[[1-[3-(2-aminoethoxy)propanoyl]pyrrolidin-3-yl]carbamoyl]-3-chloro-phenyl]-5-(2,3-difluoro-4-methoxy-phenyl)-1-methyl-imidazole-2-carboxamide;
N-[3-chloro-4-[[1-(piperidine-4-carbonyl)-4-piperidyl]carbamoyl]phenyl]-5-(2,3-difluoro-4-methoxy-phenyl)-1-methyl-imidazole-2-carboxamide;
N-[3-chloro-4-[[1-[(2S,4R)-4-hydroxypyrrolidine-2-carbonyl]-4-piperidyl]carbamoyl]phenyl]-5-(2,3-difluoro-4-methoxy-phenyl)-1-methyl-imidazole-2-carboxamide;
N-[4-[2-[1-(3-aminopropanoyl)azetidin-3-yl]ethylcarbamoyl]-3-chloro-phenyl]-5-[4-(difluoromethoxy)-2,3-difluoro-phenyl]-1-methyl-imidazole-2-carboxamide;
N-[4-[[1-(2-aminoacetyl)azetidin-3-yl]carbamoyl]-3-chloro-phenyl]-5-(2,3-difluoro-4-methoxyphenyl)-1-methyl-imidazole-2-carboxamide;
N-[4-[[3-[(3-aminocyclobutyl)carbamoyl]cyclobutyl]carbamoyl]-3-chloro-phenyl]-5-(2,3-difluoro-4-methoxy-phenyl)-1-methyl-imidazole-2-carboxamide;
N-[3-chloro-4-[[3-[[(4R)-4-hydroxypyrrolidine-2-carbonyl]amino]cyclobutyl]carbamoyl]phenyl]-5-(2,3-difluoro-4-methoxy-phenyl)-1-methylimidazole-2-carboxamide;
N-[3-[[2-chloro-4-[[5-(2,3-difluoro-4-methoxy-phenyl)-1-methyl-imidazole-2-carbonyl]amino]benzoyl]amino]cyclobutyl]piperidine-4-carboxamide; and
(3S,4S)-N-[3-[[2-chloro-4-[[5-(2,3-difluoro-4-methoxy-phenyl)-1-methyl-imidazole-2-carbonyl]amino]benzoyl]amino]cyclobutyl]-3-hydroxy-piperidine-4-carboxamide.

In one embodiment, the present invention provides a compound of formula (I) as described herein, or a pharmaceutically acceptable salt thereof, wherein said compound of formula (I) is selected from:
Example A1
   N-[4-[2-(2-Aminoethoxy)ethylcarbamoyl]-3-chloro-phenyl]-5-(2,3-difluoro-4-methoxy-phenyl)-1-methyl-imidazole-2-carboxamide
Example A2
   N-[3-chloro-4-(2-piperazin-1-ylethylcarbamoyl)phenyl]-5-(2,3-difluoro-4-methoxy-phenyl)-1-methyl-imidazole-2-carboxamide
Example A3
   N-[3-chloro-4-(pyrrolidin-3-ylcarbamoyl)phenyl]-5-(2,3-difluoro-4-methoxy-phenyl)-1-methylimidazole-2-carboxamide;formic acid
Example A4
   N-[3-chloro-4-(4-piperidylcarbamoyl)phenyl]-5-(2,3-difluoro-4-methoxy-phenyl)-1-methylimidazole-2-carboxamide;formic acid
Example A5
   N-[3-chloro-4-(4-piperidylmethylcarbamoyl)phenyl]-5-(2,3-difluoro-4-methoxy-phenyl)-1-methyl-imidazole-2-carboxamide;formic acid
Example A6
   N-[4-(6-aminohexylcarbamoyl)-3-chloro-phenyl]-5-(2,3-difluoro-4-methoxy-phenyl)-1-methylimidazole-2-carboxamide
Example A7
   N-[4-[[3-(aminomethyl)cyclobutyl]methylcarbamoyl]-3-chloro-phenyl]-5-(2,3-difluoro-4-methoxy-phenyl)-1 -methyl-imidazole-2-carboxamide;formic acid
Example A8
   N-[4-[(3-aminocyclobutyl)methylcarbamoyl]-3-chloro-phenyl]-5-(2,3-difluoro-4-methoxyphenyl)-1-methyl-imidazole-2-carboxamide;formic acid
Example A9
   N-[4-[2-(2-aminoethoxy)ethylcarbamoyl]-3-chloro-phenyl]-5-[4-(cyanomethoxy)-2,3-difluorophenyl]-1-methyl-imidazole-2-carboxamide;formic acid
Example A10
   N-[3-chloro-4-(2-piperazin-1-ylethylcarbamoyl)phenyl]-5-[4-(cyanomethoxy)-2,3-difluorophenyl]-1-methyl-imidazole-2-carboxamide;2,2,2-trifluoroacetic acid
Example A11
   N-[3-chloro-4-(3-piperazin-1-ylpropylcarbamoyl)phenyl]-5-[4-(cyanomethoxy)-2,3-difluorophenyl]-1-methyl-imidazole-2-carboxamide;2,2,2-trifluoroacetic acid
Example A12
   5-[4-(cyanomethoxy)-2,3-difluoro-phenyl]-1-methyl-N-[3-methyl-4-(3-piperazin-1-ylpropylcarbamoyl)phenyl]imidazole-2-carboxamide;2,2,2-trifluoroacetic acid
Example A13
   N-[4-[2-(2-aminoethoxy)ethylcarbamoyl]-3-ethyl-phenyl]-5-(2,3-difluoro-4-methoxy-phenyl)-1-methyl-imidazole-2-carboxamide;formic acid
Example A14
   N-[4-[2-(2-aminoethoxy)ethylcarbamoyl]-3-ethyl-phenyl]-5-[4-(difluoromethoxy)phenyl]-1-methyl-imidazole-2-carboxamide;hydrochloride
Example A15
   N-[4-[2-(2-aminoethoxy)ethylcarbamoyl]-3-ethyl-phenyl]-5-(3-fluoro-4-isopropoxy-phenyl)-1-methyl-imidazole-2-carboxamide;hydrochloride
Example A16
   N-[4-[2-(2-aminoethoxy)ethylcarbamoyl]-3-ethyl-phenyl]-5-(2-chloro-4-methoxy-phenyl)-1-methyl-imidazole-2-carboxamide;hydrochloride
Example A17
   N-[4-[2-(2-aminoethoxy)ethylcarbamoyl]-3-ethyl-phenyl]-5-[4-(difluoromethoxy)-2,3-difluorophenyl]-1-methyl-imidazole-2-carboxamide;hydrochloride
Example A18
   N-[4-[2-(2-aminoethoxy)ethylcarbamoyl]-3-ethyl-phenyl]-5-(2,6-difluoro-4-methoxy-phenyl)-1-methyl-imidazole-2-carboxamide;hydrochloride
Example A19
   N-[4-(3-Aminopropylcarbamoyl)-3-chloro-phenyl]-5-[4-(difluoromethoxy)-2,3-difluorophenyl]-1-methyl-imidazole-2-carboxamide
Example A20
   N-[4-[2-(2-aminoethoxy)ethylcarbamoyl]-3-ethyl-phenyl]-5-[4-(cyanomethoxy)-2,3-difluorophenyl]-1-methyl-imidazole-2-carboxamide;hydrochloride
Example A21
   N-[4-[2-(2-aminoethoxy)ethylcarbamoyl]-3-ethyl-phenyl]-5-[4-(2-amino-2-oxo-ethoxy)-2,3-difluoro-phenyl]-1-methyl-imidazole-2-carboxamide;hydrochloride
Example A22
   N-[4-[2-(2-aminoethoxy)ethylcarbamoyl]-3-ethyl-phenyl]-5-[2-chloro-4-(cyanomethoxy)-3 fluoro-phenyl]-1-methyl-imidazole-2-carboxamide;hydrochlorideExample B1
   N-[3-Chloro-4-[[1-(piperidine-4-carbonyl)pyrrolidin-3-yl]carbamoyl]phenyl]-5-(2,3-difluoro-4-methoxy-phenyl)-1-methyl-imidazole-2-carboxamide;formic acid
Example A23
   N-[3-Chloro-4-[[1-[2-(dimethylamino)acetyl]pyrrolidin-3-yl]carbamoyl]phenyl]-5-(2,3-difluoro-4-methoxy-phenyl)-1-methyl-imidazole-2-carboxamide
Example A24
   N-[3-chloro-4-[2-[[2-(dimethylamino)acetyl]amino]ethylcarbamoyl]phenyl]-5-(2,3-difluoro-4-methoxy-phenyl)-1-methyl-imidazole-2-carboxamide;2,2,2-trifluoroacetic acid
Example A25
   N-[3-chloro-4-[[1-[2-(dimethylamino)acetyl]-4-piperidyl]carbamoyl]phenyl]-5-(2,3-difluoro-4-methoxy-phenyl)-1-methyl-imidazole-2-carboxamide
Example A26
   N-[3-chloro-4-[5-(dimethylamino)pentylcarbamoyl]phenyl]-5-(2,3-difluoro-4-methoxy-phenyl)-1-methyl-imidazole-2-carboxamide
Example A27
   N-[3-chloro-4-(2-isopentyloxyethylcarbamoyl)phenyl]-5-[4-(cyanomethoxy)-2,3-difluorophenyl]-1-methyl-imidazole-2-carboxamide;formic acid
Example A28
   N-[3-chloro-4-[2-(3-hydroxypyrrolidin-1-yl)ethylcarbamoyl]phenyl]-5-(2,3-difluoro-4-methoxyphenyl)-1-methyl-imidazole-2-carboxamide
Example A29
   N-[3-chloro-4-[2-[3-(hydroxymethyl)pyrrolidin-1-yl]ethylcarbamoyl]phenyl]-5-(2,3-difluoro-4-methoxy-phenyl)-1-methyl-imidazole-2-carboxamide
Example A30
   5-(2,3-difluoro-4-methoxy-phenyl)-N-[4-[3-[2-(dimethylamino)ethoxy]propylcarbamoyl]-3-ethyl-phenyl]-1-methyl-imidazole-2-carboxamide
Example B2
   N-[3-chloro-4-[[1-[(3R)-pyrrolidine-3-carbonyl]pyrrolidin-3-yl]carbamoyl]phenyl]-5-(2,3-difluoro-4-methoxy-phenyl)-1-methyl-imidazole-2-carboxamide;formic acid
Example B3
   N-[3-chloro-4-[[1-[(2S,4R)-4-hydroxypyrrolidine-2-carbonyl]pyrrolidin-3-yl] carbamoyl]phenyl]-5-(2,3-difluoro-4-methoxy-phenyl)-1-methyl-imidazole-2-carboxamide; formic acid
Example B4
   N-[4-[[1-[3-(2-aminoethoxy)propanoyl]pyrrolidin-3-yl]carbamoyl]-3-chloro-phenyl]-5-(2,3-difluoro-4-methoxy-phenyl)-1-methyl-imidazole-2-carboxamide;formic acid
Example B5
   N-[3-chloro-4-[[1-(piperidine-4-carbonyl)-4-piperidyl]carbamoyl]phenyl]-5-(2,3-difluoro-4-methoxy-phenyl)-1 -methyl-imidazole-2-carboxamide;formic acid
Example B6
   N-[3-chloro-4-[[1-[(3R)-pyrrolidine-3-carbonyl]-4-piperidyl]carbamoyl]phenyl]-5-(2,3-difluoro-4-methoxy-phenyl)-1-methyl-imidazole-2-carboxamide;formic acid
Example B7
   N-[3-chloro-4-[[1-[(2S,4R)-4-hydroxypyrrolidine-2-carbonyl]-4-piperidyl]carbamoyl]phenyl]-5-(2,3-difluoro-4-methoxy-phenyl)-1-methyl-imidazole-2-carboxamide
Example B8
   N-[4-[[1-[3-(2-aminoethoxy)propanoyl]-4-piperidyl]carbamoyl]-3-chloro-phenyl]-5-(2,3-difluoro-4-methoxy-phenyl)-1-methyl-imidazole-2-carboxamide;formic acid
Example B9
   N-[4-[[3-[[3-(aminomethyl)cyclobutanecarbonyl]amino]cyclobutyl]carbamoyl]-3-chlorophenyl]-5-(2,3-difluoro-4-methoxy-phenyl)-1-methyl-imidazole-2-carboxamide;formic acid
Example B10
   N-[4-[2-[1-(3-aminopropanoyl)azetidin-3-yl]ethylcarbamoyl]-3-chloro-phenyl]-5-[4-(difluoromethoxy)-2,3-difluoro-phenyl]-1-methyl-imidazole-2-carboxamide;2,2,2-trifluoroacetic acid
Example B11
   N-[4-[3-(3-aminopropanoylamino)propylcarbamoyl]-3-chloro-phenyl]-5-[4-(difluoromethoxy)-2,3-difluoro-phenyl]-1-methyl-imidazole-2-carboxamide;2,2,2-trifluoroacetic acid
Example B12
   N-[3-[[4-[[5-[4-(difluoromethoxy)-2,3-difluoro-phenyl]-1-methyl-imidazole-2-carbonyl]amino]-2-ethyl-benzoyl]amino]propyl]piperidine-4-carboxamide;formic acid
Example B13
   N-[2-[[4-[[5-[4-(cyanomethoxy)-2,3-difluoro-phenyl]-1-methyl-imidazole-2-carbonyl]amino]-2-methyl-benzoyl]amino]ethyl]piperidine-4-carboxamide;formic acid
Example B14
   N-[2-[[4-[[5-[4-(cyanomethoxy)-2,3-difluoro-phenyl]-1-methyl-imidazole-2-carbonyl]amino]-2-methyl-benzoyl]amino]ethyl]-4-hydroxy-piperidine-4-carboxamide;formic acid
Example B15
   N-[2-[[2-chloro-4-[[5-[4-(cyanomethoxy)-2,3-difluoro-phenyl]-1-methyl-imidazole-2-carbonyl]amino]benzoyl]amino]ethyl]piperidine-4-carboxamide;2,2,2-trifluoroacetic acid
Example B16
   N-[2-[[2-chloro-4-[[5-[4-(cyanomethoxy)-2,3-difluoro-phenyl]-1-methyl-imidazole-2-carbonyl]amino]benzoyl]amino]ethyl]-4-hydroxy-piperidine-4-carboxamide;2,2,2-trifluoroacetic acid
Example B17
   N-[4-[[1-(2-aminoacetyl)azetidin-3-yl]carbamoyl]-3-chloro-phenyl]-5-(2,3-difluoro-4-methoxyphenyl)-1-methyl-imidazole-2-carboxamide;formic acid
Example B18
   N-[4-[[1-(2-aminoacetyl)azetidin-3-yl]methylcarbamoyl]-3-chloro-phenyl]-5-(2,3-difluoro-4-methoxy-phenyl)-1 -methyl-imidazole-2-carboxamide;formic acid
Example B19
   N-[4-[[3-(2-aminoethylcarbamoyl)cyclobutyl]carbamoyl]-3-chloro-phenyl]-5-(2,3-difluoro-4-methoxy-phenyl)-1 -methyl-imidazole-2-carboxamide;formic acid
Example B20
   N-[4-[[3-(3-aminoazetidine-1-carbonyl)cyclobutyl]carbamoyl]-3-chloro-phenyl]-5-(2,3-difluoro-4-methoxy-phenyl)-1-methyl-imidazole-2-carboxamide;formic acid
Example B21
   N-[4-[[3-[(3-aminocyclobutyl)carbamoyl]cyclobutyl]carbamoyl]-3-chloro-phenyl]-5-(2,3-difluoro-4-methoxy-phenyl)-1-methyl-imidazole-2-carboxamide;2,2,2-trifluoroacetic acid
Example B22
   N-[4-[[3-(2-aminoethylcarbamoyl)cyclobutyl]carbamoyl]-3-ethyl-phenyl]-5-(2,3-difluoro-4-methoxy-phenyl)-1-methyl-imidazole-2-carboxamide;2,2,2-trifluoroacetic acid
Example B23
   N-[4-[[1-(2-aminoacetyl)azetidin-3-yl]carbamoyl]-3-ethyl-phenyl]-5-(2,3-difluoro-4-methoxyphenyl)-1-methyl-imidazole-2-carboxamide;formic acid
Example B24
   5-[4-(cyanomethoxy)-2,3-difluoro-phenyl]-N-[4-[2-[[(2R,4R)-4-hydroxy-4-methyl-pyrrolidine-2-carbonyl]amino]ethylcarbamoyl]-3-methyl-phenyl]-1-methyl-imidazole-2-carboxamide; formic acid
Example B25
   5-[4-(cyanomethoxy)-2,3-difluoro-phenyl]-N-[4-[2-[[(2R,4R)-4-ethyl-4-hydroxy-pyrrolidine-2-carbonyl] amino] ethylcarbamoyl]-3-methyl-phenyl]-1-methyl-imidazole-2-carboxamide; formic acid
Example B26
   5-(2,3-difluoro-4-methoxy-phenyl)-N-[4-[2-[[(2R,4R)-4-hydroxy-4-methyl-pyrrolidine-2-carbonyl] amino] ethylcarbamoyl]-3-methyl-phenyl]-1-methyl-imidazole-2-carboxamide; formic acid
Example B27
   5-(2,3-difluoro-4-methoxy-phenyl)-N-[4-[2-[[(2R,4R)-4-ethyl-4-hydroxy-pyrrolidine-2-carbonyl] amino] ethylcarbamoyl]-3-methyl-phenyl]-1-methyl-imidazole-2-carboxamide; formic acid
Example B28
   5-(2,3-difluoro-4-methoxy-phenyl)-N-[4-[2-[[(3R,4R)-4-hydroxypyrrolidin-3-yl]carbamoylamino]ethylcarbamoyl]-3-methyl-phenyl]-1-methyl-imidazole-2-carboxamide; formic acid
Example B29
   5-(2,3-difluoro-4-methoxy-phenyl)-N-[4-[2-[[(3S,4S)-4-hydroxypyrrolidin-3-yl]carbamoylamino]ethylcarbamoyl]-3-methyl-phenyl]-1-methyl-imidazole-2-carboxamide; formic acid
Example B30
   5-[4-(cyanomethoxy)-2,3-difluoro-phenyl]-N-[4-[2-[[(3R,4R)-4-hydroxypyrrolidin-3-yl]carbamoylamino]ethylcarbamoyl]-3-methyl-phenyl]-1-methyl-imidazole-2-carboxamide; formic acid
Example B31
   5-[4-(cyanomethoxy)-2,3-difluoro-phenyl]-N-[4-[2-[[(3S,4S)-4-hydroxypyrrolidin-3-yl]carbamoylamino]ethylcarbamoyl]-3-methyl-phenyl]-1-methyl-imidazole-2-carboxamide; formic acid
Example B32
   (3R,4R)-N-[2-[[2-chloro-4-[[5-[4-(cyanomethoxy)-2,3-difluoro-phenyl]-1-methyl-imidazole-2-carbonyl]amino]benzoyl]amino]ethyl-3-hydroxy-piperidine-4-carboxamide;formic acid
Example B33
   (3S,4S)-N-[2-[[2-chloro-4-[[5-[4-(cyanomethoxy)-2,3-difluoro-phenyl]-1-methyl-imidazole-2-carbonyl]amino]benzoyl]amino]ethyl-3-hydroxy-piperidine-4-carboxamide;formic acid
Example B34
   (3S,4R)-N-[2-[[2-chloro-4-[[5-[4-(cyanomethoxy)-2,3-difluoro-phenyl]-1-methyl-imidazole-2-carbonyl]amino]benzoyl]amino]ethyl-3-hydroxy-piperidine-4-carboxamide;formic acid
Example B35
   (3R,4S)-N-[2-[[2-chloro-4-[[5-[4-(cyanomethoxy)-2,3-difluoro-phenyl]-1-methyl-imidazole-2-carbonyl]amino]benzoyl]amino]ethyl-3-hydroxy-piperidine-4-carboxamide;formic acid
Example B36
   N-[3-chloro-4-[2-[[(2R,4R)-4-hydroxy-4-methyl-pyrrolidine-2-carbonyl]amino]ethylcarbamoyl]phenyl]-5-[4-(cyanomethoxy)-2,3-difluoro-phenyl]-1-methyl-imidazole-2-carboxamide;formic acid
Example B37
   N-[3-chloro-4-[2-[[(2R,4R)-4-ethyl-4-hydroxy-pyrrolidine-2-carbonyl]amino]ethylcarbamoyl]phenyl]-5-[4-(cyanomethoxy)-2,3-difluoro-phenyl]-1-methyl-imidazole-2-carboxamide;formic acid
Example B38
   N-[3-chloro-4-[[3-[[(4R)-4-hydroxypyrrolidine-2-carbonyl]amino]cyclobutyl]carbamoyl]phenyl]-5-(2,3-difluoro-4-methoxy-phenyl)-1-methylimidazole-2-carboxamide;formic acid
Example B39
   N-[3-[[2-chloro-4-[[5-(2,3-difluoro-4-methoxy-phenyl)-1-methyl-imidazole-2-carbonyl]amino]benzoyl]amino]cyclobutyl]piperidine-4-carboxamide;formic acid
Example B40
   N-[3-[[2-chloro-4-[[5-(2,3-difluoro-4-methoxy-phenyl)-1-methyl-imidazole-2-carbonyl]amino]benzoyl]amino]cyclobutyl]-4-hydroxy-piperidine-4-carboxamide;formic acid
Example B41
   (3R,4R)-N-[3-[[2-chloro-4-[[5-(2,3-difluoro-4-methoxy-phenyl)-1-methyl-imidazole-2-carbonyl]amino]benzoyl]amino]cyclobutyl]-3-hydroxy-piperidine-4-carboxamide;formic acid
Example B42
   (3S,4S)-N-[3-[[2-chloro-4-[[5-(2,3-difluoro-4-methoxy-phenyl)-1-methyl-imidazole-2-carbonyl]amino]benzoyl]amino]cyclobutyl]-3-hydroxy-piperidine-4-carboxamide;formic acid
Example C1
   N-[4-[2-[1-(Azetidin-3-ylmethyl)azetidin-3-yl]ethylcarbamoyl]-3-chloro-phenyl]-5-[4-(difluoromethoxy)-2,3-difluoro-phenyl]-1-methyl-imidazole-2-carboxamide;2,2,2-trifluoroacetic acid
Example C2
   N-[3-[[4-[[5-[4-(difluoromethoxy)-2,3-difluoro-phenyl]-1-methyl-imidazole-2-carbonyl]amino]-2-ethyl-benzoyl]amino]propyl]-1-(pyrrolidin-3-ylmethyl)piperidine-4-carboxamide;formic acid
Example C3
   1-(azetidin-3-ylmethyl)-N-[3-[[4-[[5-[4-(difluoromethoxy)-2,3-difluoro-phenyl]-1-methylimidazole-2-carbonyl]amino]-2-ethyl-benzoyl]amino]propyl]piperidine-4-carboxamide; formic acid
Example C4
   N-[4-[[1-(azetidin-3-ylmethyl)-4-piperidyl]methylcarbamoyl]-3-chloro-phenyl]-5-(2,3-difluoro-4-methoxy-phenyl)-1-methyl-imidazole-2-carboxamide;2,2,2-trifluoroacetic acid; and
Example D1
   5-(4-Methoxyphenyl)-1-methyl-N-[3-methyl-4-(methylcarbamoyl)phenyl]imidazole-2-carboxamide.

In a preferred embodiment, the present invention provides a compound of formula (I) as described herein, or a pharmaceutically acceptable salt thereof, wherein said compound of formula (I) is selected from:
Example A25
   N-[3-chloro-4-[[1-[2-(dimethylamino)acetyl]-4-piperidyl]carbamoyl]phenyl]-5-(2,3-difluoro-4-methoxy-phenyl)-1-methyl-imidazole-2-carboxamide
Example B2
   N-[3-chloro-4-[[1-[(3R)-pyrrolidine-3-carbonyl]pyrrolidin-3-yl]carbamoyl]phenyl]-5-(2,3-difluoro-4-methoxy-phenyl)-1-methyl-imidazole-2-carboxamide;formic acid
Example B4
   N-[4-[[1-[3-(2-aminoethoxy)propanoyl]pyrrolidin-3-yl]carbamoyl]-3-chloro-phenyl]-5-(2,3-difluoro-4-methoxy-phenyl)-1-methyl-imidazole-2-carboxamide;formic acid
Example B5
   N-[3-chloro-4-[[1-(piperidine-4-carbonyl)-4-piperidyl]carbamoyl]phenyl]-5-(2,3-difluoro-4-methoxy-phenyl)-1 -methyl-imidazole-2-carboxamide;formic acid
Example B7
   N-[3-chloro-4-[[1-[(2S,4R)-4-hydroxypyrrolidine-2-carbonyl]-4-piperidyl]carbamoyl]phenyl]-5-(2,3-difluoro-4-methoxy-phenyl)-1-methyl-imidazole-2-carboxamide
Example B10
   N-[4-[2-[1-(3-aminopropanoyl)azetidin-3-yl]ethylcarbamoyl]-3-chloro-phenyl]-5-[4-(difluoromethoxy)-2,3-difluoro-phenyl]-1-methyl-imidazole-2-carboxamide;2,2,2-trifluoroacetic acid
Example B17
   N-[4-[[1-(2-aminoacetyl)azetidin-3-yl]carbamoyl]-3-chloro-phenyl]-5-(2,3-difluoro-4-methoxyphenyl)-1-methyl-imidazole-2-carboxamide;formic acid
Example B21
   N-[4-[[3-[(3-aminocyclobutyl)carbamoyl]cyclobutyl]carbamoyl]-3-chloro-phenyl]-5-(2,3-difluoro-4-methoxy-phenyl)-1-methyl-imidazole-2-carboxamide;2,2,2-trifluoroacetic acid
Example B38
   N-[3-chloro-4-[[3-[[(4R)-4-hydroxypyrrolidine-2-carbonyl]amino]cyclobutyl]carbamoyl]phenyl]-5-(2,3-difluoro-4-methoxy-phenyl)-1-methylimidazole-2-carboxamide;formic acid
Example B39
   N-[3-[[2-chloro-4-[[5-(2,3-difluoro-4-methoxy-phenyl)-1-methyl-imidazole-2-carbonyl]amino]benzoyl]amino]cyclobutyl]piperidine-4-carboxamide;formic acid; and
Example B42
   (3S,4S)-N-[3-[[2-chloro-4-[[5-(2,3-difluoro-4-methoxy-phenyl)-1-methyl-imidazole-2-carbonyl]amino]benzoyl]amino]cyclobutyl]-3-hydroxy-piperidine-4-carboxamide;formic acid.

In one embodiment, the present invention provides pharmaceutically acceptable salts of the compounds of formula (I) as described herein, especially pharmaceutically acceptable salts selected from hydrochlorides, fumarates, lactates (in particular derived from L-(+)-lactic acid), tartrates (in particular derived from L-(+)-tartaric acid) and trifluoroacetates. In yet a further particular embodiment, the present invention provides compounds according to formula (I) as described herein (i.e., as "free bases" or "free acids", respectively).

In some embodiments, the compounds of formula (I) are isotopically-labeled by having one or more atoms therein replaced by an atom having a different atomic mass or mass number. Such isotopically-labeled (i.e., radiolabeled) compounds of formula (I) are considered to be within the scope of this disclosure. Examples of isotopes that can be incorporated into the compounds of formula (I) include isotopes of hydrogen, carbon, nitrogen, oxygen, phosphorous, sulfur, fluorine, chlorine, and iodine, such as, but not limited to, ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. Certain isotopically-labeled compounds of formula (I), for example, those incorporating a radioactive isotope, are useful in drug and/or substrate tissue distribution studies. The radioactive isotopes tritium, i.e. ³H, and carbon-14, i.e., ¹⁴C, are particularly useful for this purpose in view of their ease of incorporation and ready means of detection. For example, a compound of formula (I) can be enriched with 1, 2, 5, 10, 25, 50, 75, 90, 95, or 99 percent of a given isotope.

Substitution with heavier isotopes such as deuterium, i.e. ²H, may afford certain therapeutic advantages resulting from greater metabolic stability, for example, increased *in vivo* half-life or reduced dosage requirements.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of formula (I) can generally be prepared by conventional techniques known to those skilled in the art or by processes analogous to those described in the Examples as set out below using an appropriate isotopically-labeled reagent in place of the non-labeled reagent previously employed.

### Processes of Manufacturing

The preparation of compounds of formula (I) of the present invention may be carried out in sequential or convergent synthetic routes. Syntheses of the compounds of the invention are shown in the following schemes. The skills required for carrying out the reactions and purifications of the resulting products are known to those skilled in the art. The substituents and indices used in the following description of the processes have the significance given herein before unless indicated to the contrary. In more detail, the compounds of formula (I) can be manufactured by the methods given below, by the methods given in the examples or by analogous methods. Appropriate reaction conditions for the individual reaction steps are known to a person skilled in the art. Also, for reaction conditions described in literature affecting the described reactions see for example: Comprehensive Organic Transformations: A Guide to Functional Group Preparations, 3rd Edition, Richard C. Larock. John Wiley & Sons, New York, NY. 2018). We find it convenient to carry out the reactions in the presence or absence of a solvent. There is no particular restriction on the nature of the solvent to be employed, provided that it has no adverse effect on the reaction or the reagents involved and that it can dissolve the reagents, at least to some extent. The described reactions can take place over a wide range of temperatures, and the precise reaction temperature is not critical to the invention. It is convenient to carry out the described reactions in a temperature range between -78 °C to reflux temperature. The time required for the reaction may also vary widely, depending on many factors, notably the reaction temperature and the nature of the reagents. However, a period of from 0.5 h to several days will usually suffice to yield the described intermediates and compounds. The reaction sequence is not limited to the one displayed in the schemes, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

All substituents, in particular, R¹ to R⁹ are as defined above and in the claims, unless otherwise indicated. Furthermore, and unless explicitly otherwise stated, all reactions, reaction conditions, abbreviations and symbols have the meanings well known to a person of ordinary skill in organic chemistry.

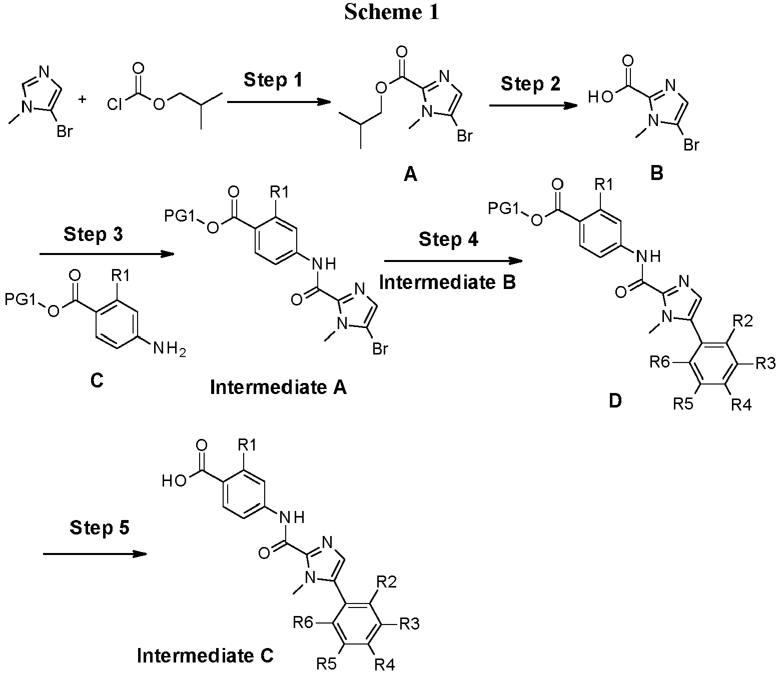

Wherein PG1 denotes a suitable protective group, such as an alkyl group, e.g. Me, Et or iso-butyl.

Compound of formula **Intermediate C** can be prepared according to Scheme 1. 5-bromo-1-methyl-imidazole can react with isobutyl carbonochloridate under basic condition to afford compound **A.** Acid **B** can be obtained by hydrolysis of **A.** Coupling of **B** and amine **C** with a condensing agent, such as HATU/DIPEA in DMSO, affords **Intermediate A.** Suzuki coupling of **Intermediate A** with bronic acids or esters (**Intermediate B**) can be achieved using palladium catalysts and phosphine ligands to give compounds **D.** Hydrolysis compounds **D** yields **Intermediate C.** 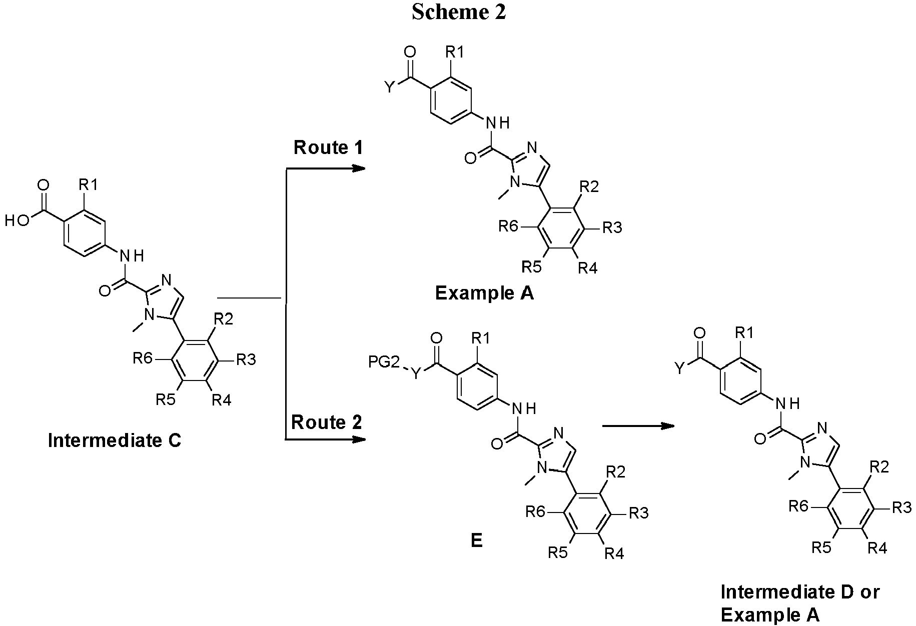 wherein PG2 is a suitable amino protective group, such as Boc.

Wherein Y is a primary amine (Intermediate D) or a secondary amine (Example A, compound E).

**Intermediate D** (Y=NH₂)/**Example A** (Y=NHR⁸) can be prepared according to **route 1 and 2** in Scheme 2. Coupling of acid **Intermediate C** with a free amine **Y** or with a protected amine **PG2-Y** using a condensing agent, such as HATU/DIPEA in DMSO, affords the compound of formula **Example A** (**Route 1**) or **compound E.** Deprotection of the **Compound E** yields **Intermediate D** or **Example A** (**Route 2**)**.** 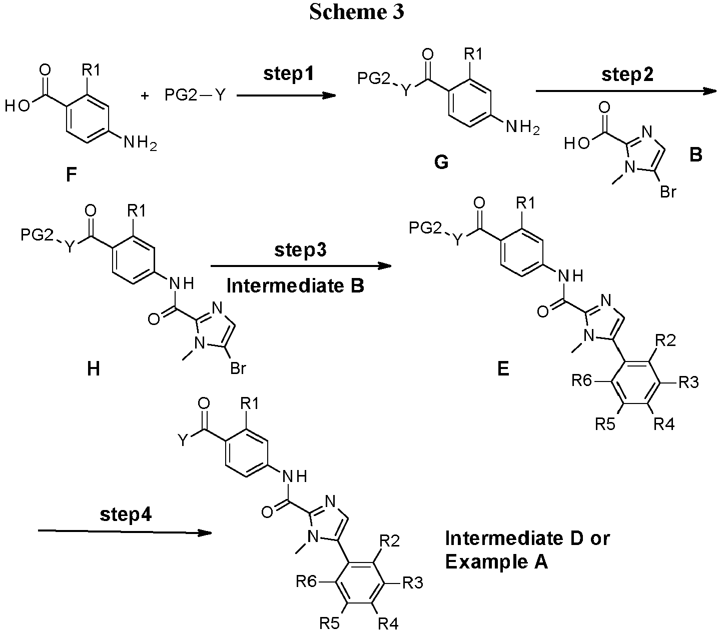
wherein PG2 is a suitable amino protective group, such as Boc; and
Y is a primary amine (Intermediate D) or a secondary amine (Example A, compound E).

**Intermediate D** (Y=NH₂)/**Example A** (Y=NHR⁸) can also be prepared according to the **route** outlined in Scheme 3. Thus, coupling of acid **Intermediate F** with protected amine **PG2-Y** using a condensing agent, such as HATU/DIPEA in DMSO, affords **compound G**. Further coupling of **B** and amine **G** using a condensing agent, such as HATU/DIPEA in DMSO, then yields **compound H.** Suzuki coupling of **Compound H** with bronic acids or esters (**Intermediate B**) can be achieved using palladium catalysts and phosphine ligands to give compounds **E**. Deprotection of Compound **E** affords Intermediate **D** or Example **A**, respectively. 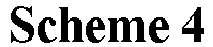 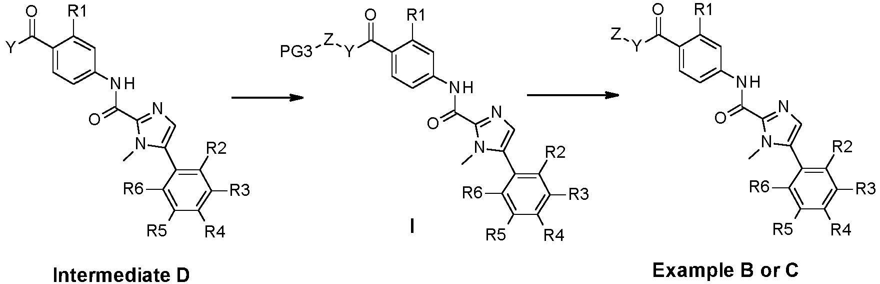
Wherein PG3 is a suitable amino protective group, such as Boc;
Y is a primary amine (Intermediate D) or a secondary amine (compound I, Examples B and C); and
Z is an amine or a group R-C(O)-NH-, such that Y and Z taken together form a group R⁸ as defined herein.

Compounds **B** and **C** can be prepared according to the route in Scheme 4. Coupling of acid **D** with protected amine **PG3-Z** using a condensing agent, such as HATU/DIPEA in DMSO, affords **compound I,** followed by deprotection of the **PG3** of **Z** to give **B.** In some case, the **compound I** was prepared through reductive amination reaction between Intermediate **D** and aldehyde **PG3-Z** using e.g. NaBH₃CN in MeOH solution, followed by deprotection of the **PG3** of **Z** to give **B.**

In one aspect, the present invention provides a process of manufacturing the compounds of formula (I) described herein, wherein said process is as described in any one of Schemes 1 to 4 above.

In a further aspect, the present invention provides a compound of formula (I) as described herein, or a pharmaceutically acceptable salt thereof, when manufactured according to the processes disclosed herein.

### Using the Compounds of the Invention

As illustrated in the experimental section, the compounds of formula (I) and their pharmaceutically acceptable salts possess valuable pharmacological properties for the treatment or prevention of infections and resulting diseases, particularly bacteremia, pneumonia, meningitis, urinary tract infection, and wound infection, caused by pathogens, particularly by bacteria, more particularly by *Acinetobacter* species, most particularly by *Acinetobacter baumannii.*

The compounds of formula (I) and their pharmaceutically acceptable salts exhibit activity as antibiotics, particularly as antibiotics against *Acinetobacter* species, more particularly as antibiotics against *Acinetobacter baumannii*, most particularly as pathogen-specific antibiotics *against Acinetobacter baumannii.*

The compounds of formula (I) and their pharmaceutically acceptable salts can be used as antibiotics, i.e. as antibacterial pharmaceutical ingredients suitable in the treatment and prevention of bacterial infections, particularly in the treatment and prevention of bacterial infections caused by *Acinetobacter* species, more particularly in the treatment and prevention of bacterial infections caused by *Acinetobacter baumannii.*

The compounds of the present invention can be used, either alone or in combination with other drugs, for the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.*

In one aspect, the present invention provides compounds of formula (I) or their pharmaceutically acceptable salts as described herein for use as therapeutically active substances.

In a further aspect, the present invention provides a compound of formula (I) as described herein, or a pharmaceutically acceptable salt thereof, for use as antibiotic.

In a further aspect, the present invention provides a compound of formula (I) as described herein, or a pharmaceutically acceptable salt thereof, for use in the treatment or prevention of nosocomial infections and resulting diseases.

In a particular embodiment, said nosocomial infections and resulting diseases are selected from bacteremia, pneumonia, meningitis, urinary tract infection and wound infection, or a combination thereof.

In a further aspect, the present invention provides a compound of formula (I) as described herein, or a pharmaceutically acceptable salt thereof, for use in the treatment or prevention of infections and resulting diseases caused by Gram-negative bacteria.

In a particular embodiment, said infections and resulting diseases caused by Gram-negative bacteria are selected from bacteremia, pneumonia, meningitis, urinary tract infection and wound infection, or a combination thereof.

In a further aspect, the present invention provides a compound of formula (I) as described herein, or a pharmaceutically acceptable salt thereof, for use in the treatment or prevention of infections and resulting diseases caused by ***E**nterococcus faecium, **S**taphylococcus aureus, **K**lebsiella pneumoniae, **A**cinetobacter baumannii, **P**seudomonas aeruginosa, **E**nterobacter species* or *E*. *coli*, or a combination therof.

In a further aspect, the present invention provides the use of a compound of formula (I) as described herein, or a pharmaceutically acceptable salt thereof, for the preparation of medicaments useful for the treatment or prevention of infections and resulting diseases caused by ***E**nterococcus faecium, **S**taphylococcus aureus, **K**lebsiella pneumoniae, **A**cinetobacter baumannii, **P**seudomonas aeruginosa, **E**nterobacter species* or *E. coli*, or a combination therof. In a particular embodiment, said infections and resulting diseases caused by ***E**nterococcus faecium, **S**taphylococcus aureus, **K**lebsiella pneumoniae, **A**cinetobacter baumannii, **P**seudomonas aeruginosa, **E**nterobacter species* or *E. coli,* or a combination therof, are selected from bacteremia, pneumonia, meningitis, urinary tract infection and wound infection, or a combination thereof.

In a further aspect, the present invention provides compounds of formula (I) or their pharmaceutically acceptable salts as defined above for use in the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.*

In a further aspect, the present invention provides the use of compounds of formula (I) or their pharmaceutically acceptable salts as defined above for the preparation of medicaments for the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.* Such medicaments comprise compounds of formula (I) or their pharmaceutically acceptable salts as defined above.

### Pharmaceutical Compositions and Administration

In one aspect, the present invention provides pharmaceutical compositions comprising compounds of formula (I) or their pharmaceutically acceptable salts as defined above and one or more pharmaceutically acceptable excipients. Exemplary pharmaceutical compositions are described in Examples 1-4.

In a further aspect, the present invention relates to pharmaceutical compositions comprising compounds of formula (I) or their pharmaceutically acceptable salts as defined above and one or more pharmaceutically acceptable excipients for the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.*

The compounds of formula (I) and their pharmaceutically acceptable salts can be used as medicaments (e.g. in the form of pharmaceutical preparations). The pharmaceutical preparations can be administered internally, such as orally (e.g. in the form of tablets, coated tablets, dragées, hard and soft gelatin capsules, solutions, emulsions or suspensions), nasally (e.g. in the form of nasal sprays) or rectally (e.g. in the form of suppositories). However, the administration can also be effected parentally, such as intramuscularly or intravenously (e.g. in the form of injection solutions or infusion solutions).

The compounds of formula (I) and their pharmaceutically acceptable salts can be processed with pharmaceutically inert, inorganic or organic excipients for the production of tablets, coated tablets, dragées and hard gelatin capsules. Lactose, corn starch or derivatives thereof, talc, stearic acid or its salts etc. can be used, for example, as such excipients for tablets, dragées and hard gelatin capsules.

Suitable excipients for soft gelatin capsules are, for example, vegetable oils, waxes, fats, semisolid substances and liquid polyols, etc.

Suitable excipients for the production of solutions and syrups are, for example, water, polyols, saccharose, invert sugar, glucose, etc.

Suitable excipients for injection solutions are, for example, water, alcohols, polyols, glycerol, vegetable oils, etc.

Suitable excipients for suppositories are, for example, natural or hardened oils, waxes, fats, semisolid or liquid polyols, etc.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should be appropriate. It will, however, be clear that the upper limit given herein can be exceeded when this is shown to be indicated.

### Co-Administration of Compounds of Formula (I) and Other Agents

The compounds of formula (I) or salts thereof or a compound disclosed herein or a pharmaceutically acceptable salt thereof may be employed alone or in combination with other agents for treatment. For example, the second agent of the pharmaceutical combination formulation or dosing regimen may have complementary activities to the compound of formula (I) such that they do not adversely affect each other. The compounds may be administered together in a unitary pharmaceutical composition or separately. In one embodiment a compound or a pharmaceutically acceptable salt can be co-administered with an antibiotic, in particular with an antibiotic for the treatment or prevention of infections and resulting diseases caused by ***E**nterococcus faecium, **S**taphylococcus aureus, K**l**ebsiella pneumoniae, **A**cinetobacter baumannii, **P**seudomonas aeruginosa, **E**nterobacter species* or *E. coli*, or a combination therof.

The term "co-administering" refers to either simultaneous administration, or any manner of separate sequential administration, of a compound of formula (I) or a salt thereof or a compound disclosed herein or a pharmaceutically acceptable salt thereof and a further active pharmaceutical ingredient or ingredients, including antibiotic agents. If the administration is not simultaneous, the compounds are administered in a close time proximity to each other. Furthermore, it does not matter if the compounds are administered in the same dosage form, e.g. one compound may be administered intravenously and another compound may be administered orally.

Typically, any agent that has antimicrobial activity may be co-administered. Particular examples of such agents are Carbapenems (meropenem), Fluoroquinolone (Ciprofloxacin), Aminoglycoside (amikacin), Tetracyclines (tigecycline), Colistin, Sulbactam, Sulbactam+Durlobactam, Cefiderocol (Fetroja), macrocyclic peptides as exemplified e.g. in WO 2017072062 A1, WO 2019185572 A1 and WO 2019206853 A1, and Macrolides (erythromycin).

In one aspect, the present invention provides a pharmaceutical composition described herein, further comprising an additional therapeutic agent.

In one embodiment, said additional therapeutic agent is an antibiotic agent.

In one embodiment, said additional therapeutic agent is an antibiotic agent that is useful for the treatment or prevention of infections and resulting diseases caused by ***E**nterococcus faecium, **S**taphylococcus aureus, **K**lebsiella pneumoniae, **A**cinetobacter baumannii, **P**seudomonas aeruginosa, **E**nterobacter species* or *E. coli*, or a combination therof.

In one embodiment, said additional therapeutic agent is an antibiotic agent selected from Carbapenems (meropenem), Fluoroquinolone (Ciprofloxacin), Aminoglycoside (amikacin), Tetracyclines (tigecycline), Colistin, Sulbactam, Sulbactam+Durlobactam, Cefiderocol (Fetroja), macrocyclic peptides as exemplified in WO 2017072062 A1, WO 2019185572 A1 and WO 2019206853 A1, and Macrolides (erythromycin).

### Examples

The invention will be more fully understood by reference to the following examples. The claims should not, however, be construed as limited to the scope of the examples.

In case the preparative examples are obtained as a mixture of enantiomers, the pure enantiomers can be separated by methods described herein or by methods known to the man skilled in the art, such as e.g., chiral chromatography (e.g., chiral SFC) or crystallization.

All reaction examples and intermediates were prepared under an argon atmosphere if not specified otherwise.

Abbreviations used herein are as follows:
- ACN or MeCN: acetonitrile
- BINAP: 2,2'-Bis(diphenylphosphino)-1,1'-binaphthalene
- CFU: colony-forming unit
- d: day
- DCM: dichloromethane
- DIPEA: N,N-diisopropylethylamine
- EtOAc or EA: ethyl acetate
- FA: formic acid
- h(s) or hr(s): hour(s)
- HATU:: 1-[Bis(dimethylamino)methylene]-1H-1,2,3-triazolo[4,5-b]pyridinium 3-oxid hexafluorophosphate
- HPLC:: high performance liquid chromatography
- HPLC-UV:: high performance liquid chromatography with ultraviolet detector
- IC50: half maximal inhibitory concentration
- IC90: 90% inhibitory concentration
- PE: petroleum ether
- PdCl₂(DPPF): [1,1'-Bis(diphenylphosphino)ferrocene]dichloropalladium(II)
- Pd₂(dba)₃: Tris(dibenzylideneacetone)dipalladium(0)
- PG: Protecting group
- Precat: precatalyst
- prep-HPLC: preparative high performance liquid chromatography
- RBF: Round bottom flask
- rt: room temperature
- sat: saturated
- SEM: 2-methoxyethyl(trimethyl)silane
- FA: Formic acid
- TFA: Trifluoroacetic Acid
- wt: weight
- X-PHOS: 2-Dicyclohexylphosphino-2' ,4' ,6' -triisopropylbiphenyl

### Intermediate A1

### Methyl 4-[(5-bromo-1-methyl-imidazole-2-carbonyl)amino]-2-chloro-benzoate

### Step1: isobutyl 5-bromo-1-methyl-imidazole-2-carboxylate

To a solution of 5-bromo-1-methyl-imidazole (20 g, 124 mmol) and DIPEA (32.1 g, 43.4 mL, 248 mmol) in DCM (140 mL) at -70 °C was added slowly a solution of isobutyl carbonochloridate (22.1 g, 161 mmol) in DCM (60 mL). The dropwise time lasted about 30 min. The mixture was stirred at -70 °C for 2 h. Then the mixture was slowly warmed to room temperature and stirred overnight. Then the solution was washed with water and concentrated in vacuum. The crude product was then purified by flash column chromatography to afford isobutyl 5-bromo-1-methyl-1H-imidazole-2-carboxylate (29 g) as a yellow oil. MS [M+H]⁺ : 261.2.

### Step2: 5-bromo-1-methyl-imidazole-2-carboxylic acid

To a solution of isobutyl 5-bromo-1-methyl-1H-imidazole-2-carboxylate (29 g, 111 mmol) in MeOH (5 mL) and THF (120 mL) was added a solution of lithium hydroxide monohydrate (9.32 g, 222 mmol) in water (60 mL). The mixture was stirred at room temperature for 3 h. The organic solvent was removed under reduced pressure. 12 N HCl aqueous solution was added under stirring until pH 4-5. The white solid was filtered, washed with MeOH and dried over anhydrous Na₂SO₄ to afford 5-bromo-1-methyl-imidazole-2-carboxylic acid (20.5 g) as a white solid. MS [M+H]⁺ : 204.8.

### Step3: methyl 4-[(5-bromo-1-methyl-imidazole-2-carbonyl)amino]-2-chloro-benzoate

A mixture of 5-bromo-1-methyl-1H-imidazole-2-carboxylic acid (13 g, 63.4 mmol), methyl 4-amino-2-chloro-benzoate (11.8 g, 63.4 mmol), HATU (24.1 g, 63.4 mmol) and DIPEA (24.6 g, 190 mmol) in DMF (50 mL) was stirred at room temperature overnight. Then the mixture was poured into water. The water phase was extracted with DCM. The combined organic phases were washed with water, dried over anhydrous Na₂SO₄ and concentrated in vacuum. The solids precipitated from the concentrated solution. The solids were collected, washed with MeOH and dried to afford methyl 4-[(5-bromo-1-methyl-imidazole-2-carbonyl)amino]-2-chloro-benzoate (18 g) as a light yellow solid.

MS [M+H]⁺ : 371.8.

### Intermediate A2

### tert-Butyl 4-[(5-bromo-1-methyl-imidazole-2-carbonyl)amino]-2-chloro-benzoate

### Step1: tert-butyl 2-chloro-4-nitro-benzoate

To a mixture of 2-chloro-4-nitro-benzoic acid (15.0 g, 74.42 mmol), N,N-dimethylpyridin-4-amine (2.73 g, 22.33 mmol) and N,N-diethylethanamine (31.12 mL, 223.26 mmol) in THF (80 mL) was added a solution of *tert*-butoxycarbonyl *tert*-butyl carbonate (24.36 g, 111.63 mmol) in THF (20 mL) at -10 °C. The resulting mixture was warmed to 25 °C and stirred for another 14 h. The mixture was concentrated. The residue was treated with EA (50 mL) and H₂O (50 mL). The mixture was extracted with EA. The combined organic layers were concentrated. The crude was then purified by flash column chromatography to afford *tert*-butyl 2-chloro-4-nitro-benzoate (18.8 g) as a colorless solid.

### Step2: tert-butyl 4-amino-2-chloro-benzoate

To a mixture of *tert*-butyl 2-chloro-4-nitro-benzoate (18.8 g, 72.96 mmol) and Ammonium chloride (19.51 g, 364.81 mmol) in ethanol (200 mL) and water (200 mL) was added Iron (20.37 g, 364.81 mmol). The mixture was stirred at 25 °C for 14 h. The mixture was filtered by Celite. The filtrate was concentrated to remove ethanol. The mixture was extracted with EA. The combined organic layers were dried over anhydrous Na₂SO₄ and concentrated to afford *tert*-butyl 4-amino-2-chloro-benzoate (16.31 g) as a light yellow solid. MS [M+H]⁺ : 228.1.

### Step3: tert-butyl 4-[(5-bromo-1-methyl-imidazole-2-carbonyl)amino]-2-chloro-benzoate

A mixture of 5-bromo-1-methyl-imidazole-2-carboxylic acid hydrochloride (7.0 g, 28.99 mmol), *tert*-butyl 4-amino-2-chloro-benzoate (6.0 g, 26.35 mmol), HATU (13.23 g, 34.79 mmol) and DIPEA (16.16 mL, 92.77 mmol) in DMF (15 mL) was stirred at 25 °C for 3 h. The mixture was added water (10 mL) and extracted with EA. The combined organic layers were concentrated. The crude was purified by FCC to afford *tert*-butyl 4-[(5-bromo-1-methyl-imidazole-2-carbonyl)amino]-2-chloro-benzoate (8 g, 19.29 mmol) as a white solid. MS [M+H]⁺ : 414.0.

The following intermediates were prepared in analogy:

| **Int.** | **Name** | **MS ESI [M+H]⁺** | **Starting Material** |
|---|---|---|---|
| **Interme diate A3** | *tert*-butyl 4-[(5-bromo-1-methyl-imidazole-2-carbonyl)amino]-2-methyl-benzoate | 395.1 | 2-Methyl-4-nitrobenzoic acid |
| **Interme diate A4** | *tert*-butyl 4-[(5-bromo-1-methyl-imidazole-2-carbonyl)amino]-2-ethyl-benzoate | 409.1 | 2-Ethyl-4-nitrobenzoic acid |

### Intermediate B1

### 2-(4-(Difluoromethoxy)-2,3-difluorophenyl)-4,4,5,5-tetramethyl-1,3,2-dioxaborolane

### Step 1: 1-bromo-4-(difluoromethoxy)-2,3-difluorobenzene

To a solution of 4-bromo-2,3-difluorophenol (25 g, 120 mmol), sodium 2-chloro-2,2-difluoroacetate (36.5 g, 239 mmol) and K₂CO₃ (19.8 g, 144 mmol) in DMF (250 mL) and Water (57 mL).The reaction mixture was heated to 100°C and stirred for 3.0 h at 100°C under N₂, The reaction mixture was poured into 1.5L l H2O and extracted with EtOAc (3 x 250 mL).The organic layers were combined, washed with sat NaCl (200 mL), The organic layers were dried over Na₂SO₄ and concentrated in vacuum. The crude material was purified by flash chromatography to afford 1-bromo-4-(difluoromethoxy)-2,3-difluorobenzene(25.2 g)

### Step 2: 2-(4-(difluoromethoxy)-2,3-difluorophenyl)-4,4,5,5-tetramethyl-1,3,2-dioxaborolane

To a 250 mL round flask was added 4,4,4',4',5,5,5',5'-octamethyl-2,2'-bi(1,3,2-dioxaborolane) (24.5 g, 96.5 mmol), 1-bromo-4-(difluoromethoxy)-2,3-difluorobenzene (25 g, 96.5 mmol), PdCl₂(DPPF)-CH₂Cl₂ adduct (3.53 g, 4.83 mmol) and potassium acetate (18.9 g, 193 mmol) in Dioxane (150 mL). The vial was capped and heated at 80 °C for 15 h under N₂.The crude reaction mixture was concentrated in vacuum. The reaction mixture was poured into 50 mL H2O and extracted with EtOAc (50 mL x 3).The organic layers were combined, washed with sat NaCl (50 mL), The organic layers were dried over Na₂SO₄ and concentrated in vacuum. The crude material was purified by flash chromatography. to afford 2-(4-(difluoromethoxy)-2,3-difluorophenyl)-4,4,5,5-tetramethyl-1,3,2-dioxaborolane (25 g, 81.7 mmol, 84.6 % yield).

The following intermediates were prepared in analogy:

| **Int.** | **Name** | **MS ESI [M+H]⁺** | **Starting Material** |
|---|---|---|---|
| **Interme diate B2** | 2-[2,3-difluoro-4-(4,4,5,5-tetramethyl-1,3,2-dioxaborolan-2-yl)phenoxy] acetonitrile | 296.2 | 4-bromo-2,3-difluorophenol and bromoacetonitrile |
| **Interme diate B3** | 2-[3-chloro-2-fluoro-4-(4,4,5,5-tetramethyl-1,3,2-dioxaborolan-2-yl)phenoxy] acetonitrile | 312.5 | 4-bromo-3-chloro-2-fluorophenol and bromoacetonitrile |

### Intermediate C1

### 2-Chloro-4-[[5-(2,3-difluoro-4-methoxy-phenyl)-1-methyl-imidazole-2-carbonyl]amino]benzoic acid

### Step 1: methyl 2-chloro-4-[[5-(2,3-difluoro-4-methoxy-phenyl)-1-methyl-imidazole-2-carbonyl]amino]benzoate

Under N₂ protection, a mixture of methyl 4-[(5-bromo-1-methyl-imidazole-2-carbonyl)amino]-2-chloro-benzoate (1 g, 2.68 mmol), (2,3-difluoro-4-methoxyphenyl)boronic acid (504 mg, 2.68 mmol), Na₂CO₃ (853 mg, 8.05 mmol) and 1,1'-bis(di-*tert*-butylphosphino)ferrocene palladium dichloride (350 mg, 537 µmol) in 1,4-Dioxane (15 mL) and water (1.5 mL) was irritated under microwave at 100 °C for 60 min. Repeat this reaction for eight times. The combined reaction solutions were concentrated. Water (40 mL) was added and the mixture was filtered. The water phase was extracted with DCM. The combined organic phases were washed with water, dried over anhydrous Na₂SO₄ and concentrated. The concentrated organic phase was filtrated and the solid was dried to afford the crude methyl 2-chloro-4-[[5-(2,3-difluoro-4-methoxy-phenyl)-1-methyl-imidazole-2-carbonyl]amino]benzoate (8.3 g) as a brown solid. MS [M+H]⁺ : 435.9.

### Step 2: 2-chloro-4-[[5-(2,3-difluoro-4-methoxy-phenyl)-1-methyl-imidazole-2-carbonyl]amino]benzoic acid

To a solution of methyl 2-chloro-4-[[5-(2,3-difluoro-4-methoxy-phenyl)-1-methyl-imidazole-2-carbonyl]amino]benzoate (8.3 g, 19 mmol) in MeOH (2 mL), THF (48 mL) and water (24 mL) was added a solution of lithium hydroxide monohydrate (3.2 g, 76.2 mmol) in water (24 mL). The mixture was stirred at room temperature overnight. Then the mixture was concentrated and acidified by 6 N HCl under stirring until pH 3-4. Some solids precipitated from the concentrated solution. The solids were filtered and dried to afford 2-chloro-4-[[5-(2,3-difluoro-4-methoxyphenyl)-1-methyl-imidazole-2-carbonyl]amino]benzoic acid (7 g) as a brown solid. MS [M+H]⁺ : 422.3.

### Intermediate C2

### 2-Chloro-4-[[5-[4-(difluoromethoxy)-2,3-difluoro-phenyl]-1-methyl-imidazole-2-carbonyl]amino]benzoic acid

### Step 1: tert-butyl 2-chloro-4-[[5-[4-(difluoromethoxy)-2,3-difluoro-phenyl]-1-methyl-imidazole-2-carbonyl]amino]benzoate

To a 25 mL microwave vial was added *tert*-butyl 4-(5-bromo-1-methyl-1H-imidazole-2-carboxamido)-2-chlorobenzoate (1 g, 2.41 mmol), 2-(4-(difluoromethoxy)-2,3-difluorophenyl)-4,4,5,5-tetramethyl-1,3,2-dioxaborolane (775 mg, 2.53 mmol), 1,1'-bis(di-*tert-*butylphosphino)ferrocene palladium dichloride (157 mg, 241 µmol) and Na₂CO₃ (767 mg, 7.23 mmol) in Dioxane (18 mL)Water (1.8 mL) under N₂.the reaction was heated at 100 °C for 15 h under N₂ .The crude reaction mixture was concentrated in vacuum. The crude material was purified by flash chromatography to afford tert-butyl 2-chloro-4-(5-(4-(difluoromethoxy)-2,3-difluorophenyl)-1-methyl-1H-imidazole-2-carboxamido)benzoate (1.2 g, 2.34 mmol, 96.8 % yield). MS [M+H]⁺ : 514.0 .

### Step 2: 2-chloro-4-[[5-[4-(difluoromethoxy)-2,3-difluoro-phenyl]-1-methyl-imidazole-2-carbonyl]amino]benzoic acid

In a 100 mL round-bottomed flask, *tert*-butyl 2-chloro-4-(5-(4-(difluoromethoxy)-2,3-difluorophenyl)-1-methyl-1H-imidazole-2-carboxamido)benzoate (1.2 g, 2.34 mmol) was combined with DCM (4 mL) to give a light yellow solution. TFA (5.4 mL, 70.1 mmol) was added. The reaction was stirred at room temperature for 30 min. The crude reaction mixture was concentrated in vacuum. The crude product was directly used to the next step, to afford 2-chloro-4-(5-(4-(difluoromethoxy)-2,3-difluorophenyl)-1-methyl-1H-imidazole-2-carboxamido)benzoic acid (1.07 g ). MS [M+H]⁺ : 458.0.

The following intermediates were prepared in analogy:

| **Int.** | **Name** | **MS ESI [M+H]⁺** | **Starting Material** |
|---|---|---|---|
| **C3** | 2-chloro-4-[[5-[4-(cyanomethoxy)-2,3-difluorophenyl]-1-methyl-imidazole-2-carbonyl]amino]benzoic acid | 447.1 | IntermediateA2 and B2 |
| **C4** | 4-[[5-[4-(cyanomethoxy)-2,3-difluoro-phenyl]-1-methylimidazole-2-carbonyl]amino]-2-methyl-benzoicacid | 427.1 | IntermediateA3 and B2 |
| **C5** | 4-[[5-(2,3-difluoro-4-methoxyphenyl)-1-methyl-imidazole-2-carbonyl]amino]-2-ethyl-benzoic acid | 436.1 | IntermediateA4 and (2,3-difluoro-4-methoxyphenyl)boronic acid |
| **C6** | 4-[[5-[4-(difluoromethoxy)phenyl]-1-methyl-imidazole-2-carbonyl]amino]-2-ethyl-benzoic acid | 416.2 | Intermediate A4 and 2-(4-(difluoromethoxy)phenyl)-4,4,5-trimethyl-1,3,2-dioxaborolane |
| **C7** | 2-ethyl-4-[[5-(3-fluoro-4-isopropoxy-phenyl)-1-methylimidazole-2-carbonyl]amino]benzoic acid | 426.3 | Intermediate A4 and (3-fluoro-4-isopropoxyphenyl)boronic acid |
| **C8** | 4-[[5-(2-chloro-4-methoxyphenyl)-1-methyl-imidazole-2-carbonyl]amino]-2-ethyl-benzoic acid | 414.2 | Intermediate A4 and (2-chloro-4-methoxyphenyl)boronic acid |
| **C9** | 4-[[5-[4-(difluoromethoxy)-2,3-difluoro-phenyl]-1-methylimidazole-2-carbonyl]amino]-2-ethyl-benzoic acid | 452.3 | Intermediate A4 and 2-(4-(difluoromethoxy)-2,3-difluorophenyl)-4,4,5,5-tetramethyl-1,3,2-dioxaborolane |
| **C10** | 4-[[5-(2,6-difluoro-4-methoxyphenyl)-1-methyl-imidazole-2-carbonyl]amino]-2-ethyl-benzoic acid | 414.3 | Intermediate A4 and (2,6-difluoro-4-methoxyphenyl)boronic acid |
| **C11** | 4-[[5-(2,3-difluoro-4-methoxyphenyl)-1-methyl-imidazole-2-carbonyl]amino]-2-methylbenzoic acid | 402.3 | Intermediate A3 and (2,3-difluoro-4-methoxyphenyl)boronic acid |

### Intermediate D1

### N-[3-Chloro-4-(pyrrolidin-3-ylcarbamoyl)phenyl]-5-(2,3-difluoro-4-methoxy-phenyl)-1-methyl-imidazole-2-carboxamide

### Step 1: tert-butyl 3-[[2-chloro-4-[[5-(2,3-difluoro-4-methoxy-phenyl)-1-methyl-imidazole-2-carbonyl]amino]benzoyl]amino]pyrrolidine-1-carboxylate

At room temperature, a mixture of *tert*-butyl 3-aminopyrrolidine-1-carboxylate (1.04 g, 5.6 mmol), 2-chloro-4-[[5-(2,3-difluoro-4-methoxy-phenyl)-1-methyl-imidazole-2-carbonyl]amino]benzoic acid (1.97 g, 4.67 mmol), DIPEA (1.81 g, 14 mmol) and HATU (1.77 g, 4.67 mmol) in DMF (10 mL) was stirred for 35 min. Then the solution was poured into water. The water layer was extracted with DCM. The combined organic layers were washed with water, dried over anhydrous Na₂SO₄ and concentrated. The residue was purired by flash column to afford *tert*-butyl 3-[[2-chloro-4-[[5-(2,3-difluoro-4-methoxy-phenyl)-1-methyl-imidazole-2-carbonyl]amino]benzoyl]amino]pyrrolidine-1-carboxylate (2.3 g) as a yellow oil. MS [M+H]⁺ : 590.0.

### Step 2: N-[3-chloro-4-(pyrrolidin-3-ylcarbamoyl)phenyl]-5-(2,3-difluoro-4-methoxyphenyl)-1-methyl-imidazole-2-carboxamide

At room temperature, a solution of *tert*-butyl 3-[[2-chloro-4-[[5-(2,3-difluoro-4-methoxyphenyl)-1-methyl-imidazole-2-carbonyl] amino]benzoyl]amino]pyrrolidine-1-carboxylate (2.3 g, 3.9 mmol) in TFA (10 mL) and DCM (10 mL) was stirred for 30 min. Then the mixture was concentrated. Water (10 mL) was added. The water layer was basified by NH₃.H₂O and extracted with DCM. The combined organic layers were washed with water, dried over anhydrous Na₂SO₄ and concentrated to afford N-[3-chloro-4-(pyrrolidin-3-ylcarbamoyl)phenyl]-5-(2,3-difluoro-4-methoxy-phenyl)-1-methyl-imidazole-2-carboxamide (1.8 g). MS [M+H]⁺ : 490.2.

The following intermediates were prepared in analogy:

| **Int.** | **Name** | **MS ESI [M+H]⁺** | **Starting Material** |
|---|---|---|---|
| **D2** | N-[3-chloro-4-(4-piperidylcarbamoyl)phenyl]-5-(2,3-difluoro-4-methoxyphenyl)-1-methyl-imidazole-2-carboxamide | 504.2 | Intermediate C1 and *tert*-butyl 4-aminopiperidine-1-carboxylate |
| **D3** | N-[4-(2-aminoethylcarbamoyl)-3- chloro-phenyl]-5-(2,3-difluoro-4-methoxy-phenyl)-1-methylimidazole-2-carboxamide | 464.1 | Intermediate C1 and *tert*-butyl N-(2-aminoethyl)carbamate |
| **D4** | N-(4-(((1s,3s)-3-aminocyclobutyl)carbamoyl)-3-chlorophenyl)-5-(2,3-difluoro-4-methoxyphenyl)-1-methyl-1H-imidazole-2-carboxamide | 490.2 | Intermediate C1 and *tert*-butyl ((1s,3s)-3-aminocyclobutyl)carbamate |
| **D5** | N-(4-((2-(azetidin-3-yl)ethyl)carbamoyl)-3- chlorophenyl)-5-(4-(difluoromethoxy)-2,3-difluorophenyl)-1-methyl-1H-imidazole-2-carboxamide | 540.0 | Intermediate C2 and *tert*-butyl 3-(2-aminoethyl)azetidine-1-carboxylate |
| **D6** | N-(4-((3-aminopropyl)carbamoyl)-3-chlorophenyl)-5-(4-(difluoromethoxy)-2,3-difluorophenyl)-1-methyl-1H-imidazole-2-carboxamide | 514.1 | Intermediate C2 and *tert*-butyl (4-aminobutyl)carbamate |
| **D8** | N-[4-(2-aminoethylcarbamoyl)-3- methyl-phenyl]-5-[4-(cyanomethoxy)-2,3-difluorophenyl]-1-methyl-imidazole-2-carboxamide | 468.9 | Intermediate C4 and *tert*-butyl N-(2-aminoethyl)carbamate |
| **D9** | N-[4-(2-aminoethylcarbamoyl)-3- chloro-phenyl]-5-[4-(cyanomethoxy)-2,3-difluorophenyl]-1-methyl-imidazole-2-carboxamide | 489.1 | Intermediate C3 and *tert*-butyl N-(2-aminoethyl)carbamate |
| **D10** | N-[4-(azetidin-3-ylcarbamoyl)-3-chloro-phenyl]-5-(2,3-difluoro-4-methoxy-phenyl)-1-methyl-imidazole-2-carboxamide;2,2,2-trifluoroacetic acid | 476.1 | Intermediate C1 and *tert*-butyl 3-aminoazetidine-1- carboxylate |
| **D11** | N-[4-(azetidin-3-ylmethylcarbamoyl)-3-chloro-phenyl]-5-(2,3-difluoro-4-methoxy-phenyl)-1-methylimidazole-2-carboxamide;2,2,2-trifluoroacetic acid | 490.1 | Intermediate C1 and *tert*-butyl 3-(aminomethyl)azetidine-1- carboxylate |
| **D12** | 3-[[2-chloro-4-[[5-(2,3-difluoro-4-methoxy-phenyl)-1-methyl-imidazole-2-carbonyl]amino]benzoyl]amino ]cyclobutanecarboxylic acid;2,2,2-trifluoroacetic acid | 519.0 | Intermediate C1 and *tert*-butyl 3-aminocyclobutanecarboxylate |
| **D13** | 3-[[4-[[5-(2,3-difluoro-4-methoxy-phenyl)-1-methylimidazole-2-carbonyl]amino]-2-ethyl-benzoyl]amino] cyclobutanecarboxylic acid;2,2,2-trifluoroacetic acid | 512.0 | Intermediate C5 and *tert*-butyl 3-aminocyclobutanecarboxylate |
| **D14** | N-[4-(2-aminoethylcarbamoyl)-3 - methyl-phenyl]-5-(2,3-difluoro-4-methoxy-phenyl)-1-methyl-imidazole-2-carboxamide | 444.2 | Intermediate C11 and *tert-*butyl N-(2-aminoethyl)carbamate |

### Intermediate D7 (Example A19)

### N-[4-(3-Aminopropylcarbamoyl)-3-chloro-phenyl]-5-[4-(difluoromethoxy)-2,3-difluorophenyl]-1-methyl-imidazole-2-carboxamide

### Step 1: tert-butyl N-[3-[(4-amino-2-ethyl-benzoyl)amino]propyl]carbamate

To a solution of intermediate 4-amino-2-ethylbenzoic acid (825 mg, 5.0 mmol), *tert*-butyl (3-aminopropyl) carbamate (1.30 g, 7.5 mmol) in anhydrous DMF (15 mL) was added DIPEA (1.30 g, 10 mmol) and then the resultant mixture was stirred for 10 min at room temperature, HATU (2.85 g, 7.5 mmol) was added in the mixture and stirred for extra 10 hr.

The mixture was poured into water (50 mL) and the aqueous solution was extracted with DCM (100 mL × 2). The organic layers were combined and washed with water and brine, dried over anhydrous sodium sulfate and concentrated under reduced pressure to give a red oil which was purified by flash column to afford tert-butyl N-[3-[(4-amino-2-ethyl-benzoyl)amino]propyl]carbamate (1.2 g) as a yellow oil. MS [M+H]⁺ : 322.2

### Step 2: tert-butyl N-[3-[[4-[(5-bromo-1-methyl-imidazole-2-carbonyl)amino]-2-ethyl-benzoyl]amino]propyl]carbamate

To a solution of 5-bromo-1-methyl-1H-imidazole-2-carboxylic acid (1.0 g, 5.0 mmol), *tert*-butyl (3-(4-amino-2-ethylbenzamido)propyl)carbamate (1.6 g, 5.0 mmol) in anhydrous DMF (25 mL) was added DIPEA (1.3 g, 10 mmol) and then the resultant mixture was stirred for 10 min at room temperature, HATU (3.8 g, 10 mmol) was added in the mixture and stirred for extra 10 hr.

The mixture was poured into water (50 mL) and the aqueous solution was extracted with DCM (150 mL × 2). The organic layers were combined and washed with water and brine, dried over anhydrous sodium sulfate and concentrated under reduced pressure to give a red oil which was purified by flash column to afford *tert*-butyl N-[3-[[4-[(5-bromo-1-methyl-imidazole-2-carbonyl)amino]-2-ethyl-benzoyl]amino]propyl]carbamate (1.8 g) as a yellow solid. MS [M+H]⁺ : 508.3

### Step 3: tert-butyl N-[3-[[4-[[5-[4-(difluoromethoxy)-2,3-difluoro-phenyl]-1-methylimidazole-2-carbonyl]amino]-2-ethyl-benzoyl]amino]propyl]carbamate

A mixture of *tert*-butyl (3-(4-(5-bromo-1-methyl-1H-imidazole-2-carboxamido)-2-ethylbenzamido)propyl)carbamate (1.73 g, 3.4 mmol), 2-(4-(difluoromethoxy)-2,3-difluorophenyl)-4,4,5,5-tetramethyl-1,3,2-dioxaborolane (1.56 g, 5.1 mmol), sodium carbonate (1.1 g, 10.2 mmol) and 1,1'-Bis (di-t-butylphosphino)ferrocene palladium dichloride (220 mg, 0.34 mmol) in 1,4-dioxane (25 ml) and water (2.5 ml) was stirred at 100 °C for 4.0 h. After cooling to room temperature, the mixture was poured into water (50 mL) and the aqueous solution was extracted with DCM (100 mL × 2). The organic layers were combined and washed with water and brine, dried over anhydrous sodium sulfate and concentrated under reduced pressure to give a red oil which was purified by flash column to provide the desired compound as a yellow oil to afford *tert*-butyl N-[3-[[4-[[5-[4-(difluoromethoxy)-2,3-difluoro-phenyl]-1-methyl-imidazole-2-carbonyl]amino]-2-ethyl-benzoyl]amino]propyl] carbamate (1.2 g). MS [M+H]⁺ : 608.2

### Step 4: N-[4-(3-aminopropylcarbamoyl)-3-ethyl-phenyl]-5-[4-(difluoromethoxy)-2,3-difluoro-phenyl]-1-methyl-imidazole-2-carboxamide

To a solution of *tert*-butyl N-[3-[[4-[[5-[4-(difluoromethoxy)-2,3-difluoro-phenyl]-1-methylimidazole-2-carbonyl]amino]-2-ethyl-benzoyl]amino]propyl]carbamate (1.2 g, 2.0 mmol) in THF (15 ml) was added 3N HCl (5.0 mL) at room temperature. The resultant mixture was stirred for 5.0 h and then adjusted to pH=7-8 with aqueous ammonia. The mixture was poured into water (50 mL) and then extracted with dichloromethane / isopropanol (100 / 10 mL), the organic layer was concentrated to give a red oil, which was purified by flash column to afford N-[4-(3-aminopropylcarbamoyl)-3-ethyl-phenyl]-5-[4-(difluoromethoxy)-2,3-difluoro-phenyl]-1-methylimidazole-2-carboxamide (0.8 g) as a light red oil. MS [M+H]⁺ : 508.2

The following intermediates were prepared in analogy:

| **Ex.** | **Name** | **Structure** | **MS ESI [M+H]⁺** | **Starting Material** |
|---|---|---|---|---|
| **A20** | N-[4-[2-(2-aminoethoxy)ethylcarba moyl]-3-ethyl-phenyl]-5- [4-(cyanomethoxy)-2,3-difluoro-phenyl]-1-methyl-imidazole-2-carboxamide;hydrochlori de | 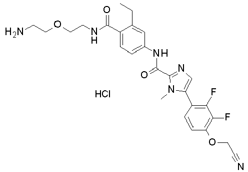 | 527.4 | *tert*-butyl N-[2-(2-aminoethoxy) ethyl] carbama te in step 1 and Intermediate B2 in step 4 |
| **A21** | N-[4-[2-(2-aminoethoxy)ethylcarba moyl]-3-ethyl-phenyl]-5- [4-(2-amino-2-oxo-ethoxy)-2,3-difluorophenyl]-1-methylimidazole-2-carboxamide;hydrochlori de | 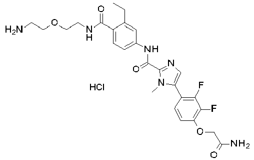 | 545.4 | Byproduct of Example in Step 4 |
| **A22** | N-[4-[2-(2-aminoethoxy)ethylcarba moyl]-3-ethyl-phenyl]-5-[2-chloro-4-(cyanomethoxy)-3- fluoro-phenyl]-1-methylimidazole-2-carboxamide;hydrochlori de | 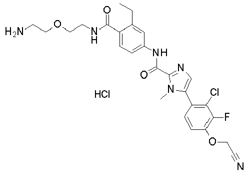 | 543.4 | *tert*-butyl N-[2-(2-aminoethoxy) ethyl] carbama te in step 1 and Intermediate B3 in step 4 |

### Intermediate E1

### (2S,4S)-1-tert-Butoxycarbonyl-4-hydroxy-4-methyl-pyrrolidine-2-carboxylic acid

(2S)-1-*tert*-butoxycarbonyl-4-oxo-pyrrolidine-2-carboxylic acid (2 g, 8.72 mmol) in THF (20 mL) was added dropwise to a solution of methyllithium (8.72 mL, 13.09 mmol) at - 20 °C under a nitrogen atmosphere. The resulting mixture was stirred at the same temperature for 1 h and then further stirred at 25 °C for 11 h. The reaction mixture was added into 1 N aqueous hydrochloric acid solution (50 mL) under ice cooling, followed by extraction with ethyl acetate (50 mL × 3). The organic layer was washed with brine and dried over anhydrous sodium sulfate. The solvent was evaporated under reduced pressure. The crude product was purified by prep-HPLC (FA) to afford 2 final compounds: P1, (2*S*,4*R*)-1-*tert*-butoxycarbonyl-4-hydroxy-4-methyl-pyrrolidine-2-carboxylic acid (50 mg) as a dark green solid and the title compound P2, (2S,4S)-1-*tert*-butoxycarbonyl-4-hydroxy-4-methyl-pyrrolidine-2-carboxylic acid (600 mg) as an off-white solid. MS[M+H]⁺ :190.0.

The following intermediates were prepared in analogy:

| **Int.** | **Name** | **MS ESI [M+H]⁺** | **Starting Material** |
|---|---|---|---|
| **E2** | (*1R*,*4S)-2-tert-*butoxycarbonyl-4-ethyl-4-hydroxy-cyclopentanecarboxylic acid | 282.0 | (2S)-1-*tert*-butoxycarbonyl-4-oxo-pyrrolidine-2-carboxylic acid and Ethylmagnesium bromide |

### Example A1

### N-[4-[2-(2-Aminoethoxy)ethylcarbamoyl]-3-chloro-phenyl]-5-(2,3-difluoro-4-methoxyphenyl)-1-methyl-imidazole-2-carboxamide

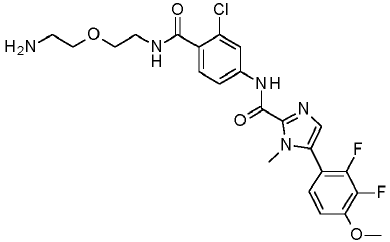

### Step 1: tert-butyl N-[2-[2-[[2-chloro-4-[[5-(2,3-difluoro-4-methoxy-phenyl)-1-methylimidazole-2-carbonyl] amino] benzoyl] amino] ethoxy] ethyl] carbamate

At room temperature, 2,4,6-tripropyl-1,3,5,2,4,6-trioxatriphosphinane 2,4,6-trioxide (226 mg, 711 µmol) was added a mixture of 2-chloro-4-[[5-(2,3-difluoro-4-methoxy-phenyl)-1-methylimidazole-2-carbonyl]amino]benzoic acid (150 mg, 356 µmol), *tert*-butyl N-[2-(2-aminoethoxy)ethyl]carbamate (72.6 mg, 356 µmol) and DIPEA (138 mg, 1.07 mmol) in DMF (2 mL). After stirring for 3 h, the reaction was completed. Then the mixture was poured into water. The water layer was extracted with DCM. The combined organic layers were dried over anhydrous Na₂SO₄ and concentrated to afford the crude product *tert*-butyl N-[2-[2-[[2-chloro-4-[[5-(2,3-difluoro-4-methoxy-phenyl)-1-methyl-imidazole-2-carbonyl]amino]benzoyl]amino]ethoxy]ethyl]carbamate (200 mg). The crude product was used into next step reaction without further purification. MS [M+H]⁺ : 607.9.

### Step 2: N-[4-[2-(2-aminoethoxy)ethylearbamoyl]-3-chloro-phenyl]-5-(2,3-difluoro-4-methoxy-phenyl)-1-methyl-imidazole-2-carboxamide

At room temperature, *tert*-butyl N-[2-[2-[[2-chloro-4-[[5-(2,3-difluoro-4-methoxy-phenyl)-1-methyl-imidazole-2-carbonyl]amino]benzoyl]amino]ethoxy]ethyl]carbamate (200 mg, 329 µmol) in TFA (3 mL) and CH₂Cl₂ (3 mL) was stirred for 1 h. Then the mixture was concentrated. Water (5 mL) was added. The solution was basified by K₂CO₃ to pH 8-9. The water phase was extracted with DCM. The combined organic phases were dried over anhydrous Na₂SO₄ and concentrated. The residue was purified by Prep-HPLC to afford N-[4-[2-(2-aminoethoxy)ethylcarbamoyl]-3-chloro-phenyl]-5-(2,3-difluoro-4-methoxy-phenyl)-1-methylimidazole-2-carboxamide (31 mg) as a white powder. MS [M+H]⁺ : 508.2.

The following compounds were prepared in analogy:

| **Ex#** | **Name** | **Structure** | **MS ESI [M+H]⁺** | **Starting Material** |
|---|---|---|---|---|
| **Example A2** | N-[3-chloro-4-(2-piperazin-1-ylethylcarbamoyl)phenyl ]-5-(2,3-difluoro-4-methoxy-phenyl)-1-methyl-imidazole-2-carboxamide | 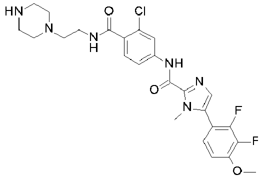 | 533.3 | Intermediate C1 and *tert-*butyl 4-(2-aminoethyl)pi perazine-1-carboxylate |
| **Example A3** | N-[3-chloro-4-(pyrrolidin-3-ylcarbamoyl)phenyl]-5-(2,3 -difluoro-4-methoxyphenyl)-1-methylimidazole-2-carboxamide;formic acid | 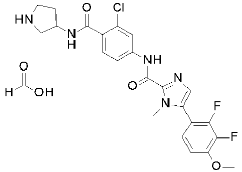 | 490.2 | Intermediate C1 and *tert-*butyl 3-aminopyrrolid ine-1-carboxylate |
| **Example A4** | N-[3-chloro-4-(4-piperidylcarbamoyl)phen yl]-5-(2,3-difluoro-4-methoxy-phenyl)-1-methyl-imidazole-2-carboxamide;formic acid | 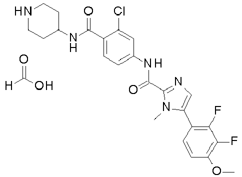 | 504.2 | Intermediate C1 and *tert-*butyl 4-aminopiperidi ne-1-carboxylate |
| **Example A5** | N-[3-chloro-4-(4-piperidylmethylcarbamo yl)phenyl]-5-(2,3-difluoro-4-methoxyphenyl)-1-methylimidazole-2-carboxamide;formic acid | 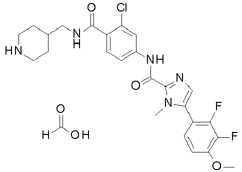 | 518.1 | Intermediate C1 and *tert-*butyl 4-(aminomethyl )piperidine-1-carboxylate |
| **Example A6** | N-[4-(6-aminohexylcarbamoyl)-3 -chloro-phenyl] -5-(2,3-difluoro-4-methoxyphenyl)-1-methylimidazole-2-carboxamide | 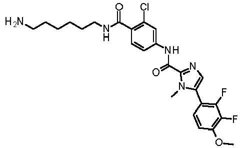 | 520.5 | Intermediate C1 and *tert-*butyl N-(6-aminohexyl)c arbamate |
| **Example A7** | N-[4-[[3-(aminomethyl)cyclobutyl ]methylcarbamoyl]-3-chloro-phenyl]-5-(2,3-difluoro-4-methoxyphenyl)-1-methylimidazole-2-carboxamide;formic acid | 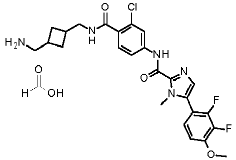 | 518.3 | Intermediate C1 and *tert-*butyl N-[[3-(aminomethyl )cyclobutyl]m ethyl] carbama te |
| **Example A8** | N-[4-[(3-aminocyclobutyl)methylc arbamoyl]-3-chloro-phenyl] -5 -(2,3 -difluoro-4-methoxy-phenyl)-1-methyl-imidazole-2-carboxamide;formic acid | 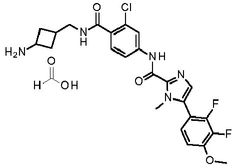 | 504.3 | Intermediate C1 and *tert-*butyl N-[3-(aminomethyl )cyclobutyl]c arbamate |
| **Example A9** | N-[4-[2-(2-aminoethoxy)ethylcarba moyl] -3 -chloro-phenyl] - 5-[4-(cyanomethoxy)-2,3-difluoro-phenyl]-1-methyl-imidazole-2-carboxamide;formic acid | 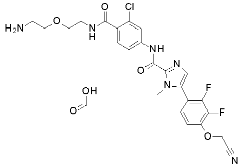 | 533.1 | Intermediate C3 and *tert-*butyl N-[2-(2-aminoethoxy) ethyl] carbama te |
| **Example A10** | N-[3-chloro-4-(2-piperazin-1-ylethylcarbamoyl)phenyl ]-5-[4-(cyanomethoxy)-2,3-difluoro-phenyl]-1-methyl-imidazole-2-carboxamide;2,2,2-trifluoroacetic acid | 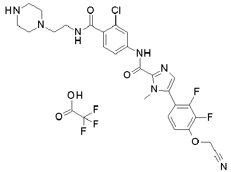 | 558.2 | Intermediate C3 and *tert-*butyl 4-(2-aminoethyl)pi perazine-1-carboxylate |
| **Example A11** | N-[3-chloro-4-(3-piperazin-1-ylpropylcarbamoyl)phen yl]-5-[4-(cyanomethoxy)-2,3-difluoro-phenyl]-1-methyl-imidazole-2-carboxamide;2,2,2-trifluoroacetic acid | 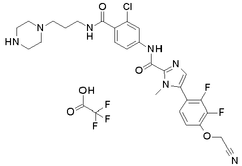 | 572.2 | Intermediate C3 and *tert-*butyl 4-(3-aminopropyl) piperazine-1-carboxylate |
| **Example A12** | 5-[4-(cyanomethoxy)-2,3-difluoro-phenyl]-1-methyl-N-[3-methyl-4-(3-piperazin-1-ylpropylcarbamoyl)phen yl]imidazole-2-carboxamide;2,2,2-trifluoroacetic acid | 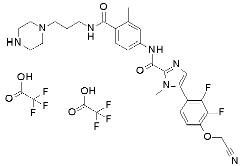 | 552.3 | Intermediate C4 and *tert-*butyl 4-(3-aminopropyl) piperazine-1-carboxylate |
| **Example A13** | N-[4-[2-(2-aminoethoxy)ethylcarba moyl]-3-ethyl-phenyl]-5-(2,3 -difluoro-4-methoxyphenyl)-1-methylimidazole-2-carboxamide;formic acid | 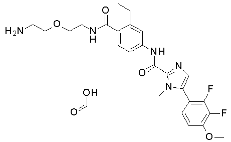 | 502.2 | Intermediate C5 and *tert-*butyl N-[2-(2-aminoethoxy) ethyl] carbama te |
| **Example A14** | N-[4-[2-(2-aminoethoxy)ethylcarba moyl]-3-ethyl-phenyl]-5-[4-(difluoromethoxy)phenyl ]-1-methyl-imidazole-2-carboxamide;hydrochlori de | 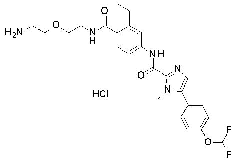 | 502.3 | Intermediate C6 and *tert-*butyl N-[2-(2-aminoethoxy) ethyl] carbama te |
| **Example A15** | N-[4-[2-(2-aminoethoxy)ethylcarba moyl]-3-ethyl-phenyl]-5-(3-fluoro-4-isopropoxyphenyl)-1-methylimidazole-2-carboxamide;hydrochlori de | 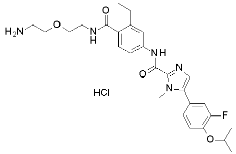 | 512.3 | Intermediate C7 and *tert-*butyl N-[2-(2-aminoethoxy) ethyl] carbama te |
| **Example A16** | N-[4-[2-(2-aminoethoxy)ethylcarba moyl]-3-ethyl-phenyl]-5-(2-chloro-4-methoxyphenyl)-1-methylimidazole-2-carboxamide;hydrochlori de | 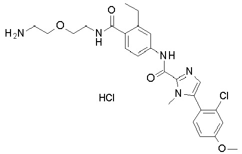 | 500.3 | Intermediate C8 and *tert-*butyl N-[2-(2-aminoethoxy) ethyl] carbama te |
| **Example A17** | N-[4-[2-(2-aminoethoxy)ethylcarba moyl]-3-ethyl-phenyl]-5-[4-(difluoromethoxy)-2,3-difluoro-phenyl]-1-methyl-imidazole-2-carboxamide;hydrochlori de | 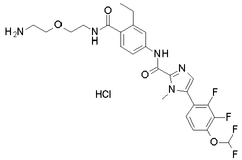 | 538.1 | Intermediate C9 and *tert-*butyl N-[2-(2-aminoethoxy) ethyl] carbamate |
| **Example A18** | N-[4-[2-(2-aminoethoxy)ethylcarba moyl]-3-ethyl-phenyl]-5-(2, 6-difluoro-4-methoxyphenyl)-1-methylimidazole-2-carboxamide;hydrochlori de | 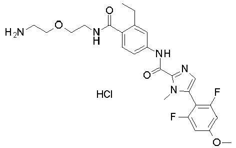 | 502.3 | Intermediate C10 and *tert-*butyl N-[2-(2-aminoethoxy) ethyl] carbama te |

### Example B1

### N-[3-Chloro-4-[[1-(piperidine-4-carbonyl)pyrrolidin-3-yl]carbamoyl]phenyl]-5-(2,3-difluoro-4-methoxy-phenyl)-1-methyl-imidazole-2-carboxamide;formic acid

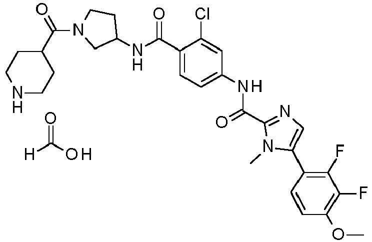

### Step 1: tert-butyl 4-[3-[[2-chloro-4-[[5-(2,3-difluoro-4-methoxy-phenyl)-1-methyl-imidazole-2-carbonyl]amino]benzoyl]amino]pyrrolidine-1-carbonyl]piperidine-1-carboxylate

A mixture of N-[3-chloro-4-(pyrrolidin-3-ylcarbamoyl)phenyl]-5-(2,3-difluoro-4-methoxyphenyl)-1-methyl-imidazole-2-carboxamide (200 mg, 408 µmol), 1-*tert-*butoxycarbonylpiperidine-4-carboxylic acid (187 mg, 816 µmol), HATU (233 mg, 612 µmol) and DIPEA (158 mg, 1.22 mmol) in DMF (5 mL) was stirred overnight. Then the mixture was poured into water. The water layer was extracted with DCM. The combined organic layers were washed with water, dried over anhydrous Na₂SO₄ and concentrated to afford the crude product *tert*-butyl 4-[3-[[2-chloro-4-[[5-(2,3-difluoro-4-methoxy-phenyl)-1-methyl-imidazole-2-carbonyl]amino]benzoyl]amino]pyrrolidine-1-carbonyl]piperidine-1-carboxylate (200 mg). The crude product was used into next step reaction directly.

### Step 2: N-[3-chloro-4-[[1-(piperidine-4-carbonyl)pyrrolidin-3-yl]carbamoyl]phenyl]-5-(2,3-difluoro-4-methoxy-phenyl)-1-methyl-imidazole-2-carboxamide;formic acid

A solution of *tert*-butyl 4-[3-[[2-chloro-4-[[5-(2,3-difluoro-4-methoxy-phenyl)-1-methylimidazole-2-carbonyl] amino] benzoyl]amino]pyrrolidine-1-carbonyl] piperidine-1 -carboxylate (200 mg, 285 µmol) in TFA (5 mL) and DCM (5 mL) was stirred for 30 min. Then the mixture was concentrated. Water (5 mL) was added. The water layer was basified by NH₃.H₂O. The water layer was extracted with DCM. The combined organic layers were washed with water, dried over anhydrous Na₂SO₄ and concentrated. The residue was purified by Prep-HPLC to afford N-[3-chloro-4-[[1-(piperidine-4-carbonyl)pyrrolidin-3-yl]carbamoyl]phenyl]-5-(2,3-difluoro-4-methoxy-phenyl)-1-methyl-imidazole-2-carboxamide;formic acid (85 mg). MS [M+H]⁺ : 601.4.

The following compounds were prepared in analogy:

| **Ex#** | **Name** | **Structure** | **MS ESI [M+H]⁺** | **Starting Material** |
|---|---|---|---|---|
| **Example B2** | N-[3-chloro-4-[[1-[(3R)-pyrrolidine-3-carbonyl]pyrrolidin-3- yl]carbamoyl]phenyl]-5- (2,3-difluoro-4-methoxyphenyl)-1-methylimidazole-2-carboxamide;formic acid | 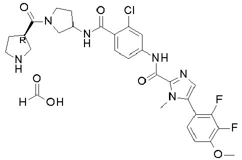 | 587.3 | Intermediate D1 and (3*R*)-1*-tert-*butoxycarbon ylpyrrolidine-3-carboxylic acid |
| **Example B3** | N-[3-chloro-4-[[1-[(2*S*,4*R*)-4-hydroxypyrrolidine-2-carbonyl]pyrrolidin-3- yl]carbamoyl]phenyl]-5-(2,3 -difluoro-4-methoxyphenyl)-1-methylimidazole-2-carboxamide;formic acid | 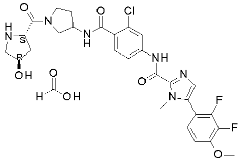 | 603.3 | Intermediate D1 and (2*S*,4*R*)-1-*tert-*butoxycarbonyl-4-hydroxy-pyrrolidine-2-carboxylic acid |
| **Example B4** | N-[4-[[1-[3-(2-aminoethoxy)propanoyl] pyrrolidin-3-yl]carbamoyl]-3-chloro-phenyl]-5-(2,3-difluoro-4-methoxy-phenyl)-1-methyl-imidazole-2-carboxamide;formic acid | 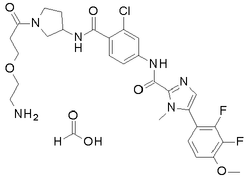 | 605.4 | Intermediate D1 and 3-[2-(*tert-*butoxycarbon ylamino)etho xy]propanoic acid |
| **Example B5** | N-[3-chloro-4-[[1-(piperidine-4-carbonyl)-4-piperidyl] carbamoyl] phe nyl]-5-(2,3-difluoro-4-methoxy-phenyl)-1-methyl-imidazole-2-carboxamide;formic acid | 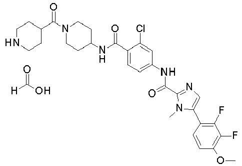 | 615.2 | Intermediate D2 and 1*-tert-*butoxycarbon ylpiperidine-4-carboxylic acid |
| **Example B6** | N-[3-chloro-4-[[1-[(3*R*)-pyrrolidine-3-carbonyl]-4-piperidyl] carbamoyl] phe nyl]-5-(2,3-difluoro-4-methoxy-phenyl)-1-methyl-imidazole-2-carboxamide;formic acid | 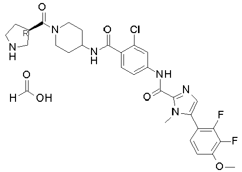 | 601.2 | Intermediate D2 and (3*R*)-1*-tert-*butoxycarbon ylpyrrolidine-3-carboxylic acid |
| **Example B7** | N-[3-chloro-4-[[1-[(2*S*,4*R*)-4-hydroxypyrrolidine-2-carbonyl]-4-piperidyl]carbamoyl]phe nyl]-5-(2,3-difluoro-4-methoxy-phenyl)-1-methyl-imidazole-2-carboxamide | 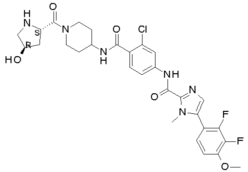 | 617.4 | Intermediate D2 and (2*S*,4*R*)-1-*tert-*butoxycarbon yl-4-hydroxy-pyrrolidine-2-carboxylic acid |
| **Example B8** | N-[4-[[1-[3-(2-aminoethoxy)propanoyl]- 4-piperidyl]carbamoyl]-3-chloro-phenyl]-5-(2,3-difluoro-4-methoxyphenyl)-1-methylimidazole-2-carboxamide;formic acid | 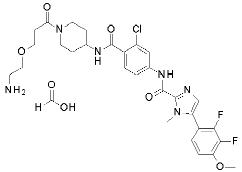 | 619.2 | Intermediate D2 and 3-[2-(*tert-*butoxycarbon ylamino)etho xy]propanoic acid |
| **Example B9** | N-[4-[[3-[[3-(aminomethyl)cyclobuta necarbonyl]amino]cyclob utyl]carbamoyl]-3- chloro-phenyl]-5-(2,3-difluoro-4-methoxyphenyl)-1-methylimidazole-2-carboxamide;formic acid | 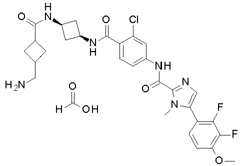 | 601.4 | Intermediate D4 and 3-[(*tert-*butoxycarbon ylamino)meth yl]cyclobutan ecarboxylic acid |
| **Example B10** | N-[4-[2-[1-(3-aminopropanoyl)azetidin -3-yl]ethylcarbamoyl]-3-chloro-phenyl]-5-[4-(difluoromethoxy)-2,3-difluoro-phenyl]-1-methyl-imidazole-2-carboxamide;2,2,2-trifluoroacetic acid | 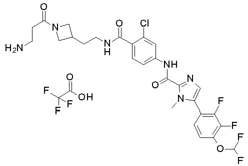 | 611.2 | Intermediate D5 and 3-(*tert-*butoxycarbonylamino)prop anoic acid |
| **Example B11** | N-[4-[3-(3-aminopropanoylamino)pr opylcarbamoyl]-3-chloro-phenyl]-5-[4-(difluoromethoxy)-2,3-difluoro-phenyl]-1-methyl-imidazole-2-carboxamide;2,2,2-trifluoroacetic acid | 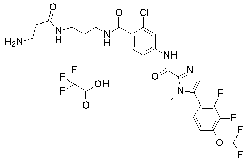 | 585.1 | Intermediate D6 and 3-(*tert-*butoxycarbon ylamino)prop anoic acid |
| **Example B12** | N-[3-[[4-[[5-[4-(difluoromethoxy)-2,3-difluoro-phenyl]-1-methyl-imidazole-2-carbonyl] amino]-2-ethyl-benzoyl] amino] propyl] pi peridine-4-carboxamide;formic acid | 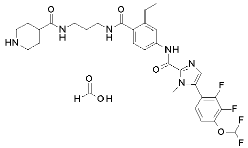 | 619.4 | Intermediate D7 and *1-tert-*butoxycarbon ylpiperidine-4-carboxylic acid |
| **Example B13** | N-[2-[[4-[[5-[4-(cyanomethoxy)-2,3-difluoro-phenyl]-1-methyl-imidazole-2-carbonyl] amino]-2-methylbenzoyl] amino] ethyl]pip eridine-4-carboxamide;formic acid | 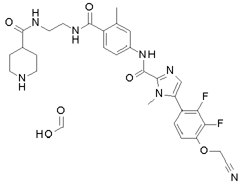 | 580.1 | Intermediate D8 and 1*-tert-*butoxycarbon ylpiperidine-4-carboxylic acid |
| **Example B14** | N-[2-[[4-[[5-[4-(cyanomethoxy)-2,3-difluoro-phenyl]-1-methyl-imidazole-2-carbonyl] amino]-2-methylbenzoyl] amino] ethyl]-4-hydroxy-piperidine-4-carboxamide;formic acid | 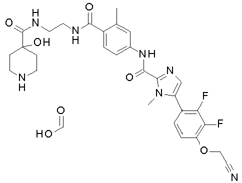 | 596.1 | Intermediate D8 and *1-tert-*butoxycarbon yl-4-hydroxy-piperidine-4-carboxylic acid |
| **Example B15** | N-[2-[[2-chloro-4-[[5-[4-(cyanomethoxy)-2,3-difluoro-phenyl]-1-methyl-imidazole-2-carbonyl] amino]benzoyl] amino]ethyl]piperidine-4-carboxamide;2,2,2-trifluoroacetic acid | 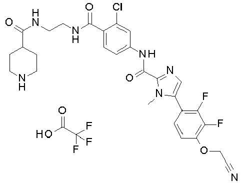 | 600.2 | Intermediate D9 and *1-tert-*butoxycarbon ylpiperidine-4-carboxylic acid |
| **Example B16** | N-[2-[[2-chloro-4-[[5-[4-(cyanomethoxy)-2,3-difluoro-phenyl]-1-methyl-imidazole-2-carbonyl]amino]benzoyl] amino]ethyl]-4-hydroxy-piperidine-4-carboxamide;2,2,2-trifluoroacetic acid | 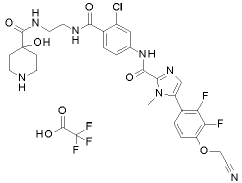 | 618.2 | Intermediate D9 and 1*-tert-*butoxycarbon yl-4-hydroxy-piperidine-4-carboxylic acid |
| **Example B17** | N-[4-[[1-(2-aminoacetyl)azetidin-3-yl]carbamoyl]-3-chloro-phenyl]-5-(2,3-difluoro-4-methoxy-phenyl)-1-methyl-imidazole-2-carboxamide;formic acid | 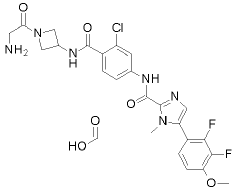 | 533.1 | Intermediate D10 and 2-(*tert-*butoxycarbonylamino)aceti c acid acid |
| **Example B18** | N-[4-[[1-(2-aminoacetyl)azetidin-3-yl]methylcarbamoyl]-3- chloro-phenyl]-5-(2,3-difluoro-4-methoxyphenyl)-1-methylimidazole-2-carboxamide;formic acid | 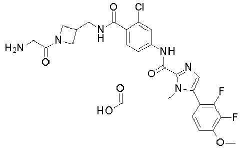 | 547.1 | Intermediate D11 and 2-(*tert-*butoxycarbon ylamino)aceti c acid acid |
| **Example B19** | N-[4-[[3-(2-aminoethylcarbamoyl)cy clobutyl]carbamoyl]-3-chloro-phenyl]-5-(2,3-difluoro-4-methoxyphenyl)-1-methylimidazole-2-carboxamide;formic acid | 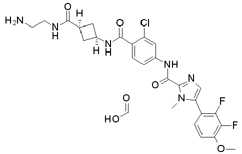 | 561.1 | Intermediate D12 and *tert-*butyl N-(2-aminoethyl)ca rbamate |
| **Example B20** | N-[4-[[3-(3-aminoazetidine-1-carbonyl)cyclobutyl]carb amoyl]-3-chloro-phenyl]-5-(2,3-difluoro-4-methoxy-phenyl)-1-methyl-imidazole-2-carboxamide;formic acid | 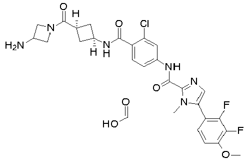 | 573.1 | Intermediate D12 and *tert-*butyl N-(azetidin-3-yl)carbamate |
| **Example B21** | N-[4-[[3-[(3-aminocyclobutyl)carbam oyl]cyclobutyl]carbamoy l]-3-chloro-phenyl]-5- (2,3-difluoro-4-methoxyphenyl)-1-methylimidazole-2-carboxamide;2,2,2-trifluoroacetic acid | 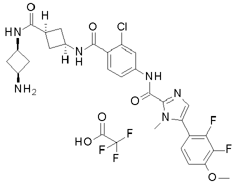 | 587.2 | Intermediate D12 and *tert-*butyl N-(3-aminocyclobu tyl)carbamate |
| **Example B22** | N-[4-[[3-(2-aminoethylcarbamoyl)cy clobutyl]carbamoyl]-3-ethyl-phenyl]-5-(2,3-difluoro-4-methoxyphenyl)-1-methylimidazole-2-carboxamide;2,2,2-trifluoroacetic acid | 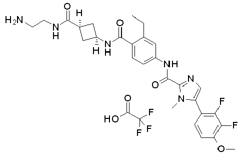 | 555.2 | Intermediate D13 and *tert-*butyl N-(2-aminoethyl)ca rbamate |
| **Example B23** | N-[4-[[1-(2-aminoacetyl)azetidin-3-yl]carbamoyl]-3-ethyl-phenyl]-5-(2,3-difluoro-4-methoxy-phenyl)-1-methyl-imidazole-2-carboxamide;formic acid | 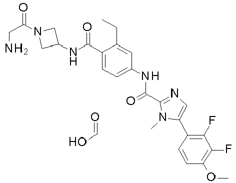 | 527.2 | Intermediate D13 and 2-(*tert-*butoxycarbon ylamino)aceti c acid acid |
| **Example B24** | 5-[4-(cyanomethoxy)-2,3-difluoro-phenyl]-N-[4-[2-[[(2R,4R)-4-hydroxy-4-methyl-pyrrolidine-2-carbonyl] amino] ethylcarbamoyl]-3-methylphenyl]-1-methylimidazole-2-carboxamide;formic acid | 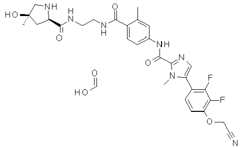 | 596.2 | Intermediate D8 and E1 |
| **Example B25** | 5-[4-(cyanomethoxy)-2,3-difluoro-phenyl]-N-[4-[2-[[(2R,4R)-4-ethyl-4-hydroxy-pyrrolidine-2-carbonyl] amino] ethylcar bamoyl]-3-methylphenyl]-1-methylimidazole-2-carboxamide;formic acid | 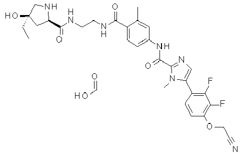 | 610.2 | Intermediate D8 and E2 |
| **Example B26** | 5-(2,3-difluoro-4-methoxy-phenyl)-N-[4-[2-[[(2R,4R)-4-hydroxy-4-methyl-pyrrolidine-2-carbonyl]amino]ethylcar bamoyl]-3-methylphenyl]-1-methylimidazole-2-carboxamide;formic acid | 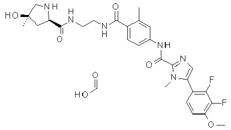 | 571.3 | Intermediate D14 and E1 |
| **Example B27** | 5-(2,3-difluoro-4-methoxy-phenyl)-N- [4-[2-[[(2*R*,4*R*)-4-ethyl-4-hydroxy-pyrrolidine-2-carbonyl] amino] ethylcar bamoyl]-3-methylphenyl]-1-methylimidazole-2-carboxamide;formic acid | 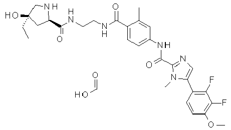 | 585.4 | Intermediate D14 and E2 |
| **Example B28** | 5-(2,3-difluoro-4-methoxy-phenyl)-N-[4-[2-[[(3*R*,4*R*)-4-hydroxypyrrolidin-3-yl] carbamoylamino] ethyl carbamoyl]-3-methylphenyl]-1-methylimidazole-2-carboxamide;formic acid | 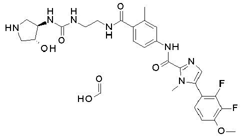 | 572.4 | Intermediate D14 and *tert-*butyl (3*R*,4*R*)-3-amino-4-hydroxy-pyrrolidine-1-carboxylate |
| **Example B29** | 5-(2,3-difluoro-4-methoxy-phenyl)-N-[4-[2-[[(3*S*,4*S*)-4-hydroxypyrrolidin-3-yl]carbamoylamino]ethyl carbamoyl]-3-methylphenyl]-1-methylimidazole-2-carboxamide;formic acid | 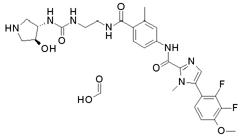 | 527.4 | Intermediate D14 and *tert-*butyl (3*S*,4*S*)-3-amino-4-hydroxy-pyrrolidine-1-carboxylate |
| **Example B30** | 5-[4-(cyanomethoxy)-2,3-difluoro-phenyl]-N-[4-[2-[[(3*R*,4*R*)-4-hydroxypyrrolidin-3-yl] carbamoylamino] ethyl carbamoyl]-3-methylphenyl]-1-methylimidazole-2-carboxamide;formic acid | 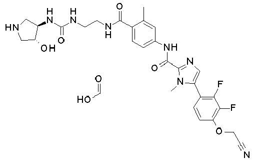 | 597.4 | Intermediate D8 and *tert-*butyl (3*R*,4*R*)-3-amino-4-hydroxy-pyrrolidine-1-carboxylate |
| **Example B31** | 5-[4-(cyanomethoxy)-2,3-difluoro-phenyl]-N-[4-[2-[[(3*S*,4*S*)-4-hydroxypyrrolidin-3-yl]carbamoylamino]ethyl carbamoyl]-3-methylphenyl]-1-methylimidazole-2-carboxamide;formic acid | 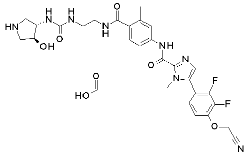 | 597.4 | Intermediate D8 and *tert-*butyl (3*S*,4*S*)-3-amino-4-hydroxy-pyrrolidine-1-carboxylate |
| **Example B32** | (3*R*,4*R*)-N-[2-[[2-chloro-4-[[5-[4-(cyanomethoxy)-2,3-difluoro-phenyl]-1-methyl-imidazole-2-carbonyl] amino]benzoyl] amino] ethyl]-3-hydroxy-piperidine-4-carboxamide;formic acid | 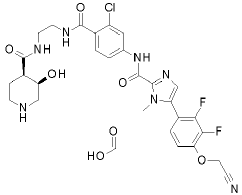 | 616.2 | Intermediate D9 and (3*R*,4*R*)-1-*tert-*butoxycarbon yl-3-hydroxy-piperidine-4-carboxylic acid |
| **Example B33** | (3*S*,4*S*)-N-[2-[[2-chloro-4-[[5-[4-(cyanomethoxy)-2,3-difluoro-phenyl]-1-methyl-imidazole-2-carbonyl]amino]benzoyl] amino]ethyl]-3-hydroxy-piperidine-4-carboxamide;formic acid | 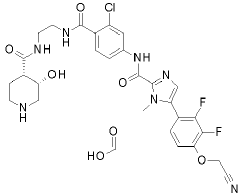 | 616.3 | Intermediate D9 and (3*S*,4*S*)-1-*tert-*butoxycarbon yl-3-hydroxy-piperidine-4-carboxylic acid |
| **Example B34** | (3*S*,4*R*)-N-[2-[[2-chloro-4-[[5-[4-(cyanomethoxy)-2,3-difluoro-phenyl]-1-methyl-imidazole-2-carbonyl]amino]benzoyl] amino]ethyl]-3-hydroxy-piperidine-4-carboxamide;formic acid | 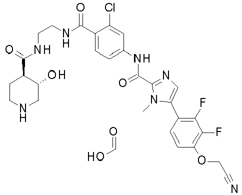 | 616.1 | Intermediate D9 and (3*S*,4*R*)-1-*tert-*butoxycarbon yl-3-hydroxy-piperidine-4-carboxylic acid |
| **Example B35** | (3*R*,4*S*)-N-[2-[[2-chloro-4-[[5-[4-(cyanomethoxy)-2,3-difluoro-phenyl]-1-methyl-imidazole-2-carbonyl] amino] benzoyl] amino]ethyl]-3-hydroxy-piperidine-4-carboxamide;formic acid | 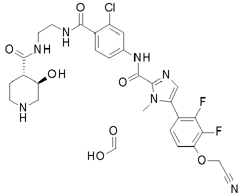 | 616.1 | Intermediate D9 and (3*R*,4*S*)-1-*tert-*butoxycarbon yl-3-hydroxy-piperidine-4-carboxylic acid |
| **Example B36** | N-[3-chloro-4-[2-[[(2*R*,4*R*)-4-hydroxy-4-methyl-pyrrolidine-2-carbonyl] amino] ethylcar bamoyl]phenyl]-5-[4-(cyanomethoxy)-2,3-difluoro-phenyl]-1-methyl-imidazole-2-carboxamide;formic acid | 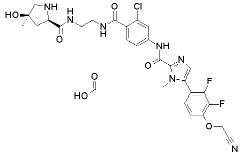 | 616.2 | Intermediate D9 and E1 |
| **Example B37** | N-[3-chloro-4-[2-[[(2*R*,4*R*)-4-ethyl-4-hydroxy-pyrrolidine-2-carbonyl] amino] ethylcar bamoyl]phenyl]-5-[4-(cyanomethoxy)-2,3-difluoro-phenyl]-1-methyl-imidazole-2-carboxamide;formic acid | 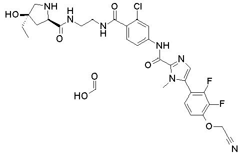 | 630.2 | Intermediate D9 and E2 |
| **Example B38** | N-[3-chloro-4-[[3-[[(4R)-4-hydroxypyrrolidine-2-carbonyl]amino]cyclobut yl]carbamoyl]phenyl]-5- (2,3-difluoro-4-methoxyphenyl)-1-methylimidazole-2-carboxamide;formic acid | 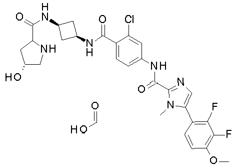 | 603.3 | Intermediate D4 and BOC-HYP-OH |
| **Example B39** | N-[3-[[2-chloro-4-[[5-(2,3 -difluoro-4-methoxyphenyl)-1-methylimidazole-2-carbonyl]amino]benzoyl] amino]cyclobutyl]piperid ine-4-carboxamide;formic acid | 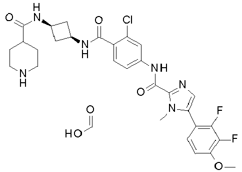 | 601.3 | Intermediate D4 and N-BOC-isonipecotic acid |
| **Example B40** | N-[3-[[2-chloro-4-[[5-(2,3-difluoro-4-methoxyphenyl)-1-methylimidazole-2-carbonyl] amino] benzoyl] amino]cyclobutyl]-4-hydroxy-piperidine-4-carboxamide;formic acid | 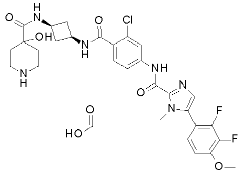 | 617.3 | Intermediate D4 and 1-*tert-*butoxycarbon yl-4-hydroxy-piperidine-4-carboxylic acid |
| **Example B41** | (3*R*,4*R*)-N-[3-[[2-chloro-4-[[5-(2,3-difluoro-4-methoxy-phenyl)-1-methyl-imidazole-2-carbonyl]amino]benzoyl] amino]cyclobutyl]-3-hydroxy-piperidine-4-carboxamide;formic acid | 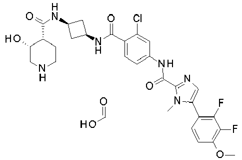 | 617.3 | Intermediate D4 and (3*R*,4*R*)-1-*tert-*butoxycarbon yl-3-hydroxy-piperidine-4-carboxylic acid |
| **Example B42** | (3*S*,4*S*)-N-[3-[[2-chloro-4-[[5-(2,3-difluoro-4-methoxy-phenyl)-1-methyl-imidazole-2-carbonyl] amino]benzoyl] amino] cyclobutyl] -3-hydroxy-piperidine-4-carboxamide;formic acid | 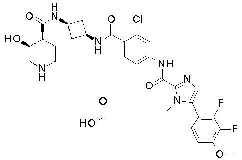 | 617.3 | Intermediate D4 and (3S,4S)-1-*tert-*butoxycarbon yl-3-hydroxy-piperidine-4-carboxylic acid |

### Example C1

### N-[4-[2-[1-(Azetidin-3-ylmethyl)azetidin-3-yl]ethylcarbamoyl]-3-chloro-phenyl]-5-[4-(difluoromethoxy)-2,3-difluoro-phenyl]-1-methyl-imidazole-2-carboxamide;2,2,2-trifluoroacetic acid

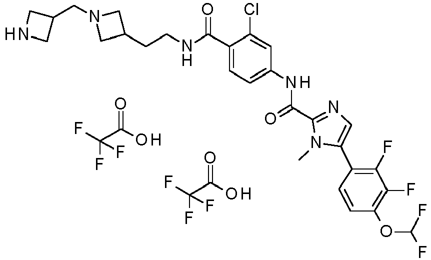

### Step 1: tert-butyl 3-[[3-[2-[[2-chloro-4-[[5-[4-(difluoromethoxy)-2,3-difluoro-phenyl]-1-methyl-imidazole-2-carbonyl]amino]benzoyl]amino]ethyl]azetidin-1-yl]methyl]

### azetidine-1-carboxylate

In a 50 mL round-bottomed flask , N-(4-((2-(azetidin-3-yl)ethyl)carbamoyl)-3-chlorophenyl)-5-(4-(difluoromethoxy)-2,3-difluorophenyl)-1-methyl-1H-imidazole-2-carboxamide (118 mg, 219 µmol), *tert*-butyl 3-formylazetidine-1-carboxylate (121 mg, 656 µmol) and NaBH₃CN (68.7 mg, 1.09 mmol) were combined with MeOH (6 mL) to give a light yellow solution .The reaction mixture was heated to 45 °C and stirred for 3 h .The crude reaction mixture was concentrated in vacuum. The reaction mixture was poured into 25 mL sat NaHCO₃ and extracted with EtOAc (25 mL × 3).The organic layers were combined, washed with sat NaCl (25 mL), The organic layers were dried over Na₂SO₄ and concentrated in vacuum.to afford tert-butyl 3-((3-(2-(2-chloro-4-(5-(4-(difluoromethoxy)-2,3-difluorophenyl)-1-methyl-1H-imidazole-2-carboxamido)benzamido)
ethyl)azetidin-1-yl)methyl)azetidine-1-carboxylate (155 mg) , MS [M+H]⁺ : 709.1.

### Step 2: N-[4-[2-[1-(azetidin-3-ylmethyl)azetidin-3-yl]ethylcarbamoyl]-3-chloro-phenyl]-5-[4-(difluoromethoxy)-2,3-difluoro-phenyl]-1-methyl-imidazole-2-carboxamide;2,2,2-trifluoroacetic acid

In a 50 mL round-bottomed flask, *tert*-butyl 3-((3-(2-(2-chloro-4-(5-(4-(difluoromethoxy)-2,3-difluorophenyl)-1-methyl-1H-imidazole-2-carboxamido)benzamido)ethyl)azetidin-1-yl)methyl)azetidine-1-carboxylate (35 mg, 49.4 µmol) was combined with DCM (3 mL) to give a light brown solution. 2,2,2-trifluoroacetic acid (1.13 g, 9.87 mmol) was added. The reaction was stirred at room temperature for 30 min. The crude reaction mixture was concentrated in vacuum. The crude material was purified by preparative HPLC.to afford N-(4-((2-(1-(azetidin-3-ylmethyl)azetidin-3-yl)ethyl)carbamoyl)-3-chlorophenyl)-5-(4-(difluoromethoxy)-2,3-difluorophenyl)-1-methyl-1H-imidazole-2-carboxamide bis(2,2,2-trifluoroacetate) (12 mg), MS [M+H]⁺ : 609.2.

The following compounds were prepared in analogy:

| **Ex#** | **Name** | **Structure** | **MS ESI [M+H]⁺** | **Starting Material** |
|---|---|---|---|---|
| **Example C2** | N-[3-[[4-[[5-[4-(difluoromethoxy)-2,3-difluoro-phenyl]-1-methyl-imidazole-2-carbonyl]amino]-2-ethyl-benzoyl]amino]propyl]-1-(pyrrolidin-3-ylmethyl)piperidine-4-carboxamide;formic acid | 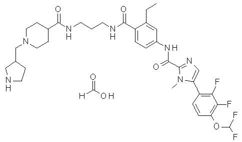 | 702.4 | Example B15 and *tert*-butyl 3-formylpyrrolidine-1-carboxylate |
| **Example C3** | 1-(azetidin-3-ylmethyl)-N-[3-[[4-[[5-[4-(difluoromethoxy)-2,3-difluoro-phenyl]-1-methyl-imidazole-2-carbonyl]amino]-2-ethyl-benzoyl]amino]propyl] pi peridine-4-carboxamide;formic acid | 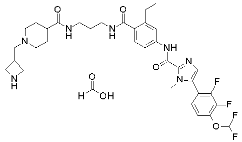 | 688.0 | Example B15 and *tert*-butyl 3-formylazetidi ne-1-carboxylate |
| **Example C4** | N-[4-[[1-(azetidin-3-ylmethyl)-4-piperidyl]methylcarbamo yl]-3-chloro-phenyl]-5- (2,3-difluoro-4-methoxyphenyl)-1-methylimidazole-2-carboxamide;2,2,2-trifluoroacetic acid | 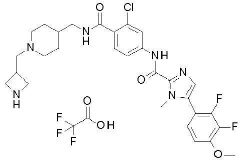 | 587.4 | Example A5 and *tert*-butyl 3-formylazetidi ne-1-carboxylate |

### Example A23

### N-[3-Chloro-4-[[1-[2-(dimethylamino)acetyl]pyrrolidin-3-yl]carbamoyl]phenyl]-5-(2,3-difluoro-4-methoxy-phenyl)-1-methyl-imidazole-2-carboxamide

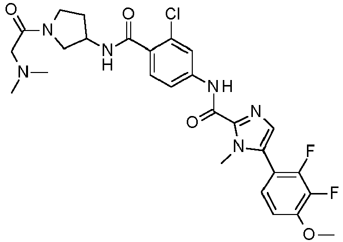

A mixture of N-[3-chloro-4-(pyrrolidin-3-ylcarbamoyl)phenyl]-5-(2,3-difluoro-4-methoxyphenyl)-1-methyl-imidazole-2-carboxamide (300 mg, 612 µmol), 2-(dimethylamino)acetic acid (126 mg, 1.22 mmol), HATU (349 mg, 919 µmol) and DIPEA (237 mg, 1.84 mmol) in DMF (5 mL) was stirred overnight. The mixture was poured into water. The water layer was extracted with DCM. The organic layer was washed with water, dried over anhydrous Na₂SO₄ and concentrated. The residue was purified by Prep-HPLC to afford N-[3-chloro-4-[[1-[2-(dimethylamino)acetyl]pyrrolidin-3-yl]carbamoyl]phenyl]-5-(2,3-difluoro-4-methoxy-phenyl)-1-methyl-imidazole-2-carboxamide 130 mg. MS [M+H]⁺ : 575.5.

The following compounds were prepared in analogy:

| **Ex#** | **Name** | **Structure** | **MS ESI [M+H]⁺** | **Starting Material** |
|---|---|---|---|---|
| **Example A24** | N-[3-chloro-4-[2-[[2-(dimethylamino)acetyl] a mino] ethylcarbamoyl] ph enyl]-5-(2,3-difluoro-4-methoxy-phenyl)-1-methyl-imidazole-2-carboxamide;2,2,2-trifluoroacetic acid | 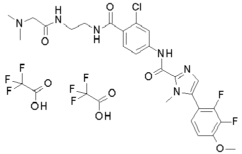 | 549.3 | Intermediate D3 and 2-(dimethylami no)acetic acid |
| **Example A25** | N-[3-chloro-4-[[1-[2-(dimethylamino)acetyl]- 4-piperidyl] carbamoyl] phe nyl]-5-(2,3-difluoro-4-methoxy-phenyl)-1-methyl-imidazole-2-carboxamide | 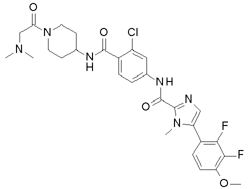 | 589.2 | Intermediate D2 and 2-(dimethylami no)acetic acid |
| **Example A26** | N-[3-chloro-4-[5-(dimethylamino)pentylca rbamoyl]phenyl]-5-(2,3-difluoro-4-methoxyphenyl)-1-methylimidazole-2-carboxamide | 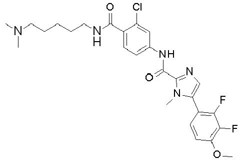 | 534.0 | Intermediate C1 and N',N'-dimethylpenta ne-1,5-diamine |
| **Example A27** | N-[3-chloro-4-(2-isopentyloxyethylcarbam oyl)phenyl]-5-[4-(cyanomethoxy)-2,3-difluoro-phenyl]-1-methyl-imidazole-2-carboxamide;formic acid | 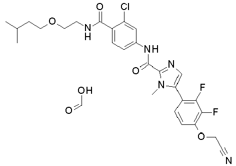 | 561.1 | Intermediate C3 and 2-[2-(dimethylami no)ethoxy]eth anamine dihydrochlori de |
| **Example A28** | N-[3-chloro-4-[2-(3-hydroxypyrrolidin-1- yl)ethylcarbamoyl]pheny l]-5-(2,3-difluoro-4-methoxy-phenyl)-1-methyl-imidazole-2-carboxamide | 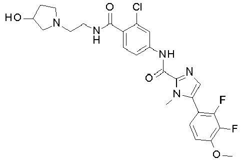 | 534.1 | Intermediate C1 and 1-(2-aminoethyl)p yrrolidin-3-ol |
| **Example A29** | N-[3-chloro-4-[2-[3-(hydroxymethyl)pyrrolidi n-1-yl] ethylcarbamoyl]pheny l]-5-(2,3-difluoro-4-methoxy-phenyl)-1-methyl-imidazole-2-carboxamide | 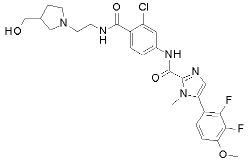 | 548.1 | Intermediate C1 and [1-(2-aminoethyl)p yrrolidin-3-yl]methanol |
| **Example A30** | 5-(2,3-difluoro-4-methoxy-phenyl)-N- [4-[3-[2-(dimethylamino)ethoxy]p ropylcarbamoyl]-3-ethyl-phenyl]-1-methylimidazole-2-carboxamide | 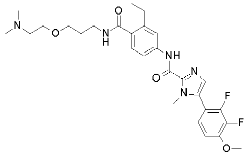 | 530.3 | Intermediate C5 and 2-(2-aminoethoxy) -N,N-dimethylethan -1-amine |

### Example D1

### 5-(4-Methoayphenyl)-1-methyl-N-[3-methyl-4-(methylcarbamoyl)phenyl]imidazole-2-carboxamide

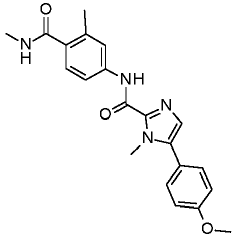

### Step 1) Ethyl 5-bromo-1-methyl-imidazole-2-carboxylate

To a stirred solution of ethyl chloroformate (2.38 mL, 24.84 mmol) and N,N-diisopropylethylamine (4.33 mL, 24.84 mmol) in ACN (50 mL) was slowly added a solution of 5-bromo-1-methyl-1H-imidazole (2 g, 12.42 mmol) in ACN (20 mL) at 0 °C. The mixture was stirred at 10 °C for 16 h. The mixture was concentrated and the residue was treated with water (100 mL) and extracted with EtOAc (100 mL × 3). The combined extracts were washed with brine, dried with MgSO₄, filtered, concentrated and purified on flash column chromatography (0-50% of EtOAc in hexanes) to afford the title compound (2.4 g) as a light yellow oil. MS (ESI, m/z): 233.0 [M+H]⁺.

### Step 2) Ethyl 5-(4-methoxyphenyl)-1-methyl-imidazole-2-carboxylate

A mixture of ethyl 5-bromo-1-methyl-imidazole-2-carboxylate (1 g, 4.29 mmol), 4-methoxyphenylboronic acid (717 mg, 4.72 mmol), [1,1'-Bis(diphenylphosphino)ferrocene]dichloropalladium (II) (314 mg, 0.430 mmol) and Sodium carbonate (0.91 g, 8.58 mmol) in 1,4-dioxane (18 mL) /water (2 mL) was stirred under N₂ at 90 °C for 16 h. The mixture was filtered and purified by silica column to afford the title compound (710 mg, 2.73 mmol) as a yellow solid. MS (ESI, m/z): 261.1 [M+H]⁺.

### Step 3) 5-(4-methoxyphenyl)-1-methyl-imidazole-2-carboxylic acid

A solution of ethyl 5-(4-methoxyphenyl)-1-methyl-imidazole-2-carboxylate (710 mg, 2.73 mmol) in THF (10 mL)/water (10 mL)/methanol (5 mL) was added lithium hydroxide (218.2 mg, 5.46 mmol) and it was stirred at 5 °C for 16 h. The mixture was poured into water (50 mL) and extracted with EtOAc (30 mL × 2), then the water layer was acidified to pH= 3 with HCl solution (1N) and extracted with EtOAc (50 mL × 2). The organic layers were concentrated to afford the title compound (205 mg) as a light yellow solid. MS (ESI, m/z): 233.0 [M+H]⁺.

### Step 4) N, 2-dimethyl-4-nitro-benzamide

A solution of methyl 2-methyl-4-nitro-benzoate (2.8 g, 14.4 mmol) in methylamine in Ethanol (30 mL, 5.0 mmol) was stirred at 70 °C for 3 h. The solution was concentrated and washed by MTBE/DCM (100 mL, 50:1) to afford the title compound (2.65 g, 13.65 mmol) as an off-white solid.

### Step 5) 4-amino-N,2-dimethyl-benzamide

A solution of Palladium (0.14 mL, 1.36 mmol) and N,2-dimethyl-4-nitro-benzamide (2.65 g, 13.6 mmol) in methanol (30 mL) was stirred under H₂ (2287 mmHg) at 5 °C for 16 h. The solution was concentrated and washed with MTBE (100 mL) to afford the title compound (1.8 g) as a white solid. MS (ESI, m/z): 165.2 [M+H]⁺.

### Step 6) 5-(4-methoayphenyl)-1-methyl-N-[3-methyl-4-(methylcarbamoyl) phenyl] imidazole-2-carboxamide

To a stirred solution of 5-(4-methoxyphenyl)-1-methyl-imidazole-2-carboxylic acid (100 mg, 0.43 mmol), triethylamine (0.18 mL, 1.3 mmol) and 4-amino-N, 2-dimethyl-benzamide (70.7 mg, 0.43 mmol) in THF (5 mL) was added 1-propanephosphonic anhydride in EtOAc (548 mg) and it was stirred at 10 °C for 16 h. The solution was poured into water (30 mL), extracted with EtOAc (30 mL) and concentrated to afford the title compound (55.1 mg) as a white solid. MS (ESI, m/z): 379.1 [M+H]⁺.

### Intermediate D15

### N-[4-[[(exo)-3-azabicyclo[3.1.0] hexan-6-yl]carbamoyl]-3-chloro-phenyl]-5-(2,3-difluoro-4-methoxy-phenyl)-1-methyl-imidazole-2-carboxamide .1:1 hydrogen chloride

### Step 1: tert-butyl (exo)-6-(4-(5-bromo-1-methyl-1H-imidazole-2-carboxamido)-2-chlorobenzamido)-3-azabicyclo[3.1.0]hexane-3-carboxylate

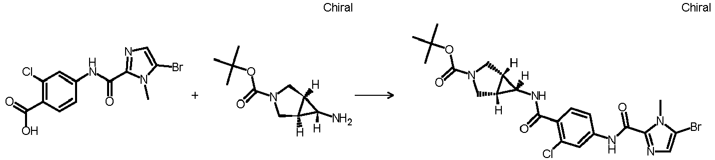

To a light brown suspension of 4-(5-bromo-1-methyl-1H-imidazole-2-carboxamido)-2-chlorobenzoic acid trifluoroacetate [2489205-90-3] (600 mg, 1.27 mmol, Eq: 1) and tert-butyl (exo)-6-amino-3-azabicyclo[3.1.0]hexane-3-carboxylate (378 mg, 1.9 mmol, Eq: 1.5) in DMF (5 ml) at RT was added DIPEA (1.33 ml, 7.62 mmol, Eq: 6), followed by addition of HATU (724 mg, 1.9 mmol, Eq: 1.5). The reaction mixture was stirred at RT overnight. Then water was added and the resulting suspension was filtered, washed with water and the collected solid dried in vacuo to afford the title compound as light brown solid (885 mg, quant yield) which was used without further purification. MS: 538.1 [M+H]+ ESI pos

### Step 2: (exo)-6-[[2-chloro-4-[[5-(2,3-difluoro-4-methoxy-phenyl)-1-methyl-imidazole-2-carbonyl]amino]benzoyl]amino]-3-azabicyclo[3.1.0]hexane-3-carboxylic acid tert-butyl ester

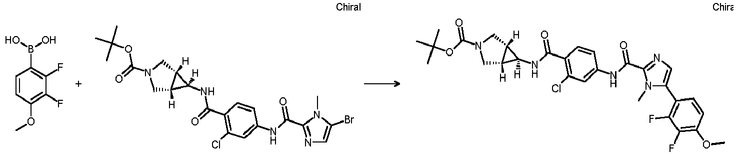

To a solution of (exo)-6-[[4-[(5-bromo-1-methyl-imidazole-2-carbonyl)amino]-2-chlorobenzoyl]amino]-3-azabicyclo[3.1.0]hexane-3-carboxylic acid tert-butyl ester (225 mg, 0.401 mmol, 1 eq) in 1,4-dioxane (2.5 mL) and water (0.250 mL) at RT, were added (2,3-difluoro-4-methoxy-phenyl)boronic acid (90 mg, 0.481 mmol, 1.2 eq), Na₂CO₃ (85 mg, 0.802 mmol, 2 eq) and 1,1'-bis(di-tert-butylphosphino)ferrocene palladium dichloride (26 mg, 0.040 mmol, 0.100 eq). After degazing for 5min with Ar, the mixture was stirred at 90°C for 4 hr. The reaction mixture was diluted with ethyl acetate, filtered over celite and concentrated. The residue was purified by column chromatography on silica gel using EtOAc in Heptane as eluent to give the title compound (151.1 mg, 58.23%) as off-white solid. MS: 602.2 [M+H]+, ESI pos.

### Step 3: N-[4-[[(exo)-3-azabicyclo[3.1.0]hexan-6-yl]carbamoyl]-3-chloro-phenyl]-5-(2,3-difluoro-4-methoxy-phenyl)-1-methyl-imidazole-2-carboxamide .1:1 hydrogen chloride

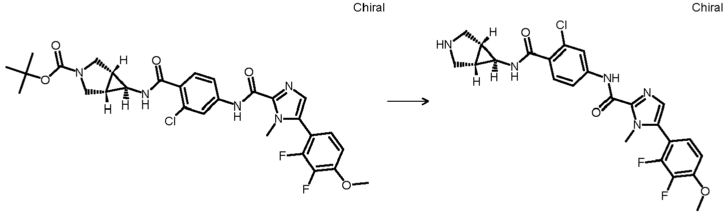

To a light brown solution of (exo)-6-[[2-chloro-4-[[5-(2,3-difluoro-4-methoxy-phenyl)-1-methylimidazole-2-carbonyl]amino]benzoyl]amino]-3-azabicyclo[3.1.0]hexane-3-carboxylic acid tert-butyl ester (151.1 mg, 0.233 mmol, 1 eq) in dichloromethane (0.500 mL) was added 4 M hydrogen chloride in dioxane (583 uL, 2.33 mmol, 10 eq) and the reaction mixture stirred at RT for 3h. The reaction mixture was concentrated to dryness to afford the title compound (149.1 mg, 92.55%) as light brown solid. MS: 502.1 [M+H]+, ESI pos.

### Example B43

### N-[3-chloro-4-[[(exo)-3-[(2S,4R)-4-hydroxyprolyl]-3-azabicyclo[3.1.0]hexan-6-yl]carbamoyl]phenyl]-5-(2,3-difluoro-4-methoxy-phenyl)-1-methyl-imidazole-2-carboxamide .1:1 hydrogen chloride

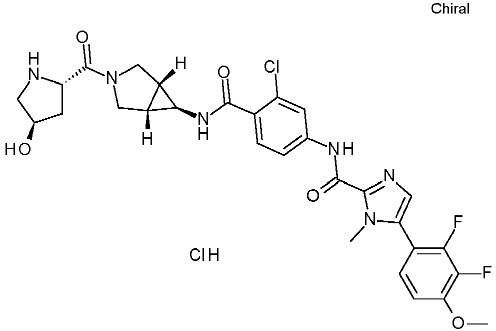

### Step 1: (2S,4R)-2-[(exo)-6-[[2-chloro-4-[[5-(2,3-difluoro-4-methoxy-phenyl)-1-methylimidazole-2-carbonyl] amino] benzoyl] amino] -3-azabicyclo [3.1.0] hexane-3-carbonyl] -4-hydroxy-pyrrolidine-1-carboxylic acid tert-butyl ester

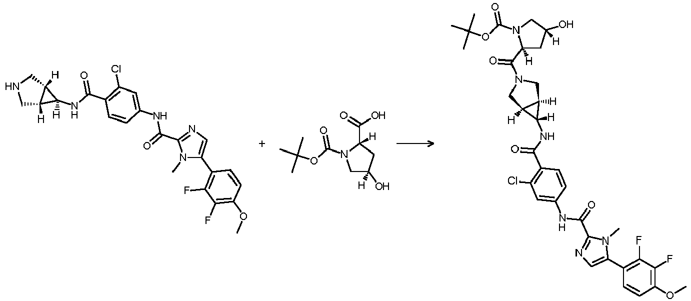

To a light brown solution of (2S,4R)-1-tert-butoxycarbonyl-4-hydroxy-proline (25 mg, 0.109 mmol, 1.5 eq) and N-[4-[[(exo)-3-azabicyclo[3.1.0]hexan-6-yl]carbamoyl]-3-chloro-phenyl]-5-(2,3-difluoro-4-methoxy-phenyl)-1-methyl-imidazole-2-carboxamide .1:1 hydrogen chloride (50 mg, 0.072 mmol, 1 eq) in N,N-dimethylformamide (1 mL) was added N,N-diisopropylethylamine (63.1 uL, 0.362 mmol, 5 eq) and HATU (41 mg, 0.109 mmol, 1.5 eq) were added, and the reaction mixture was stirred at RT for 1.5 hours. Then water was added and the mixture extracted with DCM. The organic layer was then washed twice with a 5% LiCl solution, brine. The organic layer was dried over Na₂SO₄, filtered and concentrated in vacuo. The residue was purified by column chromatography on silica gel using DCM:MeOH as eluent to afford the title compound (28.4 mg, 50.98%) as white solid. MS: 715.2 [M+H]+, ESI pos.

### Step 2: N-[3-chloro-4-[[(exo)-3-[(2S,4R)-4-hydroxyprolyl]-3-azabicyclo[3.1.0]hexan-6-yl]carbamoyl]phenyl]-5-(2,3-difluoro-4-methoxy-phenyl)-1-methyl-imidazole-2-carboxamide .1:1 hydrogen chloride

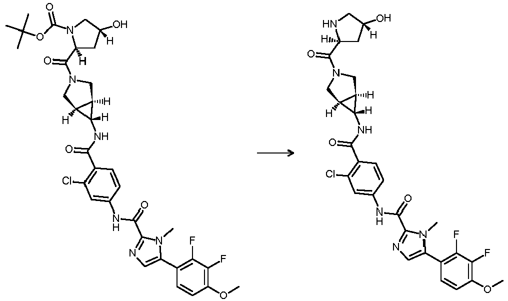

To a light brown solution of (2S,4R)-2-[(exo)-6-[[2-chloro-4-[[5-(2,3-difluoro-4-methoxyphenyl)-1-methyl-imidazole-2-carbonyl] amino]benzoyl] amino]-3-azabicyclo[3.1. 0]hexane-3-carbonyl]-4-hydroxy-pyrrolidine-1-carboxylic acid tert-butyl ester (28.4 mg, 0.037 mmol, 1 eq) in dichloromethane (300 uL) was added 4 M HCl 4M in dioxane (46 uL, 0.185 mmol, 5 eq), and the reaction mixture was stirred at RT for 1.5 hours. The reaction mixture was concentrated to dryness to afford the title compound (31.7 mg, quant yield) as off-white solid. MS: 615.2 [M+H]+, ESI pos.

### Example B44

### (exo)-6-[[2-chloro-4-[[5-(2,3-difluoro-4-methoxy-phenyl)-1-methyl-imidazole-2-carbonyl] amino] benzoyl] amino]-N- [(trans)-4-hydroxypyrrolidin-3-yl]-3-azabicyclo[3.1.0]hexane-3-carboxamide .1:1 hydrogen chloride

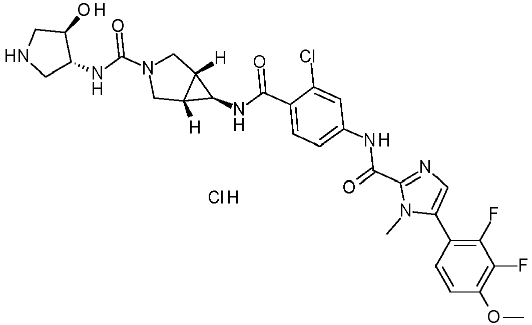

### Step 1: (trans)-3-[[(exo)-6-[[2-chloro-4-[[5-(2,3-difluoro-4-methoxy-phenyl)-1-methylimidazole-2-carbonyl] amino] benzoyl] amino] -3-azabicyclo [3.1.0] hexane-3-carbonyl] amino] - 4-hydroxy-pyrrolidine-1-carboxylic acid tert-butyl ester

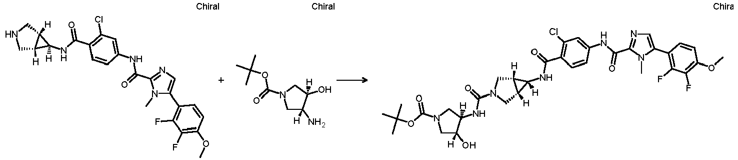

To a colorless solution of (trans)-3-amino-4-hydroxy-pyrrolidine-1-carboxylic acid tert-butyl ester (43.95 mg, 0.217 mmol, 3 eq) in N,N-dimethylformamide were added triethylamine (50.5 uL, 0.362 mmol, 5 eq) and 1,1'-carbonyldiimidazole (29.37 mg, 0.181 mmol, 2.5 eq), and the reaction mixture was stirred at RT for 20min. Then N-[4-[[(exo)-3-azabicyclo[3.1.0]hexan-6-yl]carbamoyl]-3-chloro-phenyl]-5-(2,3-difluoro-4-methoxy-phenyl)-1-methyl-imidazole-2-carboxamide . 1: 1 hydrogen chloride (50 mg, 0.072 mmol, 1 eq) was added and the stirring was continued at RT for 1.5 hours. Then water was added and the mixture extracted with DCM. The organic layer was then washed twice with a 5% LiCl solution, brine. The organic layer was dried over Na₂SO₄, filtered and concentrated in vacuo. The residue was purified by column chromatography on silica gel using DCM:MeOH as eluent to afford the title compound (51.1 mg, 87.91%) as white solid. MS: 730.3 [M+H]+, ESI pos.

### Step 2: (exo)-6-[[2-chloro-4-[[5-(2,3-difluoro-4-methoxy-phenyl)-1-methyl-imidazole-2-carbonyl] amino] benzoyl] amino]-N- [(trans)-4-hydroxypyrrolidin-3-yl]-3-azabicyclo[3.1.0]hexane-3-carboxamide .1:1 hydrogen chloride

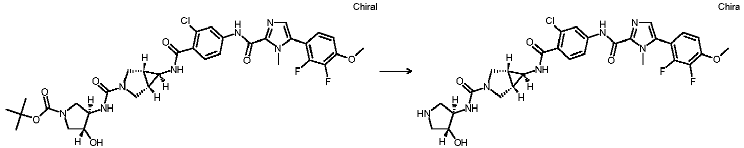

A light brown solution of (trans)-3-[[(exo)-6-[[2-chloro-4-[[5-(2,3-difluoro-4-methoxy-phenyl)-1-methyl-imidazole-2-carbonyl]amino]benzoyl]amino]-3-azabicyclo[3.1.0]hexane-3-carbonyl]amino]-4-hydroxy-pyrrolidine-1-carboxylic acid tert-butyl ester (51.1 mg, 0.064 mmol, 1 eq) in dichloromethane (500 uL) was added 4 M HCl in dioxane (79.6 uL, 0.318 mmol, 5 eq) and the reaction mixture was stirred at RT for 1.5 hours. The reaction mixture was concentrated to dryness to afford the title compound (30.6 mg, 65.6%) as off-white solid. MS: 630.2 [M+H]+, ESI pos

### Assay procedures

### Antimicrobial susceptibility testing:

### 90% Growth Inhibitory Concentration (IC90) determination

The in vitro antimicrobial activity of the compounds was determined according to the following procedure:
The assay used a 10-points Iso-Sensitest broth medium to measure quantitatively the *in vitro* activity of the compounds against *Acinetobacter baumannii* ATCC17978 or ATCC17961.

Stock compounds in DMSO were serially twofold diluted (e.g. range from 50 to 0.097 µM final concentration) in 384 wells microtiter plates and inoculated with 49 µl the bacterial suspension in Iso-Sensitest medium to have a final cell concentration of ~ 5×10⁽⁵⁾ CFU/ml in a final volume/well of 50 ul/well. Microtiter plates were incubated at 35 ± 2 °C.

Bacterial cell growth was determined with the measurement of optical density at λ=600nm each 20 minutes over a time course of 16h. Growth inhibition was calculated during the logarithmic growth of the bacterial cells with determination of the concentration inhibiting 50% (IC50) and 90% (IC90) of the growth.

Table 1 provides the 90% growth inhibitory concentrations (IC90) in micromoles per liter of the compounds of present invention obtained against the strain *Acinetobacter baumannii* ATCC17978.

Table 2 provides the 90% growth inhibitory concentrations (IC90) in micromoles per liter of the compounds of present invention obtained against the strain *Acinetobacter baumannii* ATCC17961.

Particular compounds of the present invention exhibit an IC90 (*Acinetobacter baumannii* ATCC17978 and/or ATCC17961) ≤ 25 µmol/l.

More particular compounds of the present invention exhibit an IC90 (*Acinetobacter baumannii* ATCC17978 and/or ATCC17961) ≤ 5 µmol/l.

Most particular compounds of the present invention exhibit an IC90 (*Acinetobacter baumannii* ATCC17978 and/or ATCC17961) ≤ 1 µmol/l.

**Table 1**

| **Example** | **ATCC 17978 IC90 [uM]** |
|---|---|
| A1 | 0.24 |
| A2 | 0.61 |
| A3 | 0.31 |
| A4 | 0.44 |
| A5 | 0.55 |
| A6 | 0.15 |
| A7 | 0.24 |
| A8 | 0.5 |
| A9 | 0.25 |
| A10 | 0.36 |
| A11 | 0.31 |
| A12 | 0.34 |
| A13 | 0.59 |
| A14 | 3 |
| A15 | 4.5 |
| A16 | 1.5 |
| A17 | 0.4 |
| A18 | 1.2 |
| A19 | 1.4 |
| A20 | 0.96 |
| A21 | 4.7 |
| A22 | 0.18 |
| A23 | 0.22 |
| A24 | 0.67 |
| A25 | 0.13 |
| A26 | 0.2 |
| A27 | 0.24 |
| A28 | 1.2 |
| A29 | / |
| A30 | 0.25 |
| B1 | 0.11 |
| B2 | 0.13 |
| B3 | 0.28 |
| B4 | 0.2 |
| B5 | 0.079 |
| B6 | 0.1 |
| B7 | 0.082 |
| B8 | 0.14 |
| B9 | 0.21 |
| B10 | 0.14 |
| B11 | 0.23 |
| B12 | 0.76 |
| B13 | 0.91 |
| B14 | 0.99 |
| B15 | 0.36 |
| B16 | 0.35 |
| B17 | 0.084 |
| B18 | 0.24 |
| B19 | 0.16 |
| B20 | 0.21 |
| B21 | 0.098 |
| B22 | 0.32 |
| B23 | 0.3 |
| B24 | 1 |
| B25 | 0.87 |
| B26 | 1.2 |
| B27 | 1.1 |
| B28 | 0.51 |
| B29 | 0.61 |
| B30 | 0.65 |
| B31 | 0.74 |
| B32 | 1.2 |
| B33 | 1.1 |
| B34 | 1.7 |
| B35 | 1.3 |
| B36 | 1.1 |
| B37 | 0.85 |
| B38 | 0.055 |
| B39 | 0.057 |
| B40 | 0.12 |
| B41 | 0.13 |
| B42 | 0.073 |
| C1 | 0.83 |
| C2 | 0.37 |
| C3 | 0.45 |
| C4 | 0.75 |
| D1 | 3.2 |

**Table 2**

| **Example** | **ATCC 17961 IC90 [uM]** |
|---|---|
| B43 | 0.139 |
| B44 | 0.155 |

### Example 1

A compound of formula (I) can be used in a manner known per se as the active ingredient for the production of tablets of the following composition:

| | |
|---|---|
| | Per tablet |
| | |
| Active ingredient | 200 mg |
| Microcrystalline cellulose | 155 mg |
| Corn starch | 25 mg |
| Talc | 25 mg |
| Hydroxypropylmethylcellulose | 20 mg |
| | 425 mg |

### Example 2

A compound of formula (I) can be used in a manner known per se as the active ingredient for the production of capsules of the following composition:

| | |
|---|---|
| | Per capsule |
| | |
| Active ingredient | 100.0 mg |
| Corn starch | 20.0 mg |
| Lactose | 95.0 mg |
| Talc | 4.5 mg |
| Magnesium stearate | 0.5 mg |
| | 220.0 mg |

### Example 3

A compound of formula (I) can be used in a manner known per se as the active ingredient for the production of an infusion solution of the following composition:

| | |
|---|---|
| Active ingredient | 100 mg |
| Lactic acid 90% | 100 mg |
| NaOH q.s. or HCl q.s. for adjustment to pH | 4.0 |
| Sodium chloride q.s. or glucose q.s. for adjustment of the osmolality to 290 mOsm/kg | |
| Water for injection (WFI) | ad 100 ml |

### Example 4

A compound of formula (I) can be used in a manner known per se as the active ingredient for the production of an infusion solution of the following composition:

| | |
|---|---|
| Active ingredient | 100 mg |
| Hydroxypropyl-beta-cyclodextrin | 10 g |
| NaOH q.s. or HCl q.s. for adjustment to pH 7.4 | |
| Sodium chloride q.s. or glucose q.s. for adjustment of the osmolality to 290 mOsm/kg | |
| Water for injection (WFI) | ad 100 ml |

## Claims

1. A compound of formula (I) 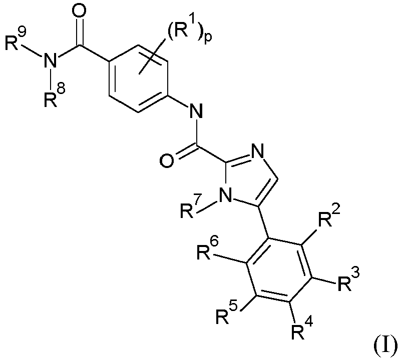 or a pharmaceutically acceptable salt thereof, wherein:
R¹ is, at each occurrence, independently selected from halogen, cyano, C₁-C₆-alkyl, C₁-C₆-alkoxy, halo-C₁-C₆-alkyl, and halo-C₁-C₆-alkoxy;
R², R³, R⁴, R⁵ and R⁶ are each independently selected from hydrogen, halogen, C₁-C₆-alkyl, C₁-C₆-alkoxy, cyano, halo-C₁-C₆-alkyl, cyano-C₁-C₆-alkyl, (C₁-C₆-alkyl)₂N-, halo-C₁-C₆-alkoxy, cyano-C₁-C₆-alkoxy, C₁-C₆-alkoxy-C₁-C₆-alkoxy-, (C₁-C₆-alkyl)zN-C(O)-, and carbamoyl-C₁-C₆-alkoxy;
R⁷ is selected from hydrogen, C₁-C₆-alkyl, and halo-C₁-C₆-alkyl;
R⁸ is selected from C₁-C₆-alkyl, amino-C₁-C₆-alkyl, amino-C₁-C₆-alkyl-C(O)-, (C₁-C₆-alkyl)₂N-C₁-C₆-alkyl-, C₁-C₆-alkyl-NH-C₁-C₆-alkyl-, amino-C₁-C₆-alkoxy-C₁-C₆-alkyl-, C₁-C₆-alkyl-NH-C₁-C₆-alkoxy-C₁-C₆-alkyl-, (C₁-C₆-alkyl)₂N-C₁-C₆-alkoxy-C₁-C₆-alkyl-, amino-C₁-C₆-alkoxy-C₁-C₆-alkyl-C(O)-, C₁-C₆-alkyl-NH-C₁-C₆-alkoxy-C₁-C₆-alkyl-C(O)-, (C₁-C₆-alkyl)₂N-C₁-C₆-alkoxy-C₁-C₆-alkyl-C(O)-, amino-C₁-C₆-alkyl-C(O)-NH-C₁-C₆-alkyl-, C₁-C₆-alkyl-NH-C₁-C₆-alkyl-C(O)-NH-C₁-C₆-alkyl-, (C₁-C₆-alkyl)₂N-C₁-C₆-alkyl-C(O)-NH-C₁-C₆-alkyl-, and a group 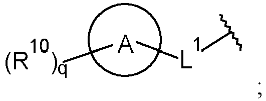
R⁹ is selected from hydrogen and C₁-C₆-alkyl;
R¹⁰ is selected from halogen, cyano, amino, hydroxy, C₁-C₆-alkyl, C₁-C₆-alkoxy, halo-C₁-C₆-alkyl, halo-C₁-C₆-alkoxy, amino-C₁-C₆-alkyl-, C₁-C₆-alkyl-NH-C₁-C₆-alkyl-, (C₁-C₆-alkyl)₂N-C₁-C₆-alkyl-, amino-C₁-C₆-alkyl-C(O)-, C₁-C₆-alkyl-NH-C₁-C₆-alkyl-C(O)-, (C₁-C₆-alkyl)₂N-C₁-C₆-alkyl-C(O)-, amino-C₁-C₆-alkyl-NH-C(O)-, amino-C₁-C₆-alkoxy-C₁-C₆-alkyl-C(O)-, hydroxy-C₁-C₆-alkyl, and a group 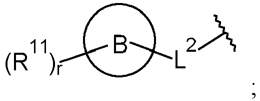
R¹¹ is selected from halogen, cyano, amino, hydroxy, C₁-C₆-alkyl, C₁-C₆-alkoxy, halo-C₁-C₆-alkyl, halo-C₁-C₆-alkoxy, amino-C₁-C₆-alkyl-, C₁-C₆-alkyl-NH-C₁-C₆-alkyl-, (C₁-C₆-alkyl)₂N-C₁-C₆-alkyl-, amino-C₁-C₆-alkyl-C(O)-, C₁-C₆-alkyl-NH-C₁-C₆-alkyl-C(O)-, (C₁-C₆-alkyl)₂N-C₁-C₆-alkyl-C(O)-, amino-C₁-C₆-alkyl-NH-C(O)-, amino-C₁-C₆-alkoxy-C₁-C₆-alkyl-C(O)-, and hydroxy-C₁-C₆-alkyl;
A and B are each independently selected from 3- to 14-membered heterocyclyl, C₃-C₁₀-cycloalkyl, 5- to 14-membered heteroaryl, and C₆-C₁₀-aryl;
L¹ is selected from a covalent bond, carbonyl, -NH-C(O)-, C₁-C₆-alkyl, -C(O)-NH-C₁-C₆-alkyl-, -C₁-C₆-alkyl-NH-C(O)-, and -NH-C(O)-NH-C₁-C₆-alkyl-;
L² is selected from a covalent bond, carbonyl, -NH-C(O)-, -C(O)-NH-, C₁-C₆-alkyl, -C(O)-NH-C₁-C₆-alkyl-, -C₁-C₆-alkyl-NH-C(O)-, and -NH-C(O)-NH-C₁-C₆-alkyl-;
p is selected from 0, 1, 2, 3, or 4; and
q and r are independently selected from 0, 1, 2, and 3.

2. The compound of formula (I) according to claim 1, or a pharmaceutically acceptable salt thereof, wherein said compound of formula (I) is a compound of formula (I-I) 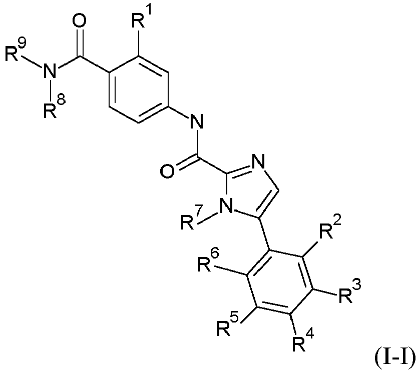 wherein R¹ to R⁹ are as defined in claim 1.

3. The compound of formula (I) according to claim 1, or a pharmaceutically acceptable salt thereof, wherein said compound of formula (I) is a compound of formula (I-II) 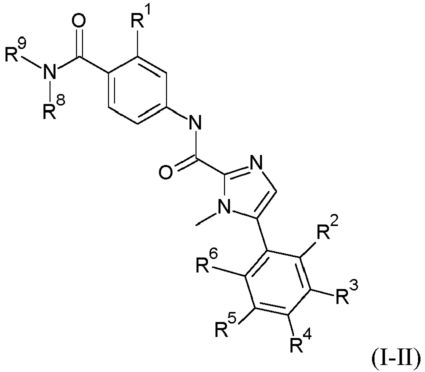 wherein R¹ to R⁶, R⁸ and R⁹ are as defined in claim 1.

4. The compound of formula (I) according to any one of claims 1 to 3, or a pharmaceutically acceptable salt thereof, wherein:
R¹ is C₁-C₆-alkyl or halogen;
R⁸ is selected from C₁-C₆-alkyl, amino-C₁-C₆-alkyl, (C₁-C₆-alkyl)₂N-C₁-C₆-alkyl-, amino-C₁-C₆-alkoxy-C₁-C₆-alkyl-, (C₁-C₆-alkyl)₂N-C₁-C₆-alkoxy-C₁-C₆-alkyl-, amino-C₁-C₆-alkyl-C(O)-NH-C₁-C₆-alkyl-, (C₁-C₆-alkyl)₂N-C₁-C₆-alkyl-C(O)-NH-C₁-C₆-alkyl-, and a group 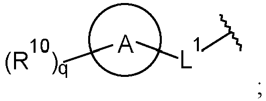
R⁹ is hydrogen;
R¹⁰ is selected from amino, hydroxy, C₁-C₆-alkyl, amino-C₁-C₆-alkyl-C(O)-, (C₁-C₆-alkyl)₂N-C₁-C₆-alkyl-C(O)-, amino-C₁-C₆-alkyl-NH-C(O)-, amino-C₁-C₆-alkoxy-C₁-C₆-alkyl-C(O)-, hydroxy-C₁-C₆-alkyl, and a group 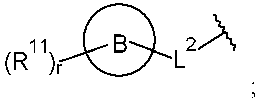
R¹¹ is selected from hydroxy, amino, and amino-C₁-C₆-alkyl;
A and B are each independently selected from 3- to 14-membered heterocyclyl and C₃-C₁₀-cycloalkyl;
L¹ is selected from a covalent bond, C₁-C₆-alkyl, -C(O)-NH-C₁-C₆-alkyl-, and -NH-C(O)-NH-C₁-C₆-alkyl-;
L² is selected from carbonyl, C₁-C₆-alkyl, -C(O)-NH-, and -NH-C(O)-;
p is 1; and
q and r are each independently selected from 0 and 1.

5. The compound of formula (I) according to claim 4, or a pharmaceutically acceptable salt thereof, wherein:
R¹ is halogen;
R⁸ is a group 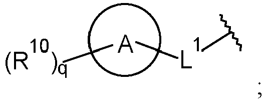
R⁹ is hydrogen;
R¹⁰ is selected from amino-C₁-C₆-alkyl-C(O)-, (C₁-C₆-alkyl)₂N-C₁-C₆-alkyl-C(O)-, amino-C₁-C₆-alkoxy-C₁-C₆-alkyl-C(O)-, and a group 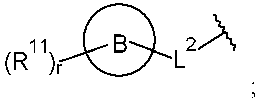
R¹¹ is selected from hydroxy and amino;
A and B are each independently selected from 3- to 14-membered heterocyclyl and C₃-C₁₀-cycloalkyl;
L¹ is selected from a covalent bond and C₁-C₆-alkyl;
L² is selected from carbonyl, -C(O)-NH-, and -NH-C(O)-;
p is 1;
q is 1; and
r is selected from 0 and 1.

6. The compound of formula (I) according to claim 5, or a pharmaceutically acceptable salt thereof, wherein:
R¹ is chloro;
R⁸ is a group 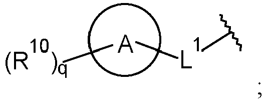
R⁹ is hydrogen;
R¹⁰ is selected from 2-aminoacetyl, 2-aminopropanoyl, 2-(dimethylamino)acetyl, 3-(2-aminoethoxy)propanoyl, and a group 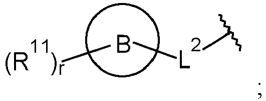
R¹¹ is selected from hydroxy and amino;
A is selected from azetidinyl, cyclobutyl, pyrrolidinyl, and piperidyl;
B is selected from cyclobutyl, pyrrolidinyl, and piperidyl;
L¹ is selected from a covalent bond and -(CH₂)₂-;
L² is selected from carbonyl, -C(O)-NH-, and -NH-C(O)-;
p is 1;
q is 1; and
r is selected from 0 and 1.

7. The compound of formula (I) according to any one of claims 1 to 6, or a pharmaceutically acceptable salt thereof, wherein:
R² is hydrogen or halogen;
R³ is hydrogen or halogen;
R⁴ is selected from C₁-C₆-alkoxy, halo-C₁-C₆-alkoxy, cyano-C₁-C₆-alkoxy, and carbamoyl-C₁-C₆-alkoxy;
R⁵ is hydrogen; and
R⁶ is hydrogen or halogen.

8. The compound of formula (I) according to claim 7, or a pharmaceutically acceptable salt thereof, wherein:
R² and R³ are both halogen;
R⁴ is selected from C₁-C₆-alkoxy and halo-C₁-C₆-alkoxy; and
R⁵ and R⁶ are both hydrogen.

9. The compound of formula (I) according to claim 8, or a pharmaceutically acceptable salt thereof, wherein:
R² and R³ are both fluoro;
R⁴ is selected from methoxy and difluoromethoxy; and
R⁵ and R⁶ are both hydrogen.

10. The compound of formula (I) according to any one of claims 1 to 9, or a pharmaceutically acceptable salt thereof, wherein R⁷ is C₁-C₆-alkyl.

11. The compound of formula (I) according to claim 10, or a pharmaceutically acceptable salt thereof, wherein R⁷ is methyl.

12. The compound of formula (I) according to claim 1, or a pharmaceutically acceptable salt thereof, wherein said compound of formula (I) is selected from:
N-[4-[2-(2-Aminoethoxy)ethylcarbamoyl]-3-chloro-phenyl]-5-(2,3-difluoro-4-methoxy-phenyl)-1-methyl-imidazole-2-carboxamide;
N-[3-chloro-4-(2-piperazin-1-ylethylcarbamoyl)phenyl]-5-(2,3-difluoro-4-methoxyphenyl)-1-methyl-imidazole-2-carboxamide;
N- [3 -chloro-4-(pyrrolidin-3 -ylcarbamoyl)phenyl] - 5 -(2,3 -difluoro-4-methoxyphenyl)-1-methyl-imidazole-2-carboxamide;
N-[3-chloro-4-(4-piperidylcarbamoyl)phenyl]-5-(2,3-difluoro-4-methoxy-phenyl)-1-methyl-imidazole-2-carboxamide;
N-[3-chloro-4-(4-piperidylmethylcarbamoyl)phenyl]-5-(2,3-difluoro-4-methoxyphenyl)-1-methyl-imidazole-2-carboxamide;
N-[4-(6-aminohexylcarbamoyl)-3-chloro-phenyl]-5-(2,3-difluoro-4-methoxyphenyl)-1-methyl-imidazole-2-carboxamide;
N-[4-[[3-(aminomethyl)cyclobutyl]methylcarbamoyl]-3-chloro-phenyl]-5-(2,3-difluoro-4-methoxy-phenyl)-1-methyl-imidazole-2-carboxamide;
N-[4-[(3-aminocyclobutyl)methylcarbamoyl]-3-chloro-phenyl]-5-(2,3-difluoro-4-methoxy-phenyl)-1-methyl-imidazole-2-carboxamide;
N-[4-[2-(2-aminoethoxy)ethylcarbamoyl]-3-chloro-phenyl]-5-[4-(cyanomethoxy)-2,3-difluoro-phenyl]-1-methyl-imidazole-2-carboxamide;
N-[3-chloro-4-(2-piperazin-1-ylethylcarbamoyl)phenyl]-5-[4-(cyanomethoxy)-2,3-difluoro-phenyl]-1-methyl-imidazole-2-carboxamide;
N-[3-chloro-4-(3-piperazin-1-ylpropylcarbamoyl)phenyl]-5-[4-(cyanomethoxy)-2,3-difluoro-phenyl]-1-methyl-imidazole-2-carboxamide;
5-[4-(cyanomethoxy)-2,3-difluoro-phenyl]-1-methyl-N-[3-methyl-4-(3-piperazin-1-ylpropylcarbamoyl)phenyl]imidazole-2-carboxamide;
N-[4-[2-(2-aminoethoxy)ethylcarbamoyl]-3-ethyl-phenyl]-5-(2,3-difluoro-4-methoxy-phenyl)-1-methyl-imidazole-2-carboxamide;
N-[4-[2-(2-aminoethoxy)ethylcarbamoyl]-3-ethyl-phenyl]-5-[4-(difluoromethoxy)phenyl]-1-methyl-imidazole-2-carboxamide;
N-[4-[2-(2-aminoethoxy)ethylcarbamoyl]-3-ethyl-phenyl]-5-(3-fluoro-4-isopropoxyphenyl)-1-methyl-imidazole-2-carboxamide;
N-[4-[2-(2-aminoethoxy)ethylcarbamoyl]-3-ethyl-phenyl]-5-(2-chloro-4-methoxyphenyl)-1-methyl-imidazole-2-carboxamide;
N-[4-[2-(2-aminoethoxy)ethylcarbamoyl]-3-ethyl-phenyl]-5-[4-(difluoromethoxy)-2,3-difluoro-phenyl]-1-methyl-imidazole-2-carboxamide;
N-[4-[2-(2-aminoethoxy)ethylcarbamoyl]-3-ethyl-phenyl]-5-(2,6-difluoro-4-methoxy-phenyl)-1-methyl-imidazole-2-carboxamide;
N-[4-(3-Aminopropylcarbamoyl)-3-chloro-phenyl]-5-[4-(difluoromethoxy)-2,3-difluoro-phenyl]-1-methyl-imidazole-2-carboxamide;
N-[4-[2-(2-aminoethoxy)ethylcarbamoyl]-3-ethyl-phenyl]-5-[4-(cyanomethoxy)-2,3-difluoro-phenyl]-1-methyl-imidazole-2-carboxamide;
N-[4-[2-(2-aminoethoxy)ethylcarbamoyl]-3-ethyl-phenyl]-5-[4-(2-amino-2-oxo-ethoxy)-2,3-difluoro-phenyl]-1-methyl-imidazole-2-carboxamide;
N- [4- [2-(2-aminoethoxy)ethylcarbamoyl] -3 -ethyl-phenyl] -5 - [2-chloro-4-(cyanomethoxy)-3-fluoro-phenyl]-1-methyl-imidazole-2-carboxamide;
N-[3-Chloro-4-[[1-(piperidine-4-carbonyl)pyrrolidin-3-yl]carbamoyl]phenyl]-5-(2,3-difluoro-4-methoxy-phenyl)-1-methyl-imidazole-2-carboxamide;
N-[3-Chloro-4-[[1-[2-(dimethylamino)acetyl]pyrrolidin-3-yl]carbamoyl]phenyl]-5-(2,3-difluoro-4-methoxy-phenyl)-1-methyl-imidazole-2-carboxamide;
N-[3-chloro-4-[2-[[2-(dimethylamino)acetyl]amino]ethylcarbamoyl]phenyl] -5 -(2,3 - difluoro-4-methoxy-phenyl)-1-methyl-imidazole-2-carboxamide;
N-[3-chloro-4-[[1-[2-(dimethylamino)acetyl]-4-piperidyl]carbamoyl]phenyl]-5-(2,3-difluoro-4-methoxy-phenyl)-1-methyl-imidazole-2-carboxamide;
N-[3-chloro-4-[5-(dimethylamino)pentylcarbamoyl]phenyl]-5-(2,3-difluoro-4-methoxy-phenyl)-1-methyl-imidazole-2-carboxamide;
N-[3-chloro-4-(2-isopentyloxyethylcarbamoyl)phenyl]-5-[4-(cyanomethoxy)-2,3-difluoro-phenyl]-1-methyl-imidazole-2-carboxamide;
N-[3-chloro-4-[2-(3-hydroxypyrrolidin-1-yl)ethylcarbamoyl]phenyl]-5-(2,3-difluoro-4-methoxy-phenyl)-1-methyl-imidazole-2-carboxamide;
N-[3-chloro-4-[2-[3-(hydroxymethyl)pyrrolidin-1-yl]ethylcarbamoyl]phenyl]-5-(2,3-difluoro-4-methoxy-phenyl)-1-methyl-imidazole-2-carboxamide;
5-(2,3-difluoro-4-methoxy-phenyl)-N-[4-[3-[2-(dimethylamino)ethoxy]propylcarbamoyl]-3-ethyl-phenyl]-1-methyl-imidazole-2-carboxamide;
N-[3-chloro-4-[[1-[(3R)-pyrrolidine-3-carbonyl]pyrrolidin-3-yl]carbamoyl]phenyl]-5-(2,3-difluoro-4-methoxy-phenyl)-1-methyl-imidazole-2-carboxamide;
N-[3-chloro-4-[[1-[(2S,4R)-4-hydroxypyrrolidine-2-carbonyl]pyrrolidin-3-yl]carbamoyl]phenyl]-5-(2,3-difluoro-4-methoxy-phenyl)-1-methyl-imidazole-2-carboxamide;
N-[4-[[1-[3-(2-aminoethoxy)propanoyl]pyrrolidin-3-yl]carbamoyl]-3-chloro-phenyl]-5-(2,3-difluoro-4-methoxy-phenyl)-1-methyl-imidazole-2-carboxamide;
N-[3-chloro-4-[[1-(piperidine-4-carbonyl)-4-piperidyl]carbamoyl]phenyl]-5-(2,3-difluoro-4-methoxy-phenyl)-1-methyl-imidazole-2-carboxamide;
N-[3-chloro-4-[[1-[(3R)-pyrrolidine-3-carbonyl]-4-piperidyl]carbamoyl]phenyl]-5-(2,3-difluoro-4-methoxy-phenyl)-1-methyl-imidazole-2-carboxamide;
N-[3-chloro-4-[[1-[(2S,4R)-4-hydroxypyrrolidine-2-carbonyl]-4-piperidyl]carbamoyl]phenyl]-5-(2,3-difluoro-4-methoxy-phenyl)-1-methylimidazole-2-carboxamide;
N-[4-[[1-[3-(2-aminoethoxy)propanoyl]-4-piperidyl]carbamoyl]-3-chloro-phenyl]-5-(2,3-difluoro-4-methoxy-phenyl)-1-methyl-imidazole-2-carboxamide;
N-[4-[[3-[[3-(aminomethyl)cyclobutanecarbonyl]amino]cyclobutyl]carbamoyl]-3-chloro-phenyl]-5-(2,3-difluoro-4-methoxy-phenyl)-1-methyl-imidazole-2-carboxamide;
N-[4-[2-[1-(3-aminopropanoyl)azetidin-3-yl]ethylcarbamoyl]-3-chloro-phenyl]-5-[4-(difluoromethoxy)-2,3-difluoro-phenyl]-1-methyl-imidazole-2-carboxamide;
N-[4-[3-(3-aminopropanoylamino)propylcarbamoyl]-3-chloro-phenyl]-5-[4-(difluoromethoxy)-2,3-difluoro-phenyl]-1-methyl-imidazole-2-carboxamide;
N-[3-[[4-[[5-[4-(difluoromethoxy)-2,3-difluoro-phenyl]-1-methyl-imidazole-2-carbonyl]amino]-2-ethyl-benzoyl]amino]propyl]piperidine-4-carboxamide;
N-[2-[[4-[[5-[4-(cyanomethoxy)-2,3-difluoro-phenyl]-1-methyl-imidazole-2-carbonyl]amino]-2-methyl-benzoyl]amino]ethyl]piperidine-4-carboxamide;
N-[2-[[4-[[5-[4-(cyanomethoxy)-2,3-difluoro-phenyl]-1-methyl-imidazole-2-carbonyl] amino]-2-methyl-benzoyl] amino] ethyl]-4-hydroxy-piperidine-4-carboxamide;
N-[2-[[2-chloro-4-[[5-[4-(cyanomethoxy)-2,3-difluoro-phenyl]-1-methyl-imidazole-2-carbonyl]amino]benzoyl]amino]ethyl]piperidine-4-carboxamide;
N-[2-[[2-chloro-4-[[5-[4-(cyanomethoxy)-2,3-difluoro-phenyl]-1-methyl-imidazole-2-carbonyl] amino] benzoyl] amino] ethyl] -4-hydroxy-piperidine-4-carboxamide;
N-[4-[[1-(2-aminoacetyl)azetidin-3-yl]carbamoyl]-3-chloro-phenyl]-5-(2,3-difluoro-4-methoxy-phenyl)-1-methyl-imidazole-2-carboxamide;
N-[4-[[1-(2-aminoacetyl)azetidin-3-yl]methylcarbamoyl]-3-chloro-phenyl-5-(2,3-difluoro-4-methoxy-phenyl)-1-methyl-imidazole-2-carboxamide;
N-[4-[[3-(2-aminoethylcarbamoyl)cyclobutyl]carbamoyl]-3-chloro-phenyl]-5-(2,3-difluoro-4-methoxy-phenyl)-1-methyl-imidazole-2-carboxamide;
N-[4-[[3-(3-aminoazetidine-1-carbonyl)cyclobutyl]carbamoyl]-3-chloro-phenyl]-5-(2,3-difluoro-4-methoxy-phenyl)-1-methyl-imidazole-2-carboxamide;
N-[4-[[3-[(3-aminocyclobutyl)carbamoyl]cyclobutyl]carbamoyl]-3-chloro-phenyl]-5-(2,3-difluoro-4-methoxy-phenyl)-1-methyl-imidazole-2-carboxamide;
N-[4-[[3-(2-aminoethylcarbamoyl)cyclobutyl]carbamoyl]-3-ethyl-phenyl]-5-(2,3-difluoro-4-methoxy-phenyl)-1-methyl-imidazole-2-carboxamide;
N-[4-[[1-(2-aminoacetyl)azetidin-3-yl]carbamoyl]-3-ethyl-phenyl]-5-(2,3-difluoro-4-methoxy-phenyl)-1-methyl-imidazole-2-carboxamide;
5-[4-(cyanomethoxy)-2,3-difluoro-phenyl]-N-[4-[2-[[(2R,4R)-4-hydroxy-4-methyl-pyrrolidine-2-carbonyl]amino]ethylcarbamoyl]-3-methyl-phenyl]-1-methylimidazole-2-carboxamide;
5-[4-(cyanomethoxy)-2,3-difluoro-phenyl]-N-[4-[2-[[(2R,4R)-4-ethyl-4-hydroxy-pyrrolidine-2-carbonyl]amino]ethylcarbamoyl]-3-methyl-phenyl]-1-methylimidazole-2-carboxamide;
5-(2,3-difluoro-4-methoxy-phenyl)-N-[4-[2-[[(2R,4R)-4-hydroxy-4-methyl-pyrrolidine-2-carbonyl]amino]ethylcarbamoyl]-3-methyl-phenyl]-1-methylimidazole-2-carboxamide;
5-(2,3-difluoro-4-methoxy-phenyl)-N-[4-[2-[[(2R,4R)-4-ethyl-4-hydroxy-pyrrolidine-2-carbonyl]amino]ethylcarbamoyl]-3-methyl-phenyl]-1-methylimidazole-2-carboxamide;
5-(2,3-difluoro-4-methoxy-phenyl)-N-[4-[2-[[(3R,4R)-4-hydroxypyrrolidin-3-yl]carbamoylamino]ethylcarbamoyl]-3-methyl-phenyl]-1-methyl-imidazole-2-carboxamide;
5-(2,3-difluoro-4-methoxy-phenyl)-N-[4-[2-[[(3S,4S)-4-hydroxypyrrolidin-3-yl]carbamoylamino]ethylcarbamoyl]-3-methyl-phenyl]-1-methyl-imidazole-2-carboxamide;
5-[4-(cyanomethoxy)-2,3-difluoro-phenyl]-N-[4-[2-[[(3R,4R)-4-hydroxypyrrolidin-3-yl]carbamoylamino]ethylcarbamoyl]-3-methyl-phenyl]-1-methyl-imidazole-2-carboxamide;
5-[4-(cyanomethoxy)-2,3-difluoro-phenyl]-N-[4-[2-[[(3S,4S)-4-hydroxypyrrolidin-3-yl]carbamoylamino]ethylcarbamoyl]-3-methyl-phenyl]-1-methyl-imidazole-2-carboxamide;
(3R,4R)-N-[2-[[2-chloro-4-[[5-[4-(cyanomethoxy)-2,3-difluoro-phenyl]-1-methylimidazole-2-carbonyl]amino]benzoyl]amino]ethyl]-3-hydroxy-piperidine-4-carboxamide;
(3S,4S)-N-[2-[[2-chloro-4-[[5-[4-(cyanomethoxy)-2,3-difluoro-phenyl]-1-methylimidazole-2-carbonyl]amino]benzoyl]amino]ethyl]-3-hydroxy-piperidine-4-carboxamide;
(3S,4R)-N-[2-[[2-chloro-4-[[5-[4-(cyanomethoxy)-2,3-difluoro-phenyl]-1-methylimidazole-2-carbonyl]amino]benzoyl]amino]ethyl]-3-hydroxy-piperidine-4-carboxamide;
(3R,4S)-N-[2-[[2-chloro-4-[[5-[4-(cyanomethoxy)-2,3-difluoro-phenyl]-1-methylimidazole-2-carbonyl]amino]benzoyl]amino]ethyl]-3-hydroxy-piperidine-4-carboxamide;
N-[3-chloro-4-[2-[[(2R,4R)-4-hydroxy-4-methyl-pyrrolidine-2-carbonyl] amino] ethylcarbamoyl]phenyl] -5 - [4-(cyanomethoxy)-2,3 -difluoro-phenyl]-1-methyl-imidazole-2-carboxamide;
N-[3-chloro-4-[2-[[(2R,4R)-4-ethyl-4-hydroxy-pyrrolidine-2-carbonyl] amino] ethylcarbamoyl]phenyl] -5 - [4-(cyanomethoxy)-2,3 -difluoro-phenyl]-1-methyl-imidazole-2-carboxamide;
N-[3-chloro-4-[[3-[[(4R)-4-hydroxypyrrolidine-2-carbonyl] amino] cyclobutyl] carbamoyl]phenyl] -5 -(2,3 -difluoro-4-methoxyphenyl)-1-methyl-imidazole-2-carboxamide;
N-[3-[[2-chloro-4-[[5-(2,3-difluoro-4-methoxy-phenyl)-1-methyl-imidazole-2-carbonyl]amino]benzoyl]amino]cyclobutyl]piperidine-4-carboxamide;
N-[3-[[2-chloro-4-[[5-(2,3-difluoro-4-methoxy-phenyl)-1-methyl-imidazole-2-carbonyl]amino]benzoyl]amino]cyclobutyl]-4-hydroxy-piperidine-4-carboxamide;
(3R,4R)-N-[3-[[2-chloro-4-[[5-(2,3-difluoro-4-methoxy-phenyl)-1-methylimidazole-2-carbonyl]amino]benzoyl]amino]cyclobutyl]-3-hydroxy-piperidine-4-carboxamide;
(3 S,4S)-N-[3-[[2-chloro-4-[[5-(2,3-difluoro-4-methoxy-phenyl)-1-methyl-imidazole-2-carbonyl] amino]benzoyl] amino] cyclobutyl] -3 -hydroxy-piperidine-4-carboxamide;
N-[4-[2-[1-(Azetidin-3-ylmethyl)azetidin-3-yl]ethylcarbamoyl]-3-chloro-phenyl]-5-[4-(difluoromethoxy)-2,3-difluoro-phenyl]-1-methyl-imidazole-2-carboxamide;
N-[3-[[4-[[5-[4-(difluoromethoxy)-2,3-difluoro-phenyl]-1-methyl-imidazole-2-carbonyl]amino]-2-ethyl-benzoyl]amino]propyl]-1-(pyrrolidin-3-ylmethyl)piperidine-4-carboxamide;
1-(azetidin-3-ylmethyl)-N-[3-[[4-[[5-[4-(difluoromethoxy)-2,3-difluoro-phenyl]-1-methyl-imidazole-2-carbonyl]amino]-2-ethyl-benzoyl]amino]propyl]piperidine-4-carboxamide;
N-[4-[[1-(azetidin-3-ylmethyl)-4-piperidyl]methylcarbamoyl]-3-chloro-phenyl]-5-(2,3-difluoro-4-methoxy-phenyl)-1-methyl-imidazole-2-carboxamide;
5-(4-Methoxyphenyl)-1-methyl-N-[3-methyl-4-(methylcarbamoyl)phenyl]imidazole-2-carboxamide;
N-[3-chloro-4-[[(exo)-3-[(2S,4R)-4-hydroxyprolyl]-3-azabicyclo[3.1.0]hexan-6-yl]carbamoyl]phenyl]-5-(2,3-difluoro-4-methoxy-phenyl)-1-methyl-imidazole-2-carboxamide; and
(exo)-6-[[2-chloro-4-[[5-(2,3-difluoro-4-methoxy-phenyl)-1-methyl-imidazole-2-carbonyl] amino]b enzoyl] amino] -N- [(trans)-4-hydroxypyrrolidin-3 -yl] -3 - azabicyclo[3.1.0]hexane-3-carboxamide.

13. A compound of formula (I) according to any one of claims 1 to 12, or a pharmaceutically acceptable salt thereof, for use as therapeutically active substance.

14. A pharmaceutical composition comprising a compound of formula (I) according to any one of claims 1 to 12, or a pharmaceutically acceptable salt thereof, and a therapeutically inert carrier.

15. A compound of formula (I) according to any of claims 1 to 12, or a pharmaceutically acceptable salt thereof, for use as antibiotic.

## Patentansprüche

1. Verbindung der Formel (I) 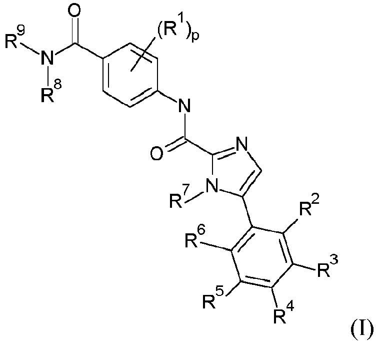 oder ein pharmazeutisch unbedenkliches Salz davon, wobei:
R¹ bei jedem Auftreten unabhängig voneinander aus Halogen, Cyano, C₁-C₆-Alkyl, C₁-C₆-Alkoxy, Halogen-C₁-C₆-alkyl und Halogen-C₁-C₆-alkoxy ausgewählt ist;
R², R³, R⁴, R⁵ und R⁶ jeweils unabhängig voneinander aus Wasserstoff, Halogen, C₁-C₆-Alkyl, C₁-C₆-Alkoxy, Cyano, Halogen-C₁-C₆-alkyl, Cyano-C₁-C₆-alkyl, (C₁-C₆-Alkyl)₂N-, Halogen-C₁-C₆-alkoxy, Cyano-C₁-C₆-alkoxy, C₁-C₆-Alkoxy-C₁-C₆-alkoxy-, (C₁-C₆-Alkyl)₂N-C(O)- und Carbamoyl-C₁-C₆-alkoxy ausgewählt sind;
R⁷ aus Wasserstoff, C₁-C₆-Alkyl und Halogen-C₁-C₆-alkyl ausgewählt ist;
R⁸ aus C₁-C₆-Alkyl, Amino-C₁-C₆-alkyl, Amino-C₁-C₆-alkyl-C(O)-, (C₁-C₆-Alkyl)₂N-C₁-C₆-alkyl-, C₁-C₆-Alkyl-NH-C₁-C₆-alkyl-, Amino-C₁-C₆-alkoxy-C₁-C₆-alkyl-, C₁-C₆-Alkyl-NH-C₁-C₆-alkoxy-C₁-C₆-alkyl-, (C₁-C₆-Alkyl)₂N-C₁-C₆-alkoxy-C₁-C₆-alkyl-, Amino-C₁-C₆-alkoxy-C₁-C₆-alkyl-C(O)-, C₁-C₆-Alkyl-NH-C₁-C₆-alkoxy-C₁-C₆-alkyl-C(O)-, (C₁-C₆-Alkyl)₂N-C₁-C₆-alkoxy-C₁-C₆-alkyl-C(O)-, Amino-C₁-C₆-alkyl-C(O)-NH-C₁-C₆-alkyl-, C₁-C₆-Alkyl-NH-C₁-C₆-alkyl-C(O)-NH-C₁-C₆-alkyl-, (C₁-C₆-Alkyl)₂N-C₁-C₆-alkyl-C(O)-NH-C₁-C₆-alkyl- und einer Gruppe 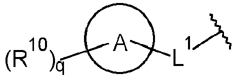 ausgewählt ist;
R⁹ aus Wasserstoff und C₁-C₆-Alkyl ausgewählt ist;
R¹⁰ aus Halogen, Cyano, Amino, Hydroxy, C₁-C₆-Alkyl, C₁-C₆-Alkoxy, Halogen-C₁-C₆-alkyl, Halogen-C₁-C₆-alkoxy, Amino-C₁-C₆-alkyl-, C₁-C₆-Alkyl-NH-C₁-C₆-alkyl-, (C₁-C₆-,Alkyl)₂N-C₁-C₆-alkyl-, Amino-C₁-C₆-alkyl-C(O)-, C₁-C₆-Alkyl-NH-C₁-C₆-alkyl-C(O)-, (C₁-C₆-Alkyl)₂N-C₁-C₆-alkyl-C(O)-, Amino-C₁-C₆-alkyl-NH-C(O)-, Amino-C₁-C₆-alkoxy-C₁-C₆-alkyl-C(O)-, Hydroxy-C₁-C₆-alkyl und einer Gruppe 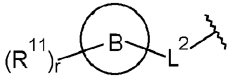 ausgewählt ist;
R¹¹ aus Halogen, Cyano, Amino, Hydroxy, C₁-C₆-Alkyl, C₁-C₆-Alkoxy, Halogen-C₁-C₆-alkyl, Halogen-C₁-C₆-alkoxy, Amino-C₁-C₆-alkyl-, C₁-C₆-Alkyl-NH-C₁-C₆-alkyl-, (C₁-C₆-Alkyl)₂N-C₁-C₆-alkyl-, Amino-C₁-C₆-alkyl-C(O)-, C₁-C₆-Alkyl-NH-C₁-C₆-alkyl-C(O)-, (C₁-C₆-Alkyl)₂N-C₁-C₆-alkyl-C(O)-, Amino-C₁-C₆-alkyl-NH-C(O)-, Amino-C₁-C₆-alkoxy-C₁-C₆-alkyl-C(O)- und Hydroxy-C₁-C₆-alkyl ausgewählt ist;
A und B jeweils unabhängig voneinander aus 3- bis 14-gliedrigem Heterocyclyl, C₃-C₁₀-Cycloalkyl, 5- bis 14-gliedrigem Heteroaryl und C₆-C₁₀-Aryl ausgewählt sind;
L¹ aus einer kovalenten Bindung, Carbonyl, -NH-C(O)-, C₁-C₆-Alkyl, -C(O)-NH-C₁-C₆-Alkyl-, -C₁-C₆-Alkyl-NH-C(O)- und -NH-C(O)-NH-C₁-C₆-Alkyl-ausgewählt ist;
L² aus einer kovalenten Bindung, Carbonyl, -NH-C(O)-, -C(O)-NH-, C₁-C₆-Alkyl, -C(O)-NH-C₁-C₆-Alkyl-, -C₁-C₆-Alkyl-NH-C(O)- und -NH-C(O)-NH-C₁-C₆-Alkyl- ausgewählt ist;
p aus 0, 1, 2, 3 oder 4 ausgewählt ist und
q und r unabhängig voneinander aus 0, 1, 2 und 3 ausgewählt sind.

2. Verbindung der Formel (I) nach Anspruch 1 oder ein pharmazeutisch unbedenkliches Salz davon, wobei die Verbindung der Formel (I) eine Verbindung der Formel (I-I) ist 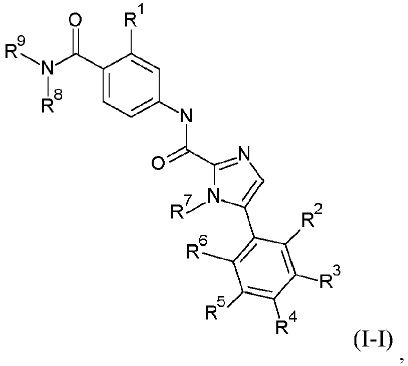 wobei R¹ bis R⁹ wie in Anspruch 1 definiert sind.

3. Verbindung der Formel (I) nach Anspruch 1 oder ein pharmazeutisch unbedenkliches Salz davon, wobei die Verbindung der Formel (I) eine Verbindung der Formel (1-11) ist 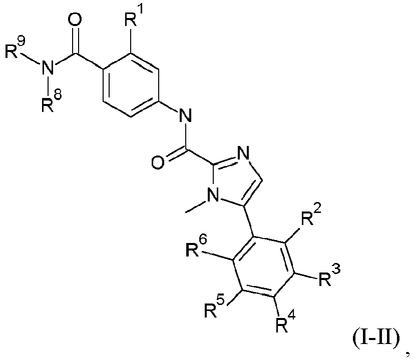 wobei R¹ bis R⁶, R⁸ und R⁹ wie in Anspruch 1 definiert sind.

4. Verbindung der Formel (I) nach einem der Ansprüche 1 bis 3 oder ein pharmazeutisch unbedenkliches Salz davon, wobei:
R¹ C₁-C₆-Alkyl oder Halogen ist;
R⁸ aus C₁-C₆-Alkyl, Amino-C₁-C₆-alkyl, (C₁-C₆-Alkyl)₂N-C₁-C₆-alkyl-, Amino-C₁-C₆-alkoxy-C₁-C₆-alkyl-, (C₁-C₆-Alkyl)₂N-C₁-C₆-alkoxy-C₁-C₆-alkyl-, Amino-C₁-C₆-alkyl-C(O)-NH-C₁-C₆-alkyl-, (C₁-C₆-Alkyl)₂N-C₁-C₆-alkyl-C(O)-NH-C₁-C₆-alkyl- und einer Gruppe ist; 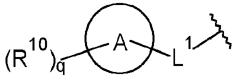 ausgewählt
R⁹ Wasserstoff ist;
R¹⁰ aus Amino, Hydroxy, C₁-C₆-Alkyl, Amino-C₁-C₆-alkyl-C(O)-, (C₁-C₆-Alkyl)₂N-C₁-C₆-alkyl-C(O)-, Amino-C₁-C₆-alkyl-NH-C(O)-, Amino-C₁-C₆-alkoxy-C₁-C₆-alkyl-C(O)-, Hydroxy-C₁-C₆-alkyl und einer Gruppe 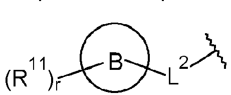 ausgewählt ist;
R¹¹ aus Hydroxy, Amino und Amino-C₁-C₆-alkyl ausgewählt ist;
A und B jeweils unabhängig voneinander aus 3- bis 14-gliedrigem Heterocyclyl und C₃-C₁₀-Cycloalkyl ausgewählt sind;
L¹ aus einer kovalenten Bindung, C₁-C₆-Alkyl, -C(O)-NH-C₁-C₆-Alkyl- und -NH-C(O)-NH-C₁-C₆-alkyl- ausgewählt ist;
L² aus Carbonyl, C₁-C₆-Alkyl, -C(O)-NH- und -NH-C(O)- ausgewählt ist;
p 1 ist und
q und r jeweils unabhängig voneinander aus 0 und 1 ausgewählt sind.

5. Verbindung der Formel (I) nach Anspruch 4 oder ein pharmazeutisch unbedenkliches Salz davon, wobei:
R¹ Halogen ist;
R⁸ eine Gruppe 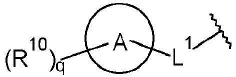 ist;
R⁹ Wasserstoff ist;
R¹⁰ aus Amino-C₁-C₆-alkyl-C(O)-, (C₁-C₆-Alkyl)₂N-C₁-C₆-alkyl-C(O)-, Amino-C₁-C₆-alkoxy-C₁-C₆-alkyl-C(O)- und einer Gruppe 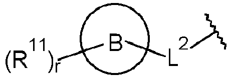 ausgewählt ist;
R¹¹ aus Hydroxy und Amino ausgewählt ist;
A und B jeweils unabhängig voneinander aus 3- bis 14-gliedrigem Heterocyclyl und C₃-C₁₀-Cycloalkyl ausgewählt sind;
L¹ aus einer kovalenten Bindung und C₁-C₆-Alkyl ausgewählt ist;
L² aus Carbonyl, -C(O)-NH- und -NH-C(O)- ausgewählt ist;
p 1 ist;
q 1 ist und
r aus 0 und 1 ausgewählt ist.

6. Verbindung der Formel (I) nach Anspruch 5 oder ein pharmazeutisch unbedenkliches Salz davon, wobei:
R¹ Chlor ist;
R⁸ eine Gruppe 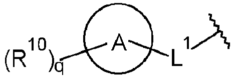 ist;
R⁹ Wasserstoff ist;
R¹⁰ aus 2-Aminoacetyl, 2-Aminopropanoyl, 2-(Dimethylamino)acetyl, 3-(2-Aminoethoxy)propanoyl und einer Gruppe ist; 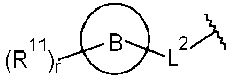 ausgewählt
R¹¹ aus Hydroxy und Amino ausgewählt ist;
A aus Azetidinyl, Cyclobutyl, Pyrrolidinyl und Piperidyl ausgewählt ist;
B aus Cyclobutyl, Pyrrolidinyl und Piperidyl ausgewählt ist;
L¹ aus einer kovalenten Bindung und -(CH₂)₂- ausgewählt ist;
L² aus Carbonyl, -C(O)-NH- und -NH-C(O)- ausgewählt ist;
p 1 ist;
q 1 ist und
r aus 0 und 1 ausgewählt ist.

7. Verbindung der Formel (I) nach einem der Ansprüche 1 bis 6 oder ein pharmazeutisch unbedenkliches Salz davon, wobei:
R² Wasserstoff oder Halogen ist;
R³ Wasserstoff oder Halogen ist;
R⁴ aus C₁-C₆-Alkoxy, Halogen-C₁-C₆-alkoxy, Cyano-C₁-C₆-alkoxy und Carbamoyl-C₁-C₆-alkoxy ausgewählt ist;
R⁵ Wasserstoff ist und
R⁶ Wasserstoff oder Halogen ist.

8. Verbindung der Formel (I) nach Anspruch 7 oder ein pharmazeutisch unbedenkliches Salz davon, wobei:
R² und R³ beide Halogen sind;
R⁴ aus C₁-C₆-Alkoxy und Halogen-C₁-C₆-alkoxy ausgewählt ist und
R⁵ und R⁶ beide Wasserstoff sind.

9. Verbindung der Formel (I) nach Anspruch 8 oder ein pharmazeutisch unbedenkliches Salz davon, wobei:
R² und R³ beide Fluor sind;
R⁴ aus Methoxy und Difluormethoxy ausgewählt ist und
R⁵ und R⁶ beide Wasserstoff sind.

10. Verbindung der Formel (I) nach einem der Ansprüche 1 bis 9 oder ein pharmazeutisch unbedenkliches Salz davon, wobei R⁷ C₁-C₆-Alkyl ist.

11. Verbindung der Formel (I) nach Anspruch 10 oder ein pharmazeutisch unbedenkliches Salz davon, wobei R⁷ Methyl ist.

12. Verbindung der Formel (I) nach Anspruch 1 oder ein pharmazeutisch unbedenkliches Salz davon, wobei die Verbindung der Formel (I) aus Folgenden ausgewählt ist:
N-[4-[2-(2-Aminoethoxy)ethylcarbamoyl]-3-chlorphenyl]-5-(2,3-difluor-4-methoxyphenyl)-1-methylimidazol-2-carboxamid;
N-[3-Chlor-4-(2-piperazin-1-ylethylcarbamoyl)phenyl]-5-(2,3-difluor-4-methoxyphenyl)-1-methylimidazol-2-carboxamid;
N-[3-Chlor-4-(pyrrolidin-3-ylcarbamoyl)phenyl]-5-(2,3-difluor-4-methoxyphenyl)-1-methylimidazol-2-carboxamid;
N-[3-Chlor-4-(4-piperidylcarbamoyl)phenyl]-5-(2,3-difluor-4-methoxyphenyl)-1-methylimidazol-2-carboxamid;
N-[3-Chlor-4-(4-piperidylmethylcarbamoyl)phenyl]-5-(2,3-difluor-4-methoxyphenyl)-1-methylimidazol-2-carboxamid;
N-[4-(6-Aminohexylcarbamoyl)-3-chlorphenyl]-5-(2,3-difluor-4-methoxyphenyl)-1-methylimidazol-2-carboxamid;
N-[4-[[3-(Aminomethyl)cyclobutyl]methylcarbamoyl]-3-chlorphenyl]-5-(2,3-difluor-4-methoxyphenyl)-1 -methylimidazol-2-carboxamid;
N-[4-[(3-Aminocyclobutyl)methylcarbamoyl]-3-chlorphenyl]-5-(2,3-difluor-4-methoxyphenyl)-1 -methylimidazol-2-carboxamid;
N-[4-[2-(2-Aminoethoxy)ethylcarbamoyl]-3-chlorphenyl]-5-[4-(cyanomethoxy)-2,3-difluorphenyl]-1-methylimidazol-2-carboxamid;
N-[3-Chlor-4-(2-piperazin-1-ylethylcarbamoyl)phenyl]-5-[4-(cyanomethoxy)-2,3-difluorphenyl]-1-methylimidazol-2-carboxamid;
N-[3-Chlor-4-(3-piperazin-1-ylpropylcarbamoyl)phenyl]-5-[4-(cyanomethoxy)-2,3-difluorphenyl]-1-methylimidazol-2-carboxamid;
5-[4-(Cyanomethoxy)-2,3-difluorphenyl]-1-methyl-N-[3-methyl-4-(3-piperazin-1-ylpropylcarbamoyl)phenyl]imidazol-2-carboxamid;
N-[4-[2-(2-Aminoethoxy)ethylcarbamoyl]-3-ethylphenyl]-5-(2,3-difluor-4-methoxyphenyl)-1 -methylimidazol-2-carboxamid;
N-[4-[2-(2-Aminoethoxy)ethylcarbamoyl]-3-ethylphenyl]-5-[4-(difluormethoxy)phenyl]-1-methylimidazol-2-carboxamid;
N-[4-[2-(2-Aminoethoxy)ethylcarbamoyl]-3-ethylphenyl]-5-(3-fluor-4-isopropoxyphenyl)-1-methylimidazol-2-carboxamid;
N-[4-[2-(2-Aminoethoxy)ethylcarbamoyl]-3-ethylphenyl]-5-(2-chlor-4-methoxyphenyl)-1 -methylimidazol-2-carboxamid;
N-[4-[2-(2-Aminoethoxy)ethylcarbamoyl]-3-ethylphenyl]-5-[4-(difluormethoxy)-2,3-difluorphenyl]-1-methylimidazol-2-carboxamid;
N-[4-[2-(2-Aminoethoxy)ethylcarbamoyl]-3-ethylphenyl]-5-(2,6-difluor-4-methoxyphenyl)-1 -methylimidazol-2-carboxamid;
N-[4-(3-Aminopropylcarbamoyl)-3-chlorphenyl]-5-[4-(difluormethoxy)-2,3-difluorphenyl]-1-methylimidazol-2-carboxamid;
N-[4-[2-(2-Aminoethoxy)ethylcarbamoyl]-3-ethylphenyl]-5-[4-(cyanomethoxy)-2,3-difluorphenyl]-1-methylimidazol-2-carboxamid;
N-[4-[2-(2-Aminoethoxy)ethylcarbamoyl]-3-ethylphenyl]-5-[4-(2-amino-2-oxoethoxy)-2,3-difluorphenyl]-1-methylimidazol-2-carboxamid;
N-[4-[2-(2-Aminoethoxy)ethylcarbamoyl]-3-ethylphenyl]-5-[2-chlor-4-(cyanomethoxy)-3-fluorphenyl]-1-methylimidazol-2-carboxamid;
N-[3-Chlor-4-[[1-(piperidin-4-carbonyl)pyrrolidin-3-yl]carbamoyl]phenyl]-5-(2,3-difluor-4-methoxyphenyl)-1-methylimidazol-2-carboxamid;
N-[3-Chlor-4-[[1-[2-(dimethylamino)acetyl]pyrrolidin-3-yl]carbamoyl]phenyl]-5-(2,3-difluor-4-methoxyphenyl)-1-methylimidazol-2-carboxamid;
N-[3-Chlor-4-[2-[[2-(dimethylamino)acetyl]amino]ethylcarbamoyl]phenyl]-5-(2,3-difluor-4-methoxyphenyl)-1-methylimidazol-2-carboxamid;
N-[3-Chlor-4-[[1-[2-(dimethylamino)acetyl]-4-piperidyl]carbamoyl]phenyl]-5-(2,3-difluor-4-methoxyphenyl)-1-methylimidazol-2-carboxamid;
N-[3-Chlor-4-[5-(dimethylamino)pentylcarbamoyl]phenyl]-5-(2,3-difluor-4-methoxyphenyl)-1-methylimidazol-2-carboxamid;
N-[3-Chlor-4-(2-isopentyloxyethylcarbamoyl)phenyl]-5-[4-(cyanomethoxy)-2,3-difluorphenyl]-1-methylimidazol-2-carboxamid;
N-[3-Chlor-4-[2-(3-hydroxypyrrolidin-1-yl)ethylcarbamoyl]phenyl]-5-(2,3-difluor-4-methoxyphenyl)-1-methylimidazol-2-carboxamid;
N-[3-Chlor-4-[2-[3-(hydroxymethyl)pyrrolidin-1-yl]ethylcarbamoyl]phenyl]-5-(2,3-difluor-4-methoxyphenyl)-1-methylimidazol-2-carboxamid;
5-(2,3-Difluor-4-methoxyphenyl)-N-[4-[3-[2-(dimethylamino)ethoxy]propylcarbamoyl]-3-ethylphenyl]-1-methylimidazol-2-carboxamid;
N-[3-Chlor-4-[[1-[(3R)-pyrrolidin-3-carbonyl]pyrrolidin-3-yl]carbamoyl]phenyl]-5-(2,3-difluor-4-methoxyphenyl)-1-methylimidazol-2-carboxamid;
N-[3-Chlor-4-[[1-[(2S,4R)-4-hydroxypyrrolidin-2-carbonyl]pyrrolidin-3-yl]carbamoyl]phenyl]-5-(2,3-difluor-4-methoxyphenyl)-1-methylimidazol-2-carboxamid;
N-[4-[[1-[3-(2-Aminoethoxy)propanoyl]pyrrolidin-3-yl]carbamoyl]-3-chlorphenyl]-5-(2,3-difluor-4-methoxyphenyl)-1-methylimidazol-2-carboxamid;
N-[3-Chlor-4-[[1-(piperidin-4-carbonyl)-4-piperidyl]carbamoyl]phenyl]-5-(2,3-difluor-4-methoxyphenyl)-1-methylimidazol-2-carboxamid;
N-[3-Chlor-4-[[1-[(3R)-pyrrolidin-3-carbonyl]-4-piperidyl]carbamoyl]phenyl]-5-(2,3-difluor-4-methoxyphenyl)-1-methylimidazol-2-carboxamid;
N-[3-Chlor-4-[[1-[(2S,4R)-4-hydroxypyrrolidin-2-carbonyl]-4-piperidyl]carbamoyl]phenyl]-5-(2,3-difluor-4-methoxyphenyl)-1-methylimidazol-2-carboxamid;
N-[4-[[1-[3-(2-Aminoethoxy)propanoyl]-4-piperidyl]carbamoyl]-3-chlorphenyl]-5-(2,3-difluor-4-methoxyphenyl)-1-methylimidazol-2-carboxamid;
N-[4-[[3-[[3-(Aminomethyl)cyclobutancarbonyl]amino]cyclobutyl]carbamoyl]-3-chlorphenyl]-5-(2,3-difluor-4-methoxyphenyl)-1-methylimidazol-2-carboxamid;
N-[4-[2-[1-(3-Aminopropanoyl)azetidin-3-yl]ethylcarbamoyl]-3-chlorphenyl]-5-[4-(difluormethoxy)-2,3-difluorphenyl]-1-methylimidazol-2-carboxamid;
N-[4-[3-(3-Aminopropanoylamino)propylcarbamoyl]-3-chlorphenyl]-5-[4-(difluormethoxy)-2,3-difluorphenyl]-1-methylimidazol-2-carboxamid;
N-[3-[[4-[[5-[4-(Difluormethoxy)-2,3-difluorphenyl]-1-methylimidazol-2-carbonyl] amino] -2-ethylbenzoyl] amino] propyl] piperidin-4-carboxamid;
N-[2-[[4-[[5-[4-(Cyanomethoxy)-2,3-difluorphenyl]-1-methylimidazol-2-carbonyl] amino] -2-methylbenzoyl] amino] ethyl] piperidin-4-carboxamid;
N-[2-[[4-[[5-[4-(Cyanomethoxy)-2,3-difluorphenyl]-1-methylimidazol-2-carbonyl] amino] -2-methylbenzoyl] amino] ethyl] -4-hydroxypiperidin-4-carboxamid;
N-[2-[[2-Chlor-4-[[5-[4-(cyanomethoxy)-2,3-difluorphenyl]-1-methylimidazol-2-carbonyl]amino]benzoyl]amino]ethyl]piperidin-4-carboxamid;
N-[2-[[2-Chlor-4-[[5-[4-(cyanomethoxy)-2,3-difluorphenyl]-1-methylimidazol-2-carbonyl]amino]benzoyl]amino]ethyl]-4-hydroxypiperidin-4-carboxamid;
N-[4-[[1-(2-Aminoacetyl)azetidin-3-yl]carbamoyl]-3-chlorphenyl]-5-(2,3-difluor-4-methoxyphenyl)-1-methylimidazol-2-carboxamid;
N-[4-[[1-(2-Aminoacetyl)azetidin-3-yl]methylcarbamoyl]-3-chlorphenyl]-5-(2,3-difluor-4-methoxyphenyl)-1-methylimidazol-2-carboxamid;
N-[4-[[3-(2-Aminoethylcarbamoyl)cyclobutyl]carbamoyl]-3-chlorphenyl]-5-(2,3-difluor-4-methoxyphenyl)-1-methylimidazol-2-carboxamid;
N-[4-[[3-(3-Aminoazetidin-1-carbonyl)cyclobutyl]carbamoyl]-3-chlorphenyl]-5-(2,3-difluor-4-methoxyphenyl)-1-methylimidazol-2-carboxamid;
N-[4-[[3-[(3-Aminocyclobutyl)carbamoyl]cyclobutyl]carbamoyl]-3-chlorphenyl]-5-(2,3-difluor-4-methoxyphenyl)-1-methylimidazol-2-carboxamid;
N-[4-[[3-(2-Aminoethylcarbamoyl)cyclobutyl]carbamoyl]-3-ethylphenyl]-5-(2,3-difluor-4-methoxyphenyl)-1-methylimidazol-2-carboxamid;
N-[4-[[1-(2-Aminoacetyl)azetidin-3-yl]carbamoyl]-3-ethylphenyl]-5-(2,3-difluor-4-methoxyphenyl)-1-methylimidazol-2-carboxamid;
5-[4-(Cyanomethoxy)-2,3-difluorphenyl]-N-[4-[2-[[(2R,4R)-4-hydroxy-4-methylpyrrolidin-2-carbonyl] amino] ethylcarbamoyl] -3-methylphenyl] -1 - methylimidazol-2-carboxamid;
5-[4-(Cyanomethoxy)-2,3-difluorphenyl]-N-[4-[2-[[(2R,4R)-4-ethyl-4-hydroxypyrrolidin-2-carbonyl]amino]ethylcarbamoyl]-3-methylphenyl]-1-methylimidazol-2-carboxamid;
5-(2,3-Difluor-4-methoxyphenyl)-N-[4-[2-[[(2R,4R)-4-hydroxy-4-methylpyrrolidin-2-carbonyl]amino]ethylcarbamoyl]-3-methylphenyl]-1-methylimidazol-2-carboxamid;
5-(2,3-Difluor-4-methoxyphenyl)-N-[4-[2-[[(2R,4R)-4-ethyl-4-hydroxypyrrolidin-2-carbonyl]amino]ethylcarbamoyl]-3-methylphenyl]-1-methylimidazol-2-carboxamid;
5-(2,3-Difluor-4-methoxyphenyl)-N-[4-[2-[[(3R,4R)-4-hydroxypyrrolidin-3-yl] carbamoylamino] ethylcarbamoyl] -3 -methylphenyl] -1 -methylimidazol-2-carboxamid;
5-(2,3-Difluor-4-methoxyphenyl)-N-[4-[2-[[(3S,4S)-4-hydroxypyrrolidin-3-yl] carbamoylamino] ethylcarbamoyl] -3 -methylphenyl] -1 -methylimidazol-2-carboxamid;
5-[4-(Cyanomethoxy)-2,3-difluorphenyl]-N-[4-[2-[[(3R,4R)-4-hydroxypyrrolidin-3-yl] carbamoylamino] ethylcarbamoyl] -3 -methylphenyl] -1 -methylimidazol-2-carboxamid;
5-[4-(Cyanomethoxy)-2,3-difluorphenyl]-N-[4-[2-[[(3S,4S)-4-hydroxypyrrolidin-3-yl] carbamoylamino] ethylcarbamoyl] -3 -methylphenyl] -1 -methylimidazol-2-carboxamid;
(3R,4R)-N-[2-[[2-Chlor-4-[[5-[4-(cyanomethoxy)-2,3-difluorphenyl]-1-methylimidazol-2-carbonyl]amino]benzoyl]amino]ethyl]-3-hydroxypiperidin-4-carboxamid;
(3S,4S)-N-[2-[[2-Chlor-4-[[5-[4-(cyanomethoxy)-2,3-difluorphenyl]-1-methylimidazol-2-carbonyl]amino]benzoyl]amino]ethyl]-3-hydroxypiperidin-4-carboxamid;
(3S,4R)-N-[2-[[2-Chlor-4-[[5-[4-(cyanomethoxy)-2,3-difluorphenyl]-1-methylimidazol-2-carbonyl]amino] benzoyl] amino] ethyl] -3 -hydroxypiperidin-4-carboxamid;
(3R,4S)-N-[2-[[2-Chlor-4-[[5-[4-(cyanomethoxy)-2,3-difluorphenyl]-1-methylimidazol-2-carbonyl]amino]benzoyl]amino]ethyl]-3-hydroxypiperidin-4-carboxamid;
N-[3-Chlor-4-[2-[[(2R,4R)-4-hydroxy-4-methylpyrrolidin-2-carbonyl]amino]ethylcarbamoyl]phenyl]-5-[4-(cyanomethoxy)-2,3-difluorphenyl]-1-methylimidazol-2-carboxamid;
N-[3-Chlor-4-[2-[[(2R,4R)-4-ethyl-4-hydroxypyrrolidin-2-carbonyl]amino]ethylcarbamoyl]phenyl]-5-[4-(cyanomethoxy)-2,3-difluorphenyl]-1-methylimidazol-2-carboxamid;
N-[3-Chlor-4-[[3-[[(4R)-4-hydroxypyrrolidin-2-carbonyl]amino]cyclobutyl]carbamoyl]phenyl]-5-(2,3-difluor-4-methoxyphenyl)-1-methylimidazol-2-carboxamid;
N-[3-[[2-Chlor-4-[[5-(2,3-difluor-4-methoxyphenyl)-1-methylimidazol-2-carbonyl] amino] benzoyl] amino] cyclobutyl]piperidin-4-carboxamid;
N-[3-[[2-Chlor-4-[[5-(2,3-difluor-4-methoxyphenyl)-1-methylimidazol-2-carbonyl]amino]benzoyl]amino]cyclobutyl]-4-hydroxypiperidin-4-carboxamid;
(3R,4R)-N-[3-[[2-Chlor-4-[[5-(2,3-difluor-4-methoxyphenyl)-1-methylimidazol-2-carbonyl]amino]benzoyl]amino]cyclobutyl]-3-hydroxypiperidin-4-carboxamid;
(3S,4S)-N-[3-[[2-Chlor-4-[[5-(2,3-difluor-4-methoxyphenyl)-1-methylimidazol-2-carbonyl]amino]benzoyl]amino]cyclobutyl]-3-hydroxypiperidin-4-carboxamid;
N-[4-[2-[1-(Azetidin-3-ylmethyl)azetidin-3-yl]ethylcarbamoyl]-3-chlorphenyl]-5-[4-(difluormethoxy)-2,3-difluorphenyl]-1-methylimidazol-2-carboxamid;
N-[3-[[4-[[5-[4-(Difluormethoxy)-2,3-difluorphenyl]-1-methylimidazol-2-carbonyl] amino] -2-ethylbenzoyl] amino] propyl] -1 -(pyrrolidin-3 - ylmethyl)piperidin-4-carboxamid;
1-(Azetidin-3-ylmethyl)-N-[3-[[4-[[5-[4-(difluormethoxy)-2,3-difluorphenyl]-1-methylimidazol-2-carbonyl]amino]-2-ethylbenzoyl]amino]propyl]piperidin-4-carboxamid;
N-[4-[[1-(Azetidin-3-ylmethyl)-4-piperidyl]methylcarbamoyl]-3-chlorphenyl]-5-(2,3-difluor-4-methoxyphenyl)-1-methylimidazol-2-carboxamid;
5-(4-Methoxyphenyl)-1-methyl-N-[3-methyl-4-(methylcarbamoyl)phenyl]imidazol-2-carboxamid;
N-[3-Chlor-4-[[(exo)-3-[(2S,4R)-4-hydroxyprolyl]-3-azabicyclo[3.1.0]hexan-6-yl]carbamoyl]phenyl]-5-(2,3-difluor-4-methoxyphenyl)-1-methylimidazol-2-carboxamid und
(exo)-6-[[2-Chlor-4-[[5-(2,3-difluor-4-methoxyphenyl)-1-methylimidazol-2-carbonyl]amino]benzoyl]amino]-N-[(trans)-4-hydroxypyrrolidin-3-yl]-3-azabicyclo[3.1.0]hexan-3-carboxamid.

13. Verbindung der Formel (I) nach einem der Ansprüche 1 bis 12 oder ein pharmazeutisch unbedenkliches Salz davon zur Verwendung als therapeutisch wirksame Substanz.

14. Pharmazeutische Zusammensetzung, umfassend eine Verbindung der Formel (I) nach einem der Ansprüche 1 bis 12 oder ein pharmazeutisch unbedenkliches Salz davon und einen therapeutisch inerten Träger.

15. Verbindung der Formel (I) nach einem der Ansprüche 1 bis 12 oder ein pharmazeutisch unbedenkliches Salz davon zur Verwendung als Antibiotikum.

## Revendications

1. Composé de formule (I) 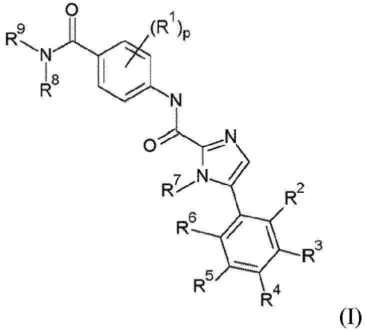 ou un sel pharmaceutiquement acceptable de celui-ci, dans lequel :
R¹ est, à chaque occurrence, indépendamment choisi parmi un halogène, un cyano, un alkyle en C₁-C₆, un alcoxy en C₁-C₆, un halogénoalkyle en C₁-C₆ et un halogénoalcoxy en C₁-C₆ ;
R², R³, R⁴, R⁵ et R⁶ sont chacun indépendamment choisis parmi un hydrogène, un halogène, un alkyle en C₁-C₆, un alcoxy en C₁-C₆, un cyano, un halogénoalkyle en C₁-C₆, un cyanoalkyle en C₁-C₆, un (alkyle en C₁-C₆)₂N-, un halogénoalcoxy en C₁-C₆, un cyanoalcoxy en C₁-C₆, un alcoxy en C₁-C₆-alcoxy en C₁-C₆-, un (alkyle en C₁-C₆)₂N-C(O)- et un carbamoylalcoxy en C₁-C₆ ;
R⁷ est choisi parmi un hydrogène, un alkyle en C₁-C₆ et un halogénoalkyle en C₁-C₆ ;
R⁸ est choisi parmi un alkyle en C₁-C₆, un aminoalkyle en C₁-C₆, un aminoalkyle en C₁-C₆-C(O)-, un (alkyle en C₁-C₆)₂N-alkyle en C₁-C₆-, un alkyle en C₁-C₆-NH-alkyle en C₁-C₆-, un aminoalcoxy en C₁-C₆-alkyle en C₁-C₆-, un alkyle en C₁-C₆-NH-alcoxy en C₁-C₆-alkyle en C₁-C₆-, un (alkyle en C₁-C₆)₂N-alcoxy en C₁-C₆-alkyle en C₁-C₆-, un aminoalcoxy en C₁-C₆-alkyle en C₁-C₆-C(O)-, un alkyle en C₁-C₆-NH-alcoxy en C₁-C₆-alkyle en C₁-C₆-C(O)-, un (alkyle en C₁-C₆)₂N-alcoxy en C₁-C₆-alkyle en C₁-C₆-C(O)-, un aminoalkyle en C₁-C₆-C(O)-NH-alkyle en C₁-C₆-, un alkyle en C₁-C₆-NH-alkyle en C₁-C₆-C(O)-NH-alkyle en C₁-C₆-, un (alkyle en C₁-C₆)₂N-alkyle en C₁-C₆-C(O)-NH-alkyle en C₁-C₆- et un groupe 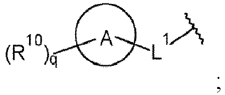
R⁹ est choisi parmi un hydrogène et un alkyle en C₁-C₆ ;
R¹⁰ est choisi parmi un halogène, un cyano, un amino, un hydroxy, un alkyle en C₁-C₆, un alcoxy en C₁-C₆, un halogénoalkyle en C₁-C₆, un halogénoalcoxy en C₁-C₆, un aminoalkyle en C₁-C₆-, un alkyle en C₁-C₆-NH-alkyle en C₁-C₆-, un (alkyle en C₁-C₆)₂N-alkyle en C₁-C₆-, un aminoalkyle en C₁-C₆-C(O)-, un alkyle en C₁-C₆-NH-alkyle en C₁-C₆-C(O)-, un (alkyle en C₁-C₆)₂N-alkyle en C₁-C₆-C(O)-, un aminoalkyle en C₁-C₆-NH-C(O)-, un aminoalcoxy en C₁-C₆-alkyle en C₁-C₆-C(O)-, un hydroxyalkyle en C₁-C₆ et un groupe 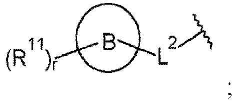
R¹¹ est choisi parmi un halogène, un cyano, un amino, un hydroxy, un alkyle en C₁-C₆, un alcoxy en C₁-C₆, un halogénoalkyle en C₁-C₆, un halogénoalcoxy en C₁-C₆, un aminoalkyle en C₁-C₆-, un alkyle en C₁-C₆-NH-alkyle en C₁-C₆-, un (alkyle en C₁-C₆)₂N-alkyle en C₁-C₆-, un aminoalkyle en C₁-C₆-C(O)-, un alkyle en C₁-C₆-NH-alkyle en C₁-C₆-C(O)-, un (alkyle en C₁-C₆)₂N-alkyle en C₁-C₆-C(O)-, un aminoalkyle en C₁-C₆-NH-C(O)-, un aminoalcoxy en C₁-C₆-alkyle en C₁-C₆-C(O)- et un hydroxyalkyle en C₁-C₆ ;
A et B sont chacun indépendamment choisis parmi un hétérocyclyle à 3 à 14 chaînons, un cycloalkyle en C₃-C₁₀, un hétéroaryle à 5 à 14 chaînons et un aryle en C₆-C₁₀ ;
L¹ est choisi parmi une liaison covalente, un carbonyle, -NH-C(O)-, un alkyle en C₁-C₆, -C(O)-NH-alkyle en C₁-C₆-, -alkyle en C₁-C₆-NH-C(O)- et-NH-C(O)-NH-alkyle en C₁-C₆- ;
L² est choisi parmi une liaison covalente, un carbonyle, -NH-C(O)-, -C(O)-NH-, un alkyle en C₁-C₆, -C(O)-NH-alkyle en C₁-C₆-, -alkyle en C₁-C₆-NH-C(O)- et-NH-C(O)-NH-alkyle en C₁-C₆- ;
p est choisi parmi 0, 1, 2, 3 ou 4 ; et
q et r sont indépendamment choisis parmi 0, 1, 2 et 3.

2. Composé de formule (I) selon la revendication 1, ou un sel pharmaceutiquement acceptable de celui-ci, dans lequel ledit composé de formule (I) est un composé de formule (I-I) 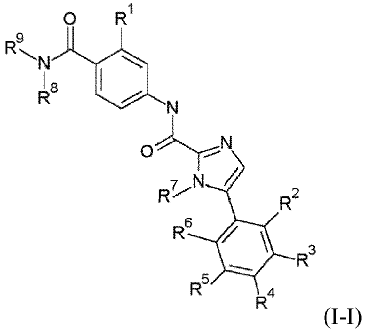 dans lequel R¹ à R⁹ sont tels que définis dans la revendication 1.

3. Composé de formule (I) selon la revendication 1, ou un sel pharmaceutiquement acceptable de celui-ci, dans lequel ledit composé de formule (I) est un composé de formule (1-11) 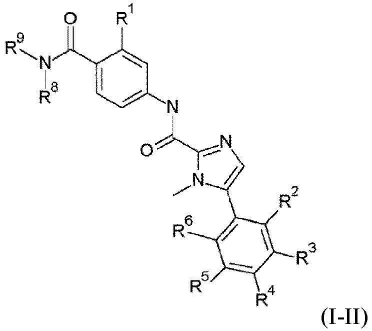 dans lequel R¹ à R⁶, R⁸ et R⁹ sont tels que définis dans la revendication 1.

4. Composé de formule (1) selon l'une quelconque des revendications 1 à 3, ou un sel pharmaceutiquement acceptable de celui-ci, dans lequel :
R¹ représente un alkyle en C₁-C₆ ou un halogène ;
R⁸ est choisi parmi un alkyle en C₁-C₆, un aminoalkyle en C₁-C₆, un (alkyle en C₁-C₆)₂N-alkyle en C₁-C₆-, un aminoalcoxy en C₁-C₆-alkyle en C₁-C₆-, un (alkyle en C₁-C₆)₂N-alcoxy en C₁-C₆-alkyle en C₁-C₆-, un aminoalkyle en C₁-C₆-C(O)-NH-alkyle en C₁-C₆-, un (alkyle en C₁-C₆)₂N-alkyle en C₁-C₆-C(O)-NH-alkyle en C₁-C₆- et un groupe 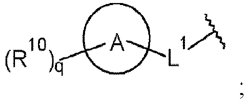
R⁹ représente un hydrogène ;
R¹⁰ est choisi parmi un amino, un hydroxy, un alkyle en C₁-C₆, un aminoalkyle en C₁-C₆-C(O)-, un (alkyle en C₁-C₆)₂N-alkyle en C₁-C₆-C(O)-, un aminoalkyle en C₁-C₆-NH-C(O)-, un aminoalcoxy en C₁-C₆-alkyle en C₁-C₆-C(O)-, un hydroxyalkyle en C₁-C₆ et un groupe 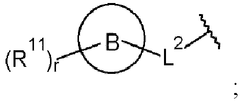
R¹¹ est choisi parmi un hydroxy, un amino et un aminoalkyle en C₁-C₆ ;
A et B sont chacun indépendamment choisis parmi un hétérocyclyle à 3 à 14 chaînons et un cycloalkyle en C₃-C₁₀ ;
L¹ est choisi parmi une liaison covalente, un alkyle en C₁-C₆, -C(O)-NH-alkyle en C₁-C₆- et -NH-C(O)-NH-alkyle en C₁-C₆- ;
L² est choisi parmi un carbonyle, un alkyle en C₁-C₆, -C(O)-NH- et -NH-C(O)- ;
p représente 1 ; et
q et r sont chacun indépendamment choisis parmi 0 et 1.

5. Composé de formule (I) selon la revendication 4, ou un sel pharmaceutiquement acceptable de celui-ci, dans lequel :
R¹ représente un halogène ;
R⁸ représente un groupe 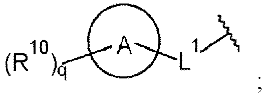
R⁹ représente un hydrogène ;
R¹⁰ est choisi parmi un aminoalkyle en C₁-C₆-C(O)-, un (alkyle en C₁-C₆)₂N-alkyle en C₁-C₆-C(O)-, un aminoalcoxy en C₁-C₆-alkyle en C₁-C₆-C(O)- et un groupe 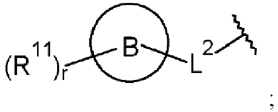
R¹¹ est choisi parmi un hydroxy et un amino ;
A et B sont chacun indépendamment choisis parmi un hétérocyclyle à 3 à 14 chaînons et un cycloalkyle en C₃-C₁₀ ;
L¹ est choisi parmi une liaison covalente et un alkyle en C₁-C₆ ;
L² est choisi parmi un carbonyle, -C(O)-NH- et -NH-C(O)- ;
p représente 1 ;
q représente 1 ; et
r est choisi parmi 0 et 1.

6. Composé de formule (I) selon la revendication 5, ou un sel pharmaceutiquement acceptable de celui-ci, dans lequel :
R¹ représente un chlore ;
R⁸ représente un groupe 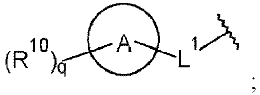
R⁹ représente un hydrogène ;
R¹⁰ est choisi parmi un 2-aminoacétyle, un 2-aminopropanoyle, un 2-(diméthylamino)acétyle, un 3-(2-aminoéthoxy)propanoyle et un groupe 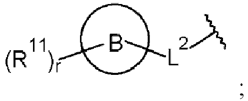
R¹¹ est choisi parmi un hydroxy et un amino ;
A est choisi parmi un azétidinyle, un cyclobutyle, un pyrrolidinyle et un pipéridyle ;
B est choisi parmi un cyclobutyle, un pyrrolidinyle et un pipéridyle ;
L¹ est choisi parmi une liaison covalente et -(CH₂)₂- ;
L² est choisi parmi un carbonyle, -C(O)-NH- et -NH-C(O)- ;
p représente 1 ;
q représente 1 ; et
r est choisi parmi 0 et 1.

7. Composé de formule (1) selon l'une quelconque des revendications 1 à 6, ou un sel pharmaceutiquement acceptable de celui-ci, dans lequel :
R² représente un hydrogène ou un halogène ;
R³ représente un hydrogène ou un halogène ;
R⁴ est choisi parmi un alcoxy en C₁-C₆, un halogénoalcoxy en C₁-C₆, un cyanoalcoxy en C₁-C₆ et un carbamoylalcoxy en C₁-C₆ ;
R⁵ représente un hydrogène ; et
R⁶ représente un hydrogène ou un halogène.

8. Composé de formule (I) selon la revendication 7, ou un sel pharmaceutiquement acceptable de celui-ci, dans lequel :
R² et R³ représentent tous deux un halogène ;
R⁴ est choisi parmi un alcoxy en C₁-C₆ et un halogénoalcoxy en C₁-C₆ ; et
R⁵ et R⁶ représentent tous deux un hydrogène.

9. Composé de formule (I) selon la revendication 8, ou un sel pharmaceutiquement acceptable de celui-ci, dans lequel :
R² et R³ représentent tous deux un fluor ;
R⁴ est choisi parmi un méthoxy et un difluorométhoxy ; et
R⁵ et R⁶ représentent tous deux un hydrogène.

10. Composé de formule (1) selon l'une quelconque des revendications 1 à 9, ou un sel pharmaceutiquement acceptable de celui-ci, dans lequel R⁷ représente un alkyle en C₁-C₆.

11. Composé de formule (I) selon la revendication 10, ou un sel pharmaceutiquement acceptable de celui-ci, dans lequel R⁷ représente un méthyle.

12. Composé de formule (I) selon la revendication 1, ou un sel pharmaceutiquement acceptable de celui-ci, dans lequel ledit composé de formule (I) est choisi parmi :
le
N-[4-[2-(2-aminoéthoxy)éthylcarbamoyl]-3-chloro-phényl]-5-(2,3-difluoro-4-méthoxy-phényl)-1-méthyl-imidazole-2-carboxamide ;
le
N-[3-chloro-4-(2-pipérazin-1-yléthylcarbamoyl)phényl]-5-(2,3-difluoro-4-méthoxy-phényl)-1-méthyl-imidazole-2-carboxamide ;
le
N-[3-chloro-4-(pyrrolidin-3-ylcarbamoyl)phényl]-5-(2,3-difluoro-4-méthoxy-phényl)-1-méthyl-imidazole-2-carboxamide ;
le
N-[3-chloro-4-(4-pipéridylcarbamoyl)phényl]-5-(2,3-difluoro-4-méthoxy-phényl)-1-méthyl-imidazole-2-carboxamide ;
le
N-[3-chloro-4-(4-pipéridylméthylcarbamoyl)phényl]-5-(2,3-difluoro-4-méthoxy-phényl)-1-méthyl-imidazole-2-carboxamide ;
le
N-[4-(6-aminohexylcarbamoyl)-3-chloro-phényl]-5-(2,3-difluoro-4-méthoxy-phényl)-1-méthyl-imidazole-2-carboxamide ;
le
N-[4-[[3-(aminométhyl)cyclobutyl]méthylcarbamoyl]-3-chloro-phényl]-5-(2,3-difluoro-4-méthoxy-phényl)-1-méthyl-imidazole-2-carboxamide ;
le
N-[4-[(3-aminocyclobutyl)méthylcarbamoyl]-3-chloro-phényl]-5-(2,3-difluoro-4-méthoxy-phényl)-1-méthyl-imidazole-2-carboxamide ;
le
N-[4-[2-(2-aminoéthoxy)éthylcarbamoyl]-3-chloro-phényl]-5-[4-(cyanométhoxy)-2,3-difluoro-phényl]-1-méthyl-imidazole-2-carboxamide ;
le
N-[3-chloro-4-(2-pipérazin-1-yléthylcarbamoyl)phényl]-5-[4-(cyanométhoxy)-2,3-difluoro-phényl]-1-méthyl-imidazole-2-carboxamide ;
le
N-[3-chloro-4-(3-pipérazin-1-ylpropylcarbamoyl)phényl]-5-[4-(cyanométhoxy)-2,3-difluoro-phényl]-1-méthyl-imidazole-2-carboxamide ;
le
5-[4-(cyanométhoxy)-2,3-difluoro-phényl]-1-méthyl-N-[3-méthyl-4-(3-pipérazin-1-ylpropylcarbamoyl)phényl]imidazole-2-carboxamide ;
le
N-[4-[2-(2-aminoéthoxy)éthylcarbamoyl]-3-éthyl-phényl]-5-(2,3-difluoro-4-méthoxy-phényl)-1-méthyl-imidazole-2-carboxamide ;
le
N-[4-[2-(2-aminoéthoxy)éthylcarbamoyl]-3-éthyl-phényl]-5-[4-(difluorométhoxy) phényl]-1-méthyl-imidazole-2-carboxamide ;
le
N-[4-[2-(2-aminoéthoxy)éthylcarbamoyl]-3-éthyl-phényl]-5-(3-fluoro-4-isopropoxy-phényl)-1-méthyl-imidazole-2-carboxamide ;
le
N-[4-[2-(2-aminoéthoxy)éthylcarbamoyl]-3-éthyl-phényl]-5-(2-chloro-4-méthoxy-phényl)-1-méthyl-imidazole-2-carboxamide ;
le
N-[4-[2-(2-aminoéthoxy)éthylcarbamoyl]-3-éthyl-phényl]-5-[4-(difluorométhoxy)-2,3-difluoro-phényl]-1-méthyl-imidazole-2-carboxamide ;
le
N-[4-[2-(2-aminoéthoxy)éthylcarbamoyl]-3-éthyl-phényl]-5-(2,6-difluoro-4-méthoxy-phényl)-1-méthyl-imidazole-2-carboxamide ;
le
N-[4-(3-aminopropylcarbamoyl)-3-chloro-phényl]-5-[4-(difluorométhoxy)-2,3-difluoro-phényl]-1-méthyl-imidazole-2-carboxamide ;
le
N-[4-[2-(2-aminoéthoxy)éthylcarbamoyl]-3-éthyl-phényl]-5-[4-(cyanométhoxy)-2,3-difluoro-phényl]-1-méthyl-imidazole-2-carboxamide ;
le
N-[4-[2-(2-aminoéthoxy)éthylcarbamoyl]-3-éthyl-phényl]-5-[4-(2-amino-2-oxo-éthoxy)-2,3-difluoro-phényl]-1-méthyl-imidazole-2-carboxamide ;
le
N-[4-[2-(2-aminoéthoxy)éthylcarbamoyl]-3-éthyl-phényl]-5-[2-chloro-4-(cyanométhoxy)-3-fluoro-phényl]-1-méthyl-imidazole-2-carboxamide ;
le
N-[3-chloro-4-[[1-(pipéridine-4-carbonyl)pyrrolidin-3-yl]carbamoyl]phényl]-5-(2,3-difluoro-4-méthoxy-phényl)-1-méthyl-imidazole-2-carboxamide ;
le
N-[3-chloro-4-[[1-[2-(diméthylamino)acétyl]pyrrolidin-3-yl]carbamoyl]phényl]-5-(2,3-difluoro-4-méthoxy-phényl)-1-méthyl-imidazole-2-carboxamide ;
le
N-[3-chloro-4-[2-[[2-(diméthylamino)acétyl]amino]éthylcarbamoyl]phényl]-5-(2,3-difluoro-4-méthoxy-phényl)-1-méthyl-imidazole-2-carboxamide ;
le
N-[3-chloro-4-[[1-[2-(diméthylamino)acétyl]-4-pipéridyl]carbamoyl]phényl]-5-(2,3-difluoro-4-méthoxy-phényl)-1-méthyl-imidazole-2-carboxamide ;
le
N-[3-chloro-4-[5-(diméthylamino)pentylcarbamoyl]phényl]-5-(2,3-difluoro-4-méthoxy-phényl)-1-méthyl-imidazole-2-carboxamide ;
le
N-[3-chloro-4-(2-isopentyloxyéthylcarbamoyl)phényl]-5-[4-(cyanométhoxy)-2,3-difluoro-phényl]-1-méthyl-imidazole-2-carboxamide ;
le
N-[3-chloro-4-[2-(3-hydroxypyrrolidin-1-yl)éthylcarbamoyl]phényl]-5-(2,3-difluoro-4-méthoxy-phényl)-1-méthyl-imidazole-2-carboxamide ;
le
N-[3-chloro-4-[2-[3-(hydroxyméthyl)pyrrolidin-1-yl]éthylcarbamoyl]phényl]-5-(2,3-difluoro-4-méthoxy-phényl)-1-méthyl-imidazole-2-carboxamide ;
le
5-(2,3-difluoro-4-méthoxy-phényl)-N-[4-[3-[2-(diméthylamino)éthoxy]propylcarbamoyl]-3-éthyl-phényl]-1-méthyl-imidazole-2-carboxamide ;
le
N-[3-chloro-4-[[1-[(3R)-pyrrolidine-3-carbonyl]pyrrolidin-3-yl]carbamoyl]phényl]-5-(2,3-difluoro-4-méthoxy-phényl)-1-méthyl-imidazole-2-carboxamide ;
le
N-[3-chloro-4-[[1-[(2S,4R)-4-hydroxypyrrolidine-2-carbonyl]pyrrolidin-3-yl]carbamoyl]phényl]-5-(2,3-difluoro-4-méthoxy-phényl)-1-méthyl-imidazole-2-carboxa mide ;
le
N-[4-[[1-[3-(2-aminoéthoxy)propanoyl]pyrrolidin-3-yl]carbamoyl]-3-chloro-phényl]-5-(2,3-difluoro-4-méthoxy-phényl)-1-méthyl-imidazole-2-carboxamide ;
le
N-[3-chloro-4-[[1-(pipéridine-4-carbonyl)-4-pipéridyl]carbamoyl]phényl]-5-(2,3-difluoro-4-méthoxy-phényl)-1-méthyl-imidazole-2-carboxamide ;
le
N-[3-chloro-4-[[1-[(3R)-pyrrolidine-3-carbonyl]-4-pipéridyl]carbamoyl]phényl]-5-(2,3-difluoro-4-méthoxy-phényl)-1-méthyl-imidazole-2-carboxamide ;
le
N-[3-chloro-4-[[1-[(2S,4R)-4-hydroxypyrrolidine-2-carbonyl]-4-pipéridyl]carbamoyl]phényl]-5-(2,3-difluoro-4-méthoxy-phényl)-1-méthyl-imidazole-2-carboxami de ;
le
N-[4-[[1-[3-(2-aminoéthoxy)propanoyl]-4-pipéridyl]carbamoyl]-3-chloro-phényl]-5-(2,3-difluoro-4-méthoxy-phényl)-1-méthyl-imidazole-2-carboxamide ;
le
N-[4-[[3-[[3-(aminométhyl)cyclobutanecarbonyl]amino]cyclobutyl]carbamoyl]-3-chloro-phényl]-5-(2,3-difluoro-4-méthoxy-phényl)-1-méthyl-imidazole-2-carboxa mide ;
le
N-[4-[2-[1-(3-aminopropanoyl)azétidin-3-yl]éthylcarbamoyl]-3-chloro-phényl]-5-[4-(difluorométhoxy)-2,3-difluoro-phényl]-1-méthyl-imidazole-2-carboxamide ;
le
N-[4-[3-(3-aminopropanoylamino)propylcarbamoyl]-3-chloro-phényl]-5-[4-(difluorométhoxy)-2,3-difluoro-phényl]-1-méthyl-imidazole-2-carboxamide ;
le
N-[3-[[4-[[5-[4-(difluorométhoxy)-2,3-difluoro-phényl]-1-méthyl-imidazole-2-carbonyl]amino]-2-éthyl-benzoyl]amino]propyl]pipéridine-4-carboxamide ;
le
N-[2-[[4-[[5-[4-(cyanométhoxy)-2,3-difluoro-phényl]-1-méthyl-imidazole-2-carbonyl]amino]-2-méthyl-benzoyl]amino]éthyl]pipéridine-4-carboxamide ;
le
N-[2-[[4-[[5-[4-(cyanométhoxy)-2,3-difluoro-phényl]-1-méthyl-imidazole-2-carbonyl]amino]-2-méthyl-benzoyl]amino] éthyl] -4-hydroxy-pipéridine-4-carboxamide
le
N-[2-[[2-chloro-4-[[5-[4-(cyanométhoxy)-2,3-difluoro-phényl]-1-méthyl-imidazole-2-carbonyl]amino]benzoyl]amino]éthyl]pipéridine-4-carboxamide ;
le
N-[2-[[2-chloro-4-[[5-[4-(cyanométhoxy)-2,3-difluoro-phényl]-1-méthyl-imidazole-2-carbonyl]amino]benzoyl]amino]éthyl]-4-hydroxy-pipéridine-4-carboxamide ;
le
N-[4-[[1-(2-aminoacétyl)azétidin-3-yl]carbamoyl]-3-chloro-phényl]-5-(2,3-difluoro-4-méthoxy-phényl)-1-méthyl-imidazole-2-carboxamide ;
le
N-[4-[[1-(2-aminoacétyl)azétidin-3-yl]méthylcarbamoyl]-3-chloro-phényl]-5-(2,3-difluoro-4-méthoxy-phényl)-1-méthyl-imidazole-2-carboxamide ;
le
N-[4-[[3-(2-aminoéthylcarbamoyl)cyclobutyl]carbamoyl]-3-chloro-phényl]-5-(2,3-difluoro-4-méthoxy-phényl)-1-méthyl-imidazole-2-carboxamide ;
le
N-[4-[[3-(3-aminoazétidine-1-carbonyl)cyclobutyl]carbamoyl]-3-chloro-phényl]-5-(2,3-difluoro-4-méthoxy-phényl)-1-méthyl-imidazole-2-carboxamide ;
le
N-[4-[[3-[(3-aminocyclobutyl)carbamoyl]cyclobutyl]carbamoyl]-3-chloro-phényl]-5-(2,3-difluoro-4-méthoxy-phényl)-1-méthyl-imidazole-2-carboxamide ;
le
N-[4-[[3-(2-aminoéthylcarbamoyl)cyclobutyl]carbamoyl]-3-éthyl-phényl]-5-(2,3-difluoro-4-méthoxy-phényl)-1-méthyl-imidazole-2-carboxamide ;
le
N-[4-[[1-(2-aminoacétyl)azétidin-3-yl]carbamoyl]-3-éthyl-phényl]-5-(2,3-difluoro-4-méthoxy-phényl)-1-méthyl-imidazole-2-carboxamide ;
le
5-[4-(cyanométhoxy)-2,3-difluoro-phényl]-N-[4-[2-[[(2R,4R)-4-hydroxy-4-méthyl-pyrrolidine-2-carbonyl]amino]éthylcarbamoyl]-3-méthyl-phényl]-1-méthyl-imidazole-2-carboxamide ;
le
5-[4-(cyanométhoxy)-2,3-difluoro-phényl]-N-[4-[2-[[(2R,4R)-4-éthyl-4-hydroxy-pyrrolidine-2-carbonyl]amino]éthylcarbamoyl]-3-méthyl-phényl]-1-méthyl-imidazole-2-carboxamide ;
le
5-(2,3-difluoro-4-méthoxy-phényl)-N-[4-[2-[[(2R,4R)-4-hydroxy-4-méthyl-pyrrolidine-2-carbonyl]amino]éthylcarbamoyl]-3-méthyl-phényl]-1-méthyl-imidazole-2-carboxamide ;
le
5-(2,3-difluoro-4-méthoxy-phényl)-N-[4-[2-[[(2R,4R)-4-éthyl-4-hydroxy-pyrrolidine-2-carbonyl]amino] éthylcarbamoyl] -3-méthyl-phényl] -1-méthyl-imidazole-2-carboxamide ;
le
5-(2,3-difluoro-4-méthoxy-phényl)-N-[4-[2-[[(3R,4R)-4-hydroxypyrrolidin-3-yl]carbamoylamino]éthylcarbamoyl]-3-méthyl-phényl]-1-méthyl-imidazole-2-carboxamide ;
le
5-(2,3-difluoro-4-méthoxy-phényl)-N-[4-[2-[[(3S,4S)-4-hydroxypyrrolidin-3-yl]carbamoylamino]éthylcarbamoyl]-3-méthyl-phényl]-1-méthyl-imidazole-2-carboxamide ;
le
5-[4-(cyanométhoxy)-2,3-difluoro-phényl]-N-[4-[2-[[(3R,4R)-4-hydroxypyrrolidin-3-yl]carbamoylamino]éthylcarbamoyl]-3-méthyl-phényl]-1-méthyl-imidazole-2-carboxamide ;
le
5-[4-(cyanométhoxy)-2,3-difluoro-phényl]-N-[4-[2-[[(3S,4S)-4-hydroxypyrrolidin-3-yl]carbamoylamino]éthylcarbamoyl]-3-méthyl-phényl]-1-méthyl-imidazole-2-carboxamide ;
le
(3R,4R)-N-[2-[[2-chloro-4-[[5-[4-(cyanométhoxy)-2,3-difluoro-phényl]-1-méthyl-imidazole-2-carbonyl]amino]benzoyl]amino]éthyl]-3-hydroxy-pipéridine-4-carboxamide ;
le
(3S,4S)-N-[2-[[2-chloro-4-[[5-[4-(cyanométhoxy)-2,3-difluoro-phényl]-1-méthyl-imidazole-2-carbonyl]amino] benzoyl]amino]éthyl]-3-hydroxy-pipéridine-4-carboxamide ;
le
(3S,4R)-N-[2-[[2-chloro-4-[[5-[4-(cyanométhoxy)-2,3-difluoro-phényl]-1-méthyl-imidazole-2-carbonyl]amino]benzoyl]amino]éthyl]-3-hydroxy-pipéridine-4-carboxamide ;
le
(3R,4S)-N-[2-[[2-chloro-4-[[5-[4-(cyanométhoxy)-2,3-difluoro-phényl]-1-méthyl-imidazole-2-carbonyl]amino]benzoyl]amino]éthyl]-3-hydroxy-pipéridine-4-carbo xamide ;
le
N-[3-chloro-4-[2-[[(2R,4R)-4-hydroxy-4-méthyl-pyrrolidine-2-carbonyl]amino]éthylcarbamoyl]phényl]-5-[4-(cyanométhoxy)-2,3-difluoro-phényl]-1-méthyl-imidazole-2-carboxamide ;
le
N-[3-chloro-4-[2-[[(2R,4R)-4-éthyl-4-hydroxy-pyrrolidine-2-carbonyl]amino]éthylcarbamoyl]phényl]-5-[4-(cyanométhoxy)-2,3-difluoro-phényl]-1-méthyl-imidazole-2-carboxamide ;
le
N-[3-chloro-4-[[3-[[(4R)-4-hydroxypyrrolidine-2-carbonyl]amino]cyclobutyl]carbamoyl]phényl]-5-(2,3-difluoro-4-méthoxy-phényl)-1-méthyl-imidazole-2-carboxamide ;
le
N-[3-[[2-chloro-4-[[5-(2,3-difluoro-4-méthoxy-phényl)-1-méthyl-imidazole-2-carbonyl]amino]benzoyl]amino]cyclobutyl]pipéridine-4-carboxamide ;
le
N-[3-[[2-chloro-4-[[5-(2,3-difluoro-4-méthoxy-phényl)-1-méthyl-imidazole-2-carbonyl]amino]benzoyl]amino]cyclobutyl]-4-hydroxy-pipéridine-4-carboxamide ;
le
(3R,4R)-N-[3-[[2-chloro-4-[[5-(2,3-difluoro-4-méthoxy-phényl)-1-méthyl-imidazole-2-carbonyl]amino] benzoyl]amino] cyclobutyl]-3-hydroxy-pipéridine-4-carboxamide ;
le
(3S,4S)-N-[3-[[2-chloro-4-[[5-(2,3-difluoro-4-méthoxy-phényl)-1-méthyl-imidazole-2-carbonyl]amino]benzoyl]amino]cyclobutyl]-3-hydroxy-pipéridine-4-carboxamide ;
le
N-[4-[2-[1-(azétidin-3-ylméthyl)azétidin-3-yl]éthylcarbamoyl]-3-chloro-phényl]-5-[4-(difluorométhoxy)-2,3-difluoro-phényl]-1-méthyl-imidazole-2-carboxamide ;
le
N-[3-[[4-[[5-[4-(difluorométhoxy)-2,3-difluoro-phényl]-1-méthyl-imidazole-2-carbonyl]amino]-2-éthyl-benzoyl]amino]propyl]-1-(pyrrolidin-3-ylméthyl)pipéridine-4-carboxamide ;
le
1-(azétidin-3-ylméthyl)-N-[3-[[4-[[5-[4-(difluorométhoxy)-2,3-difluoro-phényl]-1-méthyl-imidazole-2-carbonyl]amino]-2-éthyl-benzoyl]amino]propyl]pipéridine-4-carboxamide ;
le
N-[4-[[1-(azétidin-3-ylméthyl)-4-pipéridyl]méthylcarbamoyl]-3-chloro-phényl]-5-(2,3-difluoro-4-méthoxy-phényl)-1-méthyl-imidazole-2-carboxamide ;
le
5-(4-méthoxyphényl)-1-méthyl-N-[3-méthyl-4-(méthylcarbamoyl)phényl]imidazole-2-carboxamide ;
le
N-[3-chloro-4-[[(exo)-3-[(2S,4R)-4-hydroxyprolyl]-3-azabicyclo[3.1.0]hexan-6-yl]carbamoyl]phényl]-5-(2,3-difluoro-4-méthoxy-phényl)-1-méthyl-imidazole-2-carboxamide ; et
le
(exo)-6-[[2-chloro-4-[[5-(2,3-difluoro-4-méthoxy-phényl)-1-méthyl-imidazole-2-carbonyl]amino]benzoyl]amino]-N-[(trans)-4-hydroxypyrrolidin-3-yl]-3-azabicyclo[3.1.0]hexane-3-carboxamide.

13. Composé de formule (1) selon l'une quelconque des revendications 1 à 12, ou un sel pharmaceutiquement acceptable de celui-ci, pour une utilisation comme substance thérapeutiquement active.

14. Composition pharmaceutique comprenant un composé de formule (1) selon l'une quelconque des revendications 1 à 12, ou un sel pharmaceutiquement acceptable de celui-ci, et un véhicule thérapeutiquement inerte.

15. Composé de formule (1) selon l'une quelconque des revendications 1 à 12, ou un sel pharmaceutiquement acceptable de celui-ci, pour une utilisation comme antibiotique.
